# EUROPÄISCHE PATENTSCHRIFT

(11) **EP 1 716 105 B1**
(45) Veröffentlichungstag und Bekanntmachung des Hinweises auf die Patenterteilung: **27.04.2011**
(21) Anmeldenummer: 05707029.4
(22) Anmeldetag: 27.01.2005
(51) Int. Cl.: C07C 253/10, C07C 255/07

(54) **VERFAHREN ZUR HERSTELLUNG VON LINEAREM PENTENNITRIL**
METHOD FOR PRODUCING LINEAR PENTENENITRILE
PROCEDE DE PRODUCTION DE PENTENE-NITRILE LINEAIRE

(30) Priorität: 29.01.2004 DE 102004004671; 02.09.2004 DE 102004042949; 23.12.2004 DE 102004063381
(43) Veröffentlichungstag der Anmeldung: 02.11.2006
(73) Patentinhaber: BASF SE, 67056 Ludwigshafen (DE)
(72) Erfinder: JUNGKAMP, Tim, B-2950 Kapellen (BE); BAUMANN, Robert, 68159 Mannheim (DE); BARTSCH, Michael, 67433 Neustadt (DE); HADERLEIN, Gerd, 67269 Grünstadt (DE); LUYKEN, Hermann, 67069 Ludwigshafen (DE); SCHEIDEL, Jens, 69493 Hirschberg (DE); AECHTNER, Tobias, 68163 Mannheim (DE); PFAB, Peter, 67433 Neustadt (DE); DECKERT, Petra, 69245 Bammental (DE); BASSLER, Peter, 68519 Viernheim (DE); SIEGEL, Wolfgang, 67117 Limburgerhof (DE)
(86) Internationale Anmeldenummer: PCT/EP2005/000781
(87) Internationale Veröffentlichungsnummer: WO 2005/073174

(56) Entgegenhaltungen:
- EP-A- 0 274 401
- WO-A-99/07671
- US-A- 3 536 748

## Beschreibung

Die vorliegende Erfindung betrifft ein Verfahren zur Herstellung von 3-Pentenitril durch Isomerisierung von Strömen, enthaltend 2-Methyl-3-butennitril.

Bei der Herstellung von Adipodinitril, einem wichtigen Intermediat in der Nylonproduktion, wird 1,3-Butadien zunächst mit Cyanwasserstoff in Gegenwart von Nickel(0), das mit Phosphorliganden stabilisiert ist, zu Pentennitrilen umgesetzt. Neben den Hauptprodukten der Hydrocyanierung, 3-Pentennitril und 2-Methyl-3-butennitril, werden auch zahlreiche Nebenkomponenten erhalten. Beispiele hierfür sind 2-Pentennitrile, 2-Methyl-2-butennitrile, C₉-Nitrile und Methylglutardinitril. 2-Methyl-3-butennitril entsteht in bedeutenden Mengen. So kann je nach eingesetztem Katalysator das molare Verhältnis von gebildetem 2-Methyl-3-butennitril zu 3-Pentennitril bis zu 2:1 betragen.

In einer zweiten Hydrocyanierung wird anschließend 3-Pentennitril mit Cyanwasserstoff zu Adipodinitril an dem gleichen Nickel-Katalysator unter Zusatz einer Lewis-Säure umgesetzt. Für die zweite Hydrocyanierung ist es wesentlich, dass das 3-Pentennitril möglichst frei von 2-Methyl-3-butennitril ist. Eine Hydrocyanierung von 2-Methyl-3-butennitril würde zu Methylglutardinitril führen, das ein unerwünschtes Nebenprodukt darstellt. Demnach muss in einem wirtschaftlichen Verfahren zur Herstellung von Adipodinitril eine Trennung von 3-Pentennitril und 2-Methyl-3-butennitril erfolgen.

Um 2-Methyl-3-butennitril ebenfalls für die Herstellung Adipodinitril nutzen zu können, wurden Verfahren zur Isomerisierung von 2-Methyl-3-butennitril zu linearem Pentennitril, insbesondere 3-Pentennitril, vorgeschlagen.

So beschreibt US 3,676,481 die diskontinuierliche, chargenweise Isomerisierung von 2-Methyl-3-butennitril in Gegenwart von Ni(0), einem Phosphit-Liganden und bestimmten Lewis-Säuren. Nach der Isomerisierung wird das entstandene Produktgemisch von dem Katalysatorsystem abdestilliert. Nachteilig bei diesem Verfahren sind die hohen Verweilzeiten während der Isomerisierung, die hohe thermische Belastung des thermisch empfindlichen Katalysators während der Isomerisierung und während der nachfolgenden Destillation. Die hohe thermische Belastung des Katalysators führt zu einer unerwünschten Degradation des Katalysators.

Die prioritätsältere, nicht vorveröffentlichte deutsche Patentanmeldung DE 103 11 119.0 der BASF AG beschreibt ein Verfahren zur Isomerisierung von 2-Methyl-3-butennitril zu linearem Pentennitril in Gegenwart eines Systems, enthaltend Ni(0)-Katalysatoren und Lewis-Säuren. Dabei entnimmt man destillativ während der Isomerisierung dem Reaktionsgemisch eine Mischung, enthaltend 2-Methyl-3-butennitril und lineares Pentennitril. Nachteilig bei diesem Verfahren ist, dass der entnommene Produktstrom nach wie vor deutliche Mengen nicht umgesetztes 2-Methyl-3-butennitril enthält.

Allen bekannten Verfahren zur Isomerisierung von 2-Methyl-3-butennitril ist gemeinsam, dass 2-Methyl-3-butennitril wegen der thermodynamischen Gleichgewichtslage nicht vollständig zu 3-Pentennitril umgesetzt werden kann. Nicht umgesetztes 2-Methyl-3-butennitril muss für eine wirtschaftliche Ausübung des Verfahrens dem Isomerisierungsschritt wieder zugeführt werden. Bei der Isomerisierung von 2-Methyl-3-butennitril wird aber als Nebenprodukt (Z)-2-Methyl-2-butennitril erhalten, das sich bei Rückführung von 2-Methyl-3-butennitril im Kreislaufstrom aufpegeln würde, da es bei der Abtrennung von 3-Pentennitril aus dem lsomerisierungsproduktstrom durch Destillation wegen der sehr ähnlichen Dampfdrücke zusammen mit dem 2-Methyl-3-butennitril übergeht.

US 3,865,865 beschreibt die Abtrennung von 2-Methyl-2-butennitril aus einer Mischung mit 2-Methyl-3-butennitril. Die Abtrennung wird durchgeführt, indem die Mischung der Nitrile mit einer wässrigen Lösung behandelt wird, die aus Sulfit- und Bisulfitionen besteht. Hierbei bildet sich das Bisulfit-Addukt von 2-Methyl-2-butennitril, das in die wässrige Phase übergeht. Die resultierende organische Phase wird dabei auf 50 % des ursprünglichen Gehalts an 2-Methyl-2-butennitril abgereichert. Das Verfahren gemäß US 3,865,865 ist umständlich, da eine Phasentrennung einer organischen von einer wässrigen Phase erforderlich ist. Darüber hinaus lässt sich diese Trennung nur schwierig in ein Gesamtverfahren zur Herstellung von Adipodinitril integrieren. Nachteilig bei diesem Verfahren ist zusätzlich, dass die erhaltene organische Phase vor der Weiterverwendung in Hydrocyanierungsreaktionen unter Verwendung von Nickel(0)-Katalysatoren mit Phosphor(III)-haltigen Liganden erst vollständig von Wasser befreit werden muss, da ansonsten die Phosphor(III)-haltigen Liganden irreversibel hydrolysiert und damit inaktiviert werden. Weiterhin nachteilig bei diesem Verfahren ist, dass die erhaltenen Bisulfit-Addukte zwecks Weiterverwendung der konjugierten Nitrile, wie in US 3,865,865 beschrieben, nur unter drastischen Bedingungen und nur mit mäßiger Ausbeute zurückspaltbar sind. US-A-3 536 748 beschreibt die Isomerisierung von 2-Methyl-3-butennitril.

Aufgabe der vorliegenden Erfindung ist somit, ein Verfahren zur Herstellung von 3-Pentennitril durch Isomerisierung von 2-Methyl-3-butennitril zur Verfügung zu stellen, wobei der Katalysator zur Isomerisierung in einfacher Weise aus der Reaktionsmischung abgetrennt und zurückgeführt werden kann und sowohl die Abtrennung von (Z)-2-Methyl-2-butennitril von 2-Methyl-3-butennitril als auch die Rückführung des an (Z)-2-Methyl-2-butennitril abgereicherten 2-Methyl-3-butennitrils ermöglicht wird. Das Verfahren sollte vorzugsweise technisch einfach und wirtschaftlich durchführbar sein und sich in ein Gesamtverfahren zur Herstellung von Adipodinitril einbinden lassen. Diese Aufgabe wird erfindungsgemäß durch ein Verfahren zur Herstellung von 3-Pentennitril gelöst.

### Ausführungsform I

Das Verfahren ist in einer Ausführungsform I durch die folgenden Verfahrensschritte gekennzeichnet:
(a) Isomerisierung eines Eduktstroms, der 2-Methyl-3-butennitril enthält, an mindestens einem gelösten oder dispergierten Isomerisierungskatalysator zu einem Strom 1, der den mindestens einen Isomerisierungskatalysator, 2-Methyl-3-butennitril, 3-Pentennitril und (Z)-2-Methyl-2-butennitril enthält,
(b) Destillation des Stromes 1 unter Erhalt eines Stromes 2 als Kopfprodukt, der 2-Methyl-3-butennitril, 3-Pentennitril und (Z)-2-Methyl-2-butennitril enthält, und eines Stromes 3 als Sumpfprodukt, der den mindestens einen lsomerisierungskatalysator enthält,
(c) Destillation des Stromes 2 unter Erhalt eines Stromes 4 als Kopfprodukt, der gegenüber dem Strom 2 an (Z)-2-Methyl-2-butennitril, bezogen auf die Summe aller Pentennitrile im Strom 2, angereichert ist, und eines Stromes 5 als Sumpf produkt, der gegenüber dem Strom 2 an 3-Pentennitril und 2-Methyl-3-butennitril, bezogen auf die Summe aller Pentennitrile im Strom 2, angereichert ist,
(d) Destillation des Stromes 5 unter Erhalt eines Stromes 6 als Sumpfprodukt, der 3-Pentennitril enthält, und eines Stromes 7 als Kopfprodukt, der 2-Methyl-3-butennitril enthält.

### Eduktstrom

In Verfahrensschritt (a) findet eine Isomerisierung eines Eduktstromes, der 2-Methyl-3-butennitril enthält, an mindestens einem Isomerisierungskatalysator statt.

In einer besonderen Ausführungsform des erfindungsgemäßen Verfahrens ist der Eduktstrom durch folgende Verfahrensschritte erhältlich:
(e) Hydrocyanierung von 1,3-Butadien an mindestens einem Hydrocyanierungskatalysator mit Cyanwasserstoff unter Erhalt eines Stromes 8, der den mindestens einen Hydrocyanierungskatalysator, 3-Pentennitril, 2-Methyl-3-butennitril, 1,3-Butadien und Reste Cyanwasserstoff enthält,
(f) ein- oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält,
(g) Destillation des Stromes 11 unter Erhalt eines Stromes 12 als Sumpfprodukt, der 3-Pentennitril enthält, und eines Stromes 13 als Kopfprodukt, der 2-Methyl-3-butennitril enthält.

In Verfahrensschritt (e) findet zur Herstellung des Eduktstroms zunächst eine Hydrocyanierung von 1,3-Butadien an mindestens einem Hydrocyanierungskatalysator mit Cyanwasserstoff statt unter Erhalt eines Stromes 8, der den mindestens einen Hydrocyanierungskatalysator, 3-Pentennitril, 2-Methyl-3-butennitril und nicht umgesetztes 1,3-Butadien enthält.

Als Hydrocyanierungskatalysator wird vorzugsweise ein homogener Nickel(0)-Katalysator verwendet, der mit Phosphorliganden stabilisiert ist.

Die phosphorhaltigen Liganden der Nickel(0)-Komplexe und die freien phosphorhaltigen Ligandensind vorzugsweise ausgewählt aus mono- oder bidentaten Phosphinen, Phosphiten, Phosphiniten und Phosphoniten.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer bevorzugten Ausführungsform sollten alle der Gruppen X¹, X² und X³ für Sauerstoff stehen, so dass Verbindung I vorteilhaft ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer bevorzugten Ausführungsform kommen als Gruppen R¹, R² und R³ Reste ausgewählt aus der Gruppe bestehend aus Phenyl, o-Tolyl, m-Tolyl und p-Tolyl in Betracht. In einer besonders bevorzugten Ausführungsform sollten dabei maximal zwei der Gruppen R¹, R² und R³ Phenyl-Gruppen sein.

In einer anderen bevorzugten Ausführungsform sollten dabei maximal zwei der Gruppen R¹, R² und R³ o-Tolyl-Gruppen sein.

Als besonders bevorzugte Verbindungen I können solche der Formel I a

(o-Tolyl-O-)_{w} (m-Tolyl-O-)ₓ (p-Tolyl-O-)_{y} (Phenyl-O-)_{z} P (I a)

eingesetzt werden, wobei w, x, y und z eine natürliche Zahl bedeuten, und folgende Bedingungen gelten: w + x + y + z = 3 und w, z ≤2.

Solche Verbindungen I a sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P , (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen. Gemische enthaltend (m-Tolyl-O-)₃P, (m -Tolyl-O-)₂(p-Tolyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer anderen, ebenfalls bevorzugten Ausführungsform kommen als phosphorhaltige Liganden die in der DE-A 199 53 058 näher beschriebenen Phosphite der Formel I b in Betracht:

P (O-R¹)ₓ (O-R²)_{y} (O-R³)_{z} (O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem a- romatischen System verbindet, anellierten aromatischen System, wobei der aro- matische Rest in o-Stellung zu dem Sauerstoffatom, das das Phosphoratom mit dem aromatischen System verbindet, ein Wasserstoffatom trägt,
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel I b sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-lsopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung I b ergeben sich folgende Möglichkeiten:

| | | | |
|---|---|---|---|
| x | y | z | p |
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel I b sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel I b sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-Isopropyl-phenyl-Rest, R² der 2-Naphthylrest und R³ der p-Tolylrest ist mit den in der Tabelle genannten Indizes; sowie Gemische dieser Phosphite.

Phosphite der Formel I b können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH, R³OH und R⁴OH oder deren Gemische umsetzt unter Erhalt eines Phosphits der Formel I b.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird; sowie deren Gemische in Betracht. Es können auch Gemische verschiedener gleich oder unterschiedlich halogensubstituierter Phosphine als Phosphortrihalogenid eingesetzt werden. Besonders bevorzugt ist PCl₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist allerdings bevorzugt, dass der phosphorhaltige Ligand mehrzähnig, insbesondere zweizähnig ist. Daher weist der verwendete Ligand vorzugsweise die Formel II 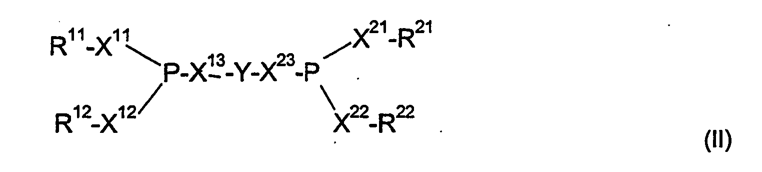 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹ , R²²: unabhängig voneinander gleiche oder unterschiedliche, einzelne oder verbrückte organische Reste,
- Y: Brückengruppe

Unter Verbindung II wird im Sinne der vorliegenden Erfindung eine einzelne Verbindung oder ein Gemisch verschiedener Verbindungen der vorgenannten Formel verstanden.

In einer bevorzugten Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen bevorzugten Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen bevorzugten Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen bevorzugten Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer besonders bevorzugten Ausführungsform kommen die in US 5,723,641 genannten Verbindungen der Formel I, II, III, IV und V in Betracht. In einer besonders bevorzugten Ausführungsform kommen die in US 5,512,696 genannten Verbindungen der Formel I, II, III IV, V, VI und VII, insbesondere die dort in den Beispielen 1 bis 31 eingesetzten Verbindungen, in Betracht. In einer besonders bevorzugten Ausführungsform kommen die in US 5,821,378 genannten Verbindungen der Formel I, II, III, IV, V, VI, VII, VIII, IX, X, XI, XII, XIII, XIV und XV, insbesondere die dort in den Beispielen 1 bis 73 eingesetzten Verbindungen, in Betracht.

In einer besonders bevorzugten Ausführungsform kommen die in US 5,512,695 genannten Verbindungen der Formel I, II, III, IV, V und VI, insbesondere die dort in den Beispielen 1 bis 6 eingesetzten Verbindungen, in Betracht. In einer besonders bevorzugten Ausführungsform kommen die in US 5,981,772 genannten Verbindungen der Formel I, II, III, IV, V, VI, VII, VIII, IX, X, XI, XII, XIII und XIV, insbesondere die dort in den Beispielen 1 bis 66 eingesetzten Verbindungen, in Betracht.

In einer besonders bevorzugten Ausführungsform kommen die in US 6,127,567 genannten Verbindungen und dort in den Beispielen 1 bis 29 eingesetzten Verbindungen in Betracht. In einer besonders bevorzugten Ausführungsform kommen die in US 6,020,516 genannten Verbindungen der Formel I, II, III, IV, V, VI, VII, VIII, IX und X, insbesondere die dort in den Beispielen 1 bis 33 eingesetzten Verbindungen, in Betracht. In einer besonders bevorzugten Ausführungsform kommen die in US 5,959,135 genannten Verbindungen und dort in den Beispielen 1 bis 13 eingesetzten Verbindungen in Betracht.

In einer besonders bevorzugten Ausführungsform kommen die in US 5,847,191 genannten Verbindungen der Formel I, II und III in Betracht. In einer besonders bevorzugten Ausführungsform kommen die in US 5,523,453 genannten Verbindungen, insbesondere die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer besonders bevorzugten Ausführungsform kommen die in WO 01/14392 genannten Verbindungen, vorzugsweise die dort in Formel V, VI VII, VIII, IX, X, XI, XII, XIII, XIV, XV, XVI, XVII, XXI, XXII, I, XXIII dargestellten Verbindungen, in Betracht.

In einer besonders bevorzugten Ausführungsform kommen die in WO 98/27054 genannten Verbindungen in Betracht. In einer besonders bevorzugten Ausführungsform kommen die in WO 99/13983 genannten Verbindungen in Betracht. In einer besonders bevorzugten Ausführungsform kommen die in WO 99/64155 genannten Verbindungen in Betracht.

In einer besonders bevorzugten Ausführungsform kommen die in der deutschen Patentanmeldung DE 100 380 37 genannten Verbindungen in Betracht. In einer besonders bevorzugten Ausführungsform kommen die in der deutschen Patentanmeldung DE 100 460 25 genannten Verbindungen in Betracht. In einer besonders bevorzugten Ausführungsform kommen die in der deutschen Patentanmeldung DE 101 502 85 genannten Verbindungen in Betracht.

In einer besonders bevorzugten Ausführungsform kommen die in der deutschen Patentanmeldung DE 101 502 86 genannten Verbindungen in Betracht. In einer besonders bevorzugten Ausführungsform kommen die in der deutschen Patentanmeldung DE 102 071 65 genannten Verbindungen in Betracht. In einer weiteren besonders bevorzugten Ausführungsform der vorliegenden Erfindung kommen die in der US 2003/0100442 A1 genannten phosphorhaltigen Chelatliganden in Betracht.

In einer weiteren besonders bevorzugten Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nicke (0)-Komplexes und/oder der freie phosphorhaltige Ligand ausgewählt aus Tritolylphosphit, bidentaten phosphorhaltigen Chelatliganden, sowie den Phosphiten der Formel I b

P (O-R¹)ₓ (O-R²)_{y} (O-R³)_{z} (O-R4⁴)ₚ (I b)

worin R¹, R² und R³ unabhängig voneinander ausgewählt sind aus o-lsopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen.

Der Verfahrensschritt (e) kann in jeder geeigneten, dem Fachmann bekannten Apparatur durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaten durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einen Katalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Reaktion kann in Batchfahrweise, kontinuierlich oder im Semibatchbetrieb durchgeführt werden.

Die Hydrocyanierungsreaktion kann durchgeführt werden, indem die Vorrichtung mit allen Reaktanten bestückt wird. Bevorzugt ist allerdings, wenn die Vorrichtung mit dem Katalysator, der ungesättigten organischen Verbindung und gegebenenfalls dem Lösemittel gefüllt wird. Vorzugsweise schwebt der gasförmige Cyanwasserstoff über der Oberfläche der Reaktionsmischung oder wird durch die Reaktionsmischung geleitet. Eine weitere Verfahrensweise zum Bestücken der Vorrichtung ist das Befüllen der Vorrichtung mit dem Katalysator, Cyanwasserstoff und gegebenenfalls dem Lösemittel und das langsame Zuspeisen der ungesättigten Verbindung zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor dem Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn das erfindungsgemäße Verfahren im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Reaktion wird vorzugsweise bei absoluten Drücken von 0,1 bis 500 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (e) wird ein Strom 8, der 3-Pentennitril, 2-Methyl-3-butennitril, den mindestens eine Katalysator und nicht umgesetztes 1,3-Butadien enthält, erhalten.

Der Strom 8, der 3-Pentennitril, 2-Methyl-3-butennitril, den mindestens einen Katalysator und nicht umgesetztes 1,3-Butadien enthält, wird anschließend in Verfahrensschritt (f) in eine Destillationsvorrichtung überführt, In dieser Destillationsvorrichtung erfolgt eine einfache oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält.

Die Destillation des Verfahrensschrittes (f) kann zweistufig erfolgen, wie in der DE-A-102 004 004 720, Verfahrensschritte (b) und (c), beschrieben. Die Destillation des Verfahrensschrittes (f) kann auch gemäß der DE-A-102 004 004729, Verfahrensschritte (b) und (c) erfolgen.

Die Destillation(en) des Verfahrensschrittes (f) kann (können) in jeder geeigneten, dem Fachmann bekannten Apparatur durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die einzelnen Destillationen kann man jeweils in mehreren, wie zwei oder drei Apparaturen, vorteilhaft in jeweils einer einzigen Apparatur durchführen.

Die Destillation(en) kann/können zudem jeweils einstufig im Sinne einer Teilverdampfung des Zulaufstroms erfolgen.

Der Druck in Verfahrensschritt (f) beträgt vorzugsweise 0,001 bis 10 bar, besonders bevorzugt 0,010 bis 1 bar, insbesondere 0,02 bis 0,5 bar. Die Destillation(en) wird/werden so durchgeführt, dass die Temperatur(en) im Sumpf der Destillationsvorrichtung(en) vorzugsweise 30 bis 200 °C, besonders bevorzugt 50 bis 150 °C, insbesondere 60 bis 120 °C, beträgt / betragen. Die Destillation(en) wird/werden so durchgeführt, dass die Kondensationstemperaturen am Kopf der Destillationsvorrichtung vorzugsweise -50 bis 150 °C, besonders bevorzugt -15 bis 60 °C, insbesondere 5 bis 45°C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung(en) eingehalten.

Der Strom 11 wird anschließend in einem weiteren Verfahrensschritt (g) einer Destillation unterzogen. Diese Destillation kann in jeder geeigneten, dem Fachmann bekannten Apparatur durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Druck in Verfahrensschritt (g) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

Im Verfahrensschritt (g) wird ein Strom 12 als Sumpfprodukt, der 1,3-Pentennitril enthält, und Strom 13 als Kopfprodukt, der 2-Methyl-3-butennitril enthält, erhalten. Der Strom 13 wird vorzugsweise als Eduktstrom in dem erfindungsgemäßen Verfahren zur Herstellung von 3-Pentennitril verwendet.

In weiteren bevorzugten Ausführungsformen des erfindungsgemäßen Verfahrens wird der in Verfahrensschritt (e) erhaltene Strom 8 direkt in den Verfahrensschritt (g) überführt. In diesem Verfahrensschritt (g) wird dann über den Sumpf ein Strom erhalten, der im Wesentlichen 3-Pentennitril und den mindestens einen Hydrocyanierungskatalysator enthält. Darüber hinaus wird über Kopf ein Strom erhalten, der im Wesentlichen 2-Methyl-3-butennitril und 1,3-Butadien enthält. Dieser an 2-Methyl-3-butennitril und 1,3-Butadien reiche Strom kann ebenfalls als Eduktstrom in dem erfindungsgemäßen Verfahren zur Herstellung von 3-Pentennitril verwendet werden. Falls dieser Eduktstrom in dem erfindungsgemäßen Verfahren verwendet wird, so beträgt der Gehalt an 2-Methyl-3-butennitril in diesem Strom vorzugsweise 10 bis 90 Gew.-%, besonders bevorzugt 20 bis 85 Gew.%, insbesondere 30 bis 80 Gew.%, jeweils bezogen auf den Strom.

Alternativ ist es auch möglich, den in Verfahrensschritt (e) erhaltenen Strom 8 in dem Verfahrensschritt (f) nur an 1,3-Butadien abzureichem. Über den Sumpf des Verfahrensschrittes (f) wird dann ein Strom 11a erhalten, der im Wesentlichen 3-Pentennitril, 2-Methyl-3-butennitril und den mindestens einen Hydrocyanierungskatalysator enthält. Dieser Strom 11a wird dann anschließend in dem Verfahrensschritt (g) unter Abtrennung von 3-Pentennitril und dem mindestens einen Hydrocyanierungskatalysator einerseits sowie von 2-Methyl-3-butennitril andererseits weiter aufgearbeitet. Der aus dem Verfahrensschritt (g) stammende Strom 13a am Kopf der Destillation enthält im Wesentlichen 2-Methyl-3-butennitril. Dieser Strom 13a kann ebenfalls als Eduktstrom in dem erfindungsgemäßen Verfahren zur Herstellung von 3-Pentennitril verwendet werden.

In einer weiteren Ausführungsform wird der Strom 8 aus dem Verfahrensschritt (e) in Verfahrensschritt (f) nur an 1,3-Butadien abgereichert und in Verfahrensschritt (g) überführt, wo im Sumpf ein Strom 12 mit 3-Pentennitril und dem Hydrocyanierungskatalysator erhalten wird.

In einer weiteren Ausführungsform der vorliegenden Erfindung wird ein Eduktstrom verwendet, der aus einer Hydrocyanierung des Verfahrensschrittes (e) und einer anschließenden Aufarbeitung in Verfahrensschritt (f) stammt, wobei im Verfahrensschritt (f) gegebenenfalls nur eine Abreicherung an 1,3-Butadien vorgenommen wird. Der hieraus resultierende Strom 11b wird anschließend in den Verfahrensschritt (a) des erfindungsgemäßen Verfahrens überführt. Der in diesem Strom 11 b enthaltene Hydrocyanierungskatalysator wird dann vorzugsweise als der mindestens eine Isomerisierungskatalysator in dem Verfahrensschritt (a) des erfindungsgemäßen Verfahrens verwendet. Es kann eine geeignete Lewis-Säure zusätzlich zugegeben werden, wie beispielsweise in der DE-A-102 004 004 696 beschrieben.

In einer weiteren Ausführungsform der vorliegenden Erfindung ist es möglich, dass der in dem erfindungsgemäßen Verfahrensschritt (a) verwendete Eduktstrom dem Strom 11 des Verfahrensschrittes (f) entspricht, so dass auf eine Auftrennung des Stromes 11 in dem Verfahrensschritt (g) verzichtet wird.

In einer weiteren Ausführungsform des erfindungsgemäßen Verfahrens wird als Eduktstrom der Strom 8, der aus dem Verfahrensschritt (e) stammt, verwendet. In diesem Falle entfallen somit die Verfahrensschritte (f) und (g) in der Herstellung des Eduktstromes für das erfindungsgemäße Verfahren.

### Verfahrensschritt (a)

In Verfahrensschritt (a) findet eine Isomerisierung des Eduktstromes, der 2-Methyl-3-butennitril enthält, an mindestens einem Isomerisierungskatalysator statt. Dabei wird ein Strom 1, der den Isomerisierungskatalysator, nicht umgesetztes 2-Methyl-3-butennitril, 3-Pentennitril und (Z)-2-Methyl-2-butennitril enthält, erhalten.

Erfindungsgemäß führt man die Isomerisierung in Gegenwart eines Systems, enthaltend
a) Nickel(0),
b) eine Nickel(0) als Ligand komplexierende, dreibindigen Phosphor enthaltende Verbindung und gegebenenfalls
c) eine Lewis-Säure
durch.

Die Herstellung von Nickel(0) enthaltenden Katalysatorsystemen kann nach an sich bekannten Verfahren erfolgen.

Als Liganden für den Isomerisierungskatalysator können die gleichen phosphorhaltigen Liganden wie für den in Verfahrensschritt (e) verwendeten Hydrocyanierungskatalysator verwendet werden. Somit kann der Hydrocyanierungskatalysator identisch zu dem lsomerisierungskatalysator sein. Die Auswahl der Liganden für die Reaktionen in den Verfahrensschritten (a) und (e) muss aber nicht zwingend gleich sein.

Weiterhin enthält das System gegebenenfalls eine Lewis-Säure.

Im Sinne der vorliegenden Erfindung wird unter einer Lewis-Säure eine einzelne Lewis-Säure oder ein Gemisch aus mehreren, wie zwei, drei oder vier Lewis-Säuren, verstanden.

Als Lewis-Säure kommen dabei anorganische oder organische Metall-Verbindungen in Betracht, in denen das Kation ausgewählt ist aus der Gruppe, bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele sind ZnBr₂, Znl₂, Inch, ZnSO₄, CuCl₂, CuCl, Cu(O₃SCF₃)₂, CoCl₂, Col₂, Fel₂, FeCl₃, FeCl₂, FeCl₂(THF)₂, TiCl₄(THF)₂, TiCl₄, TiCl₃, ClTi(O-i-Propyl)₃, MnCl₂, ScCl₃, AlCl₃, (C₈H₁₇)AlCl₂, (C₈H₁₇)₂AlCl, (i-C₄H₉)₂AlCl, (C₆H₅)₂AlCl, (C₆H₅)AlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, wie beispielsweise in US 6,127,567, US 6,171,996 und US 6,380,421 beschrieben. Weiterhin kommen in Betracht Metallsalze, wie ZnCl₂, Col₂ und SnCl₂ sowie organometallische Verbindungen, wie RAlCl₂, R₂AlCl, RSnO₃SCF₃ und R₃B, wobei R eine Alkyl- oder Aryl-Gruppe ist, wie beispielsweise in US 3,496,217, US 3,496,218 und US 4,774,353 beschrieben. Weiterhin können gemäß US 3,773,809 als Promotor ein Metall in kationischer Form, ausgewählt aus der Gruppe, bestehend aus Zink, Cadmium, Beryllium, Aluminium, Gallium, Indium, Thallium, Titan, Zirkonium, Hafnium, Erbium, Germanium, Zinn, Vanadium, Niob, Scandium, Chrom, Molybdän, Wolfram, Mangan, Rhenium, Palladium, Thorium, Eisen und Kobalt, vorzugsweise Zink, Cadmium, Titan, Zinn, Chrom, Eisen und Kobalt, eingesetzt werden, wobei der anionische Teil der Verbindung ausgewählt sein kann aus der Gruppe, bestehend aus Halogeniden, wie Fluorid, Chlorid, Bromid und Jodid, Anionen niedriger Fettsäuren mit von 2 bis 7 Kohlenstoffatomen, HPO₃²', H₃PO²-, CF₃COO⁻, C₇H₁₅OSO₂⁻ oder SO₄²⁻. Weiterhin sind aus US 3,773,809 als geeignete Promotoren Borhydride, Organoborhydride und Borsäureester der Formel R₃B und B(OR)₃, wobei R ausgewählt ist aus der Gruppe, bestehend aus Wasserstoff, Aryl-Radikalen mit zwischen 6 und 18 Kohlenstoff-Atomen, mit Alkyl-Gruppen mit 1 bis 7 Kohlenstoff Atomen substituierten Aryl-Radikalen und mit Cyanosubstituierten Alkyl-Gruppen mit 1 bis 7 Kohlenstoff Atomen substituierten Aryl-Radikalen, vorteilhaft Triphenylbor, genannt. Weiterhin können, wie in US 4,874,884 beschrieben, synergistisch wirksame Kombinationen von Lewis-Säuren eingesetzt werden, um die Aktivität des Katalysatorsystems zu erhöhen. Geeignete Promotoren können beispielsweise aus der Gruppe bestehend aus CdCl₂, FeCl₂, ZnCl₂, B(C₆H₅)₃ und (C₆H₅)₃SnX, mit X=CF₃SO₃, CH₃C₆H₄SO₃ oder (C₆H₅)₃BCN ausgewählt werden, wobei für das Verhältnis von Promotor zu Nickel ein Bereich von vorzugsweise etwa 1:16 bis etwa 50:1 genannt ist.

Im Sinne der vorliegenden Erfindung umfasst der Begriff Lewis-Säure auch die in US 3,496,217, US 3,496,218, US 4,774,353, US 4,874,884, US 6,127,567, US 6,171,996 und US 6,380,421 genannten Promotoren.

Als besonders bevorzugte Lewis-Säuren kommen unter den genannten insbesondere Metallsalze, besonders bevorzugt Metallhalogenide, wie Fluoride, Chloride, Bromide, Jodide, insbesondere Chloride, in Betracht, von denen wiederum Zinkchlorid, Eisen-(II)-Chlorid und Eisen-(III)-chlorid besonders bevorzugt sind.

Die Isomerisierung kann in Gegenwart eines flüssigen Verdünnungsmittels,
- beispielsweise eines Kohlenwasserstoffs, wie Hexan, Heptan, Oktan, Cyclohexan, Methylcyclohexan, Benzol, Decahydronaphthalin
- beispielsweise eines Ethers, wie Diethylether, Tetrahydrofuran, Dioxan, Glykoldimethylether, Anisol,
- beispielsweise eines Esters, wie Ethylacetat, Methylbenzoat, oder
- beispielsweise eines Nitrils, wie Acetonitril, Benzonitril, oder
- Gemischen solcher Verdünnungsmittel durchgeführt werden.

In einer besonders bevorzugten Ausführungsform kommt eine Isomerisierung in Abwesenheit eines solchen flüssigen Verdünnungsmittels in Betracht.

Weiterhin hat es sich als vorteilhaft erwiesen, wenn die Isomerisierung in Verfahrensschritt (a) in nicht-oxidierend wirkender Atmosphäre, wie beispielsweise unter einer Schutzgasatmosphäre aus Stickstoff oder einem Edelgas, wie Argon, durchgeführt wird.

Der Verfahrensschritt (a) kann in jeder geeigneten, dem Fachmann bekannten Apparatur durchgeführt werden. Für die Reaktion kommen hierfür übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren. Die Reaktion kann in mehreren, wie zwei oder drei Apparaten, durchgeführt werden.

In einer bevorzugten Ausführungsform des erfindungsgemäßen Verfahrens wird die Isomerisierung in einem kompartimentiertem Rohrreaktor durchgeführt.

In einer weiteren bevorzugten Ausführungsform des erfindungsgemäßen Verfahrens wird die Isomerisierung in mindestens zwei in Serie geschalteten Reaktoren durchgeführt, wobei der erste Reaktor im Wesentlichen Rührkesselcharakteristik aufweist und der zweite Reaktor so ausgeführt ist, dass er im Wesentlichen Rohrcharakteristik aufweist.

In einer besonders bevorzugten Ausführungsform des erfindungsgemäßen Verfahrens wird die Isomerisierung in einem Reaktor durchgeführt, wobei der Reaktor die Charakteristik einer Rührkesselkaskade aufweist, die 2 bis 20 Rührkesseln, insbesondere 3 bis 10 Rührkesseln, entspricht.

Die Reaktion kann in einer Ausführungsform des erfindungsgemäßen Verfahrens in einer Destillationsapparatur durchgeführt werden, wobei die Isomerisierungsreaktion zumindest im Sumpfbereich der Destillationsapparatur stattfindet. Geeignet ist jede, dem Fachmann bekannte Destillationsapparatur, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation mit gleichzeitig stattfindender Reaktion kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (a) des erfindungsgemäßen Verfahrens wird vorzugsweise bei einem absoluten Druck von 0,1 mbar bis 100 bar, besonders bevorzugt 1 mbar bis 16 bar, insbesondere 10 mbar bis 6 bar, durchgeführt. Die Temperatur beträgt in Verfahrensschritt (a) vorzugsweise 25 bis 250 °C, besonders bevorzugt 30 bis 180 °C, insbesondere 40 bis 140 °C.

Die Zusammensetzung des entnommen Stroms hinsichtlich des molaren Verhältnisses von 2-Methyl-3-butennitril zu linearem Pentennitril und damit der Umsatzgrad an eingesetztem 2-Methyl-3-butennitril kann in Abhängigkeit von der Zusammensetzung des zugeführten Stroms auf technisch einfache Weise durch die Temperatur, die Katalysatorkonzentration, die Verweilzeit und die Gestaltung des Reaktors eingestellt werden. In einer bevorzugten Ausführungsform des erfindungsgemäßen Verfahrens wird der Umsatzgrad mit Hilfe dieser Maßnahmen auf Werte im Bereich 10 bis 99 %, besonders bevorzugt 30 bis 95 %, insbesondere 60 bis 90 %, eingestellt.

### Verfahrensschritt (b)

In Verfahrensschritt (b) wird der in Verfahrensschritt (a) erhaltene Strom 1 destilliert. Hierbei erhält man als Kopfprodukt einen Strom 2, der 2-Methyl-3-butennitril, 3-Pentennitril und (Z)-2-Methyl-2-butennitril enthält. Darüber hinaus wird in Verfahrensschritt (b) ein Strom 3 als Sumpfprodukt erhalten, der den mindestens einen Isomerisierungskatalysator enthält.

Der Verfahrensschritt (b) des erfindungsgemäßen Verfahrens kann in jeder geeigneten, dem Fachmann bekannten Destillationsvorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (b) des erfindungsgemäßen Verfahrens wird vorzugsweise bei einem absoluten Druck von 0,1 mbar bis 100 bar, besonders bevorzugt 1 mbar bis 6 bar, insbesondere 10 mbar bis 500 mbar, durchgeführt. Die Destillation wird so durchgeführt, dass die Temperatur im Sumpf der Destillationsvorrichtung vorzugsweise 25 bis 250 °C, besonders bevorzugt 40 bis 180 °C, insbesondere 60 bis 140 °C, beträgt. Die Destillation wird so durchgeführt, dass die Temperatur am Kopf der Destillationsvorrichtung vorzugsweise -15 bis 200 °C, besonders bevorzugt 5 bis 150 °C, insbesondere 10 bis 100 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In einer besonders bevorzugten Ausführungsform der vorliegenden Erfindung findet die in Verfahrensschritt (b) durchgeführte Destillation des Stromes 1 unter Druck- und Temperaturbedingungen statt, bei welcher der in der Mischung vorhandene Isomerisierungskatalysator weniger als in Verfahrensschritt (a) oder nicht aktiv ist.

In einer bevorzugten Ausführungsform der vorliegenden Erfindung wird der in Verfahrensschritt (b) erhaltene Strom 3, der den mindestens einen lsomerisierungskatalysator enthält, zumindest teilweise in den Verfahrensschritt (a) zurückgeführt.

In einer weiteren Ausführungsform des erfindungsgemäßen Verfahrens finden die Verfahrensschritte (a) und (b) in der gleichen Vorrichtung statt. Dabei ist es auch möglich, dass der Strom 3, der den mindestens einen Isomerisierungskatalysator enthält, aus dem Verfahrensschritt (b) nicht entnommen wird und in der gemeinsamen Vorrichtung der Verfahrensschritte (a) und (b) verweilt.

Alternativ ist es auch möglich, dass der aus Verfahrensschritt (b) stammende Strom 3, der den mindestens einen Isomerisierungskatalysator enthält, zumindest teilweise zur Herstellung des erfindungsgemäß verwendeten Eduktstromes in Verfahrensschritt (e) verwendet wird. In Verfahrensschritt (e) fungiert dieser mindestens eine lsomerisierungskatalysator dann als Hydrocyanierungskatalysator.

### Verfahrensschritt (c)

In Verfahrensschritt (c) findet eine Destillation des Stromes 2 statt. Hierbei wird ein Strom 4 als Kopfprodukt erhalten, der gegenüber dem Strom 2 an (Z)-2-Methyl-2-butennitril in Bezug auf die Summe aller in Strom 2 enthaltenen Pentennitrile angereichert ist. Darüber hinaus wird ein Strom 5 als Sumpfprodukt erhalten, der gegenüber dem Strom 2 an (Z)-2-Methyl-2-butennitril in Bezug auf die Summe aller in Strom 2 enthaltenen Pentennitrile abgereichert ist.

Der Verfahrensschritt (c) kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (c) des erfindungsgemäßen Verfahrens wird vorzugsweise bei einem absoluten Druck von 0,1 mbar bis 100 bar, besonders bevorzugt 1 mbar bis 6 bar, insbesondere 10 mbar bis 500 mbar, durchgeführt. Die Destillation wird so durchgeführt, dass die Temperatur im Sumpf der Destillationsvorrichtung vorzugsweise 25 bis 250 °C, besonders bevorzugt 40 bis 180 °C, insbesondere 60 bis 140 °C, beträgt. Die Destillation wird so durchgeführt, dass die Temperatur am Kopf der Destillationsvorrichtung vorzugsweise -15 bis 200 °C, besonders bevorzugt 5 bis 150 °C, insbesondere 10 bis 100 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In einer besonders bevorzugten Ausführungsform des erfindungsgemäßen Verfahrens werden die Verfahrensschritte (b) und (c) zusammen in einer Destillationsvorrichtung durchgeführt, wobei der Strom 3, der den mindestens einen Isomerisierungskatalysator enthält, als Sumpfprodukt, der Strom 4, der (Z)-2-Methyl-2-butennitril enthält, als Kopfprodukt und der Strom 5, der 3-Pentennitril und 2-Methyl-3-butennitril enthält, an einem Seitenabzug der Kolonne erhalten werden.

In einer weiteren bevorzugten Ausführungsform des erfindungsgemäßen Verfahrens werden die Verfahrensschritte (a), (b) und (c) zusammen in einer Destillationsvorrichtung durchgeführt. Dabei wird der Strom 4, der (Z)-2-Methyl-2-butennitril enthält, als Kopfprodukt erhalten. Der Strom 5, der 3-Pentennitril und 2-Methyl-3-butennitril enthält, wird an einem Seitenabzug der Destillationskolonne erhalten. Der lsomerisierungskatalysator verbleibt in dieser Ausführungsform vorzugsweise im Sumpf der Destillationskolonne.

### Verfahrensschritt (d)

Der in Verfahrensschritt (c) erhaltene Strom 5, der 3-Pentennitril und 2-Methyl-3-butennitril enthält, wird anschließend in eine weitere Destillationsvorrichtung überführt. In dieser Destillationsvorrichtung wird der Strom 5 in einen 3-Pentennitril-Strom, der als Sumpfprodukt entnommen wird, und einen 2-Methyl-3-butennitril-Strom, der am Kopf entnommen wird, aufgetrennt.

Der Verfahrensschritt (d) kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der absolute Druck in Verfahrensschritt (d) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt.

In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten

In einer besonders bevorzugten Ausführungsform des erfindungsgemäßen Verfahrens werden der Verfahrensschritt (d) und der Verfahrensschritt (g) in der gleichen Destillationsvorrichtung durchgeführt. Dabei fallen die die Ströme 6 und 12 sowie 7 und 13 zusammen. Ferner wird in dieser bevorzugten Ausführungsform der Strom 5 direkt in die gemeinsame Vorrichtung der Verfahrensschritte (d) und (g) geführt. Dabei können die Zulaufstellen der Ströme 5 und 11 im Falle einer Destillationskolonne als Destillationsvorrichtung gleich oder unterschiedlich sein.

In einer weiteren Ausführungsform des erfindungsgemäßen Verfahrens werden die Verfahrensschritte (c) und (g) in einer gemeinsamen Destillationskolonne durchgeführt, wobei der Verfahrensschritt (d) entfällt, der Strom 2 aus Verfahrensschritt (b) sowie Strom 11 aus Verfahrensschritt (f) in Verfahrensschritt (g) geführt werden, in Verfahrensschritt (g) der Strom 4 als Kopfprodukt, enthaltend (Z)-2-Methyl-2-butennitril; der Strom 12 als Sumpfprodukt, enthaltend 3-Pentennitril und der Strom 13 als Seitenabzugsstrom, enthaltend 2-Methyl-3-butennitril erhalten werden.

In dem erfindungsgemäßen Verfahren gemäß Ausführungsform I ist es möglich, dass der Strom 2 direkt in den Verfahrensschritt (g) zurückgeführt wird und der Eduktstrom direkt in den Verfahrensschritt (c) gefahren wird, wobei ein Strom 5a aus Verfahrensschritt (c) in die Isomerisierung von Verfahrensschritt (a) zurückgeführt wird.

Alternativ ist es auch möglich, den Strom 2 direkt in den Verfahrensschritt (g) zurückzuführen und den Eduktstrom in Verfahrensschritt (c) zu fahren, wobei der Strom 5 aus Verfahrensschritt (c) in den Verfahrensschritt (f) zurückgeführt wird.

Alternativ ist es auch möglich, dass der Strom 2 direkt in den Verfahrensschritt (g) zurückgeführt wird und der Eduktstrom in Verfahrensschritt (c) gefahren wird und der Strom 5 aus Verfahrensschritt (c) in den Verfahrensschritt (e) zurückgeführt wird.

### Ausführungsform II

Weiterer Gegenstand der vorliegenden Erfindung ist ein Verfahren zur Herstellung von 3-Pentennitril gemäß einer Ausführungsform II, das durch die folgenden Verfahrensschritte gekennzeichnet ist:
(a') Isomerisierung eines Eduktstroms, der 2-Methyl-3-butennitril enthält, an mindestens einem gelösten oder dispergierten Isomerisierungskatalysator zu einem Strom 1', der 3-Pentennitril, 2-Methyl-3-butennitril, den mindestens einen Isomerisierungskatalysator und (Z)-2-Methyl-2-butennitril enthält,
(b') Destillation des Stromes 1' unter Erhalt eines Stromes 2', der (Z)-2-Methyl-2-butennitril, 2-Methyl-3-butennitril enthält und in den lsomerisierungsschritt (a') zurückgeführt wird, eines Stromes 3' als Sumpfprodukt, der den mindestens einen Isomerisierungskatalysator enthält und in den lsomerisierungsschritt (a') zurückgeführt wird, und eines Stromes 4', der 3-Pentennitril enthält, an einem Seitenabzug der Destillationskolonne.

Der Eduktstrom, der in dem Verfahrensschritt (a') des erfindungsgemäßen Verfahrens gemäß Ausführungsform II verwendet wird, kann nach den oben beschriebenen Verfahren zur Herstellung des Eduktstroms für das erfindungsgemäße Verfahren gemäß Ausführungsform I erhalten werden.

Für den Verfahrensschritt (a') gemäß Ausführungsform II gelten die selben Bedingungenwie für Verfahrensschritt (a) gemäß Ausführungsform I, insbesondere hinsichtlich des verwendeten Katalysatorkomplexes und des freien Liganden.

Der absolute Druck in Verfahrensschritt (b') beträgt vorzugsweise 0,001 bis 100 bar, besonders bevorzugt 0,01 bis 20 bar, insbesondere 0,1 bis 2 bar. Die Destillation wird so durchgeführt, dass die Temperatur im Sumpf der Destillationsvorrichtung vorzugsweise 25 bis 250 °C, besonders bevorzugt 40 bis 180 °C, insbesondere 60 bis 40 °C, beträgt. Die Destillation wird so durchgeführt, dass die Kondensationstemperatur am Kopf der Destillationsvorrichtung vorzugsweise -50 bis 250 °C, besonders bevorzugt 0 bis 150 °C, insbesondere 10 bis 100 °C, beträgt.

Eine Teilausschleusung des Stromes 2' ist gegebenenfalls angezeigt, um Aufpegelung von (Z)-2-Methyl-2-butennitril zu vermeiden. Der Reststrom wird in Schritt (a') zurückgeführt.

In einer Variante des vorliegenden Verfahrens gemäß Ausführungsform II wird der Eduktstrom anstelle in Verfahrensschritt (a') in den Verfahrensschritt (b') geführt.

Der Strom 2', der in dem erfindungsgemäßen Verfahren gemäß Ausführungsform II den Verfahrensschritt (b') verlässt, kann gegebenenfalls in einem weiteren optionalen Verfahrensschritt (c') einer Destillation unterzogen werden. Dabei bildet sich vorzugsweise ein an (Z)-2-Methyl-2-butennitril angereichter Strom 5' und ein an (Z)-2-Methyl-2-butennitril abgereicherter Strom 6', wobei der Strom 5' vorzugsweise in den Verfahrensschritt (a') zurückgefahren wird.

Der gegebenenfalls durchzuführende Verfahrensschritt (c') kann auch in der Vorrichtung von Verfahrensschritt (a') durchgeführt werden, wobei dann im Verfahrensschritt (a') eine Destillationsvorrichtung verwendet wird, in deren Sumpf die lsomerisierungsreaktion stattfindet, über den Sumpf der Destillationsvorrichtung der Strom 1' abgezogen wird und über Kopf der Destillationsvorrichtung der an (Z)-2-Methyl-2-butennitril reiche Strom 6' abgezogen wird.

Erfindungsgemäß erhält man in den Verfahren gemäß Ausführungsform I und II 3-Pentennitril. Im Sinne der vorliegenden Erfindung werden unter dem Begriff 3-Pentennitril ein einziges Isomer von 3-Pentennitril oder ein Gemisch aus zwei, drei, vier oder fünf verschiedener solcher isomeren verstanden. Als Isomere kommen cis-2-Pentennitril, trans-2-Pentennitril, cis-3-Pentennitril, trans-3-Pentennitril, 4-Pentennitril oder deren Gemische, vorzugsweise cis-3-Pentennitril, trans-3-Pentennitril, 4-Pentennitril oder deren Gemische, die im Sinne der vorliegenden Erfindung sowohl jeweils einzeln, als auch im Gemisch als 3-Pentennitril bezeichnet werden, in Betracht.

Mit dem erfindungsgemäßen Verfahren sind Vorteile verbunden. So ist in einem integrierten Verfahren zur Herstellung von Adipodinitril beispielsweise die Rückführung von nicht umgesetztem 2-Methyl-3-butennitril aus der Isomerisierung wirtschaftlich notwendig, weil der Umsatzgrad von 2-Methyl-3-butennitril zu 3-Pentennitril durch das thermodynamische Gleichgewicht begrenzt ist. Die Rückführung erfordert, dass (Z)-2-Methyl-2-butennitril, das sich im 2-Methyl-3-butennitril-Kreislauf aufpegelt, abgetrennt wird. Die Abtrennung erfolgt im erfindungsgemäßen Verfahren durch Destillation zur Trennung von 2-Methyl-3-butennitril und (Z)-2-Methyl-2-butennitril vorzugsweise erst nach Durchführung von Schritt (a) in Schritt (c), um gezielt Verluste von Wertprodukten zu minimieren.

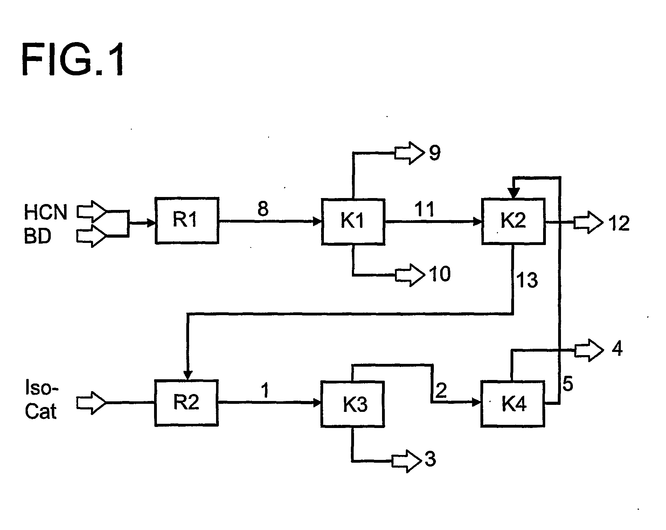

Das erfindungsgemäße Verfahren gemäß einer bevorzugten Ausprägung der Ausführungsform I wird anhand der Figur 1 näher erläutert:
In einem Reaktor R1 wird in Gegenwart eines Nickel(0)-Katalysators Cyanwasserstoff und 1,3-Butadien eingespeist. In dem Reaktor findet eine Hydrocyanierung unter Ausbildung eines Stromes 8 statt. Dieser Strom 8 enthält 3-Pentennitril, 2-Methyl-3-butennitril, den Hydrocanierungskatalysator und nicht umgesetztes 1,3-Butadien. Anschließend wird der Strom 8 in eine Destillationskolonne K1 überführt, in der über Kopf 1,3-Butadien (Strom 9) aus dem Strom 8 entfernt wird. Im Sumpf in der Destillationskolonne K1 wird ein Strom 10 gewonnen, der den Hydrocyanierungskatalysator enthält. Am Seitenabzug der Destillationskolonne K1 wird ein Strom 11 gewonnen, der 3-Pentennitril und 2-Methyl-3-butennitril enthält. Dieser Strom 11 wird anschließend in eine Destillationskolonne K2 überführt.

In der Destillationskolonne K2 erfolgt eine Trennung des Stromes 11 in einen Strom 12, der 3-Pentennitril enthält, und einen Strom 13, der 2-Methyl-3-butennitril enthält.

Der Strom 13 wird anschließend in eine Isomerisierungsapparatur R2 überführt. In dieser Isomerisierungsapparatur R2 erfolgt eine Isomerisierung des 2-Methyl-3-butennitrils, das in dem Strom 13 enthalten ist, an einem lsomerisierungskatalysator. Der aus dieser Isomerisierung stammende Strom 1 enthält 3-Pentennitril, 2-Methyl-3-butennitril, (Z)-2-Methyl-2-butennitril sowie den Isomerisierungskatalysator.

Dieser Strom 1 wird anschließend in einer Destillationsapparatur K3 aufgetrennt. Dabei bildet sich der Strom 3, der den lsomerisierungskatalysator enthält (Sumpf). Am Kopf der Destillationsapparatur K3 wird der Strom 2 entnommen. Dieser Strom 2 enthält 3-Pentennitril, (Z)-2-Methyl-2-butennitril und 2-Methyl-3-butennitril. Dieser Strom 2 wird, anschließend in eine Destillationskolonne K4 überführt.

In dieser Destillationskolonne K4 erfolgt eine Trennung des Stromes 2 in (Z)-2-Methyl-2-butennitril, das während der Isomerisierung gebildet wurde (Strom 4). Darüber hinaus wird in der Destillationskolonne K4 im Sumpf der Strom 5 erhalten, der 3-Pentenntril und 2-Methyl-3-butennitril enthält. Dieser Strom 5 wird in die Destillationskolonne K2 überführt, wobei aus dem Strom 5 in der Destillationskolonne das 3-Pentennitril gewonnen wird.

Die Ströme 9 und 10 können ganz, teilweise oder gar nicht in den Reaktor R1 zurückgeführt werden. Gleiches gilt für den Strom 3 in Richtung Reaktor R2. Diese Varianten sind nicht in Figur 1 abgebildet.

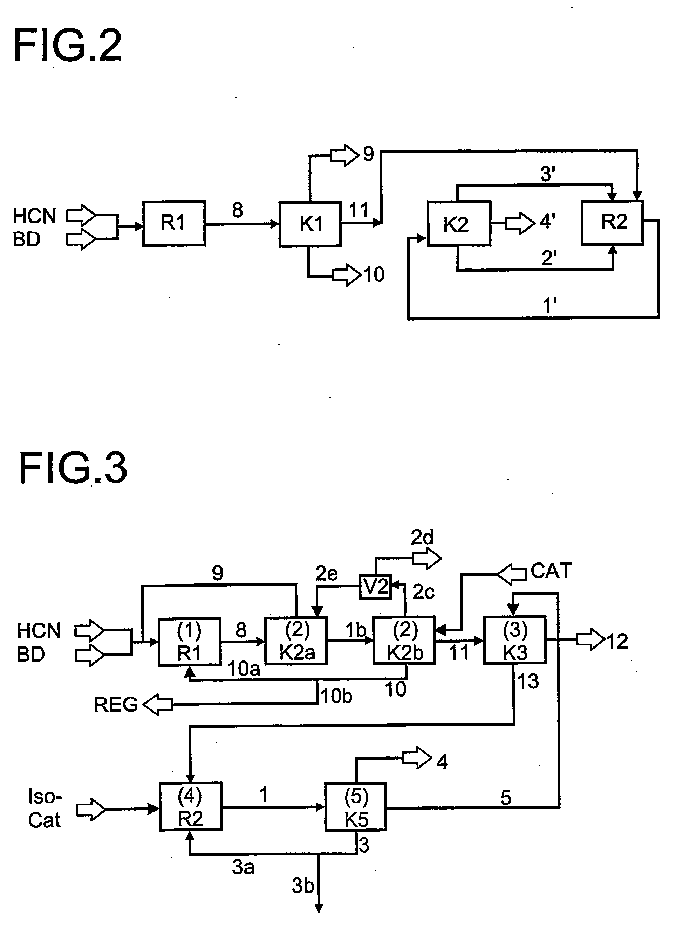

Das erfindungsgemäße Verfahren gemäß einer bevorzugten Ausprägung der Ausführungsform II wird anhand der Figur 2 näher erläutert:
In einem Reaktor R1 wird in Gegenwart eines Nickel(0)-Katalysators Cyanwasserstoff und 1,3-Butadien eingespeist. In dem Reaktor findet eine Hydrocyanierung unter Ausbildung eines Stromes 8 statt. Dieser Strom 8 enthält 3-Pentennitril, 2-Methyl-3-butennitril, den Hydrocanierungskatalysator und nicht umgesetztes 1,3-Butadien. Anschließend wird der Strom 8 in eine Destillationskolonne K1 überführt, in der über Kopf 1,3-Butadien aus dem Strom 8 entfernt wird (Strom 9). Im Sumpf in der Destillationskolonne K1 wird ein Strom 10 gewonnen, der den Hydrocyanierungskatalysator enthält. Am Seitenabzug der Destillationskolonne K1 wird ein Strom 11 gewonnen, der 3-Pentennitril und 2-Methyl-3-butennitril enthält. Dieser Strom 11 wird anschließend in eine lsomersierungsvorrichtung R2 überführt.

In die Isomerisierungsvorrichtung R2 werden zusätzlich lsomersisierungskatalysator (Strom 3') und 2-Methyl-3-butennitril (Strom 2'), jeweils aus dem Destillationskolonne K2 stammend, eingeführt. In der Isomerisierungsvorrichtung R2 findet eine Isomersierung statt. Der hieraus resultierende Strom 1' wird anschließend in die Destillationsvorrichtung K2 überführt, in welcher der Strom 1' aufgetrennt wird in einen Strom 2' (2-Methyl-3-butennitril), der in R2 zurückgeführt wird, einen Strom 3' (Isomerisierungskatalysator), der in R2 zurückgeführt wird, und in einen Strom 4', der 3-Pentennitril enthält.

Durch Zuführung eines lsomerisierungskatalysator enthaltenden Stromes zu R2 können gegebenenfalls notwendige Ausschleusungen aus dem Strom 3' ausgeglichen werden, so dass der Ni(0)-Gehalt im R2 konstant bleibt.

Die Ströme 9 und 10 können ganz, teilweise oder gar nicht in den Reaktor R1 zurückgeführt werden.

Diese Rückführungs- und Ausschleusungsvarianten sind in Figur 2 nicht abgebildet.

### Ausführungsform III

In der Ausführungsform III werden Hydrocyanierungs- und Isomerisierungs-Ni(0)-Katalysatoren von derartigen Liganden verwendet, die die Verfahrensschritte a*) und e*) katalysieren.

Bei den als Katalysator bevorzugt verwendeten Nickel(0)-Komplexen, die phosphorhaltige Liganden und/oder freie phosphorhaltige Liganden enthalten, handelt es sich bevorzugt um homogen gelöste Nickel(0)-Komplexe.

Die phosphorhaltigen Liganden der Nickel(0)-Komplexe und die freien phosphorhaltigen Liganden sind vorzugsweise ausgewählt aus der Gruppe der mono- oder bidentaten Phosphine, Phosphite, Phosphinite und Phosphonite, bevorzugt der mono- oder bidentaten Phosphite, Phosphinite und Phosphonite, besonders bevorzugt der mono- oder bidentaten Phosphite und Phosphonite, insbesondere der monodentaten Phosphite, Phosphinite und Phosphonite, ganz besonders bevorzugt der monodentaten Phosphite und Phosphonite.

Diese phosphorhaltigen Liganden weisen vorzugsweise die Formel I auf:

P (X¹R¹) (X²R²) (X³R³) (I)

Unter Verbindung I wird im Sinne der vorliegenden Erfindung eine einzelne Verbindung oder ein Gemisch verschiedener Verbindungen der vorgenannten Formel verstanden.

Erfindungsgemäß sind X¹, X², X³ unabhängig voneinander Sauerstoff oder Einzelbindung. Falls alle der Gruppen X¹, X² und X³ für Einzelbindungen stehen, so stellt Verbindung I ein Phosphin der Formel P(R¹R²R³) mit den für R¹, R² und R³ in dieser Beschreibung genannten Bedeutungen dar.

Falls zwei der Gruppen X¹, X² und X³ für Einzelbindungen stehen und eine für Sauerstoff, so stellt Verbindung I ein Phosphinit der Formel P(OR¹)(R²)(R³) oder P(R¹)(OR²)(R³) oder P(R¹)(R²)(OR³) mit den für R¹, R² und R³ weiter unten genannten Bedeutungen dar.

Falls eine der Gruppen X¹, X² und X³ für eine Einzelbindung steht und zwei für Sauerstoff, so stellt Verbindung I ein Phosphonit der Formel P(OR¹)(OR²)(R³) oder P(R¹)(OR²)(OR³) oder P(OR¹)(R²)(OR³) mit den für R¹, R² und R³ in dieser Beschreibung genannten Bedeutungen dar.

In einer Ausführungsform sollten alle der Gruppen X¹, X² und X³ für Sauerstoff stehen, so dass Verbindung I ein Phosphit der Formel P(OR¹)(OR²)(OR³) mit den für R¹, R² und R³ weiter unten genannten Bedeutungen darstellt.

Erfindungsgemäß stehen R¹, R², R³ unabhängig voneinander für gleiche oder unterschiedliche organische Reste. Als R¹, R² und R³ kommen unabhängig voneinander Alkylreste, vorzugsweise mit 1 bis 10 Kohlenstoffatomen, wie Methyl, Ethyl, n-Propyl, i-Propyl, n-Butyl, i-Butyl, s-Butyl, t-Butyl, Aryl-Gruppen, wie Phenyl, o-Tolyl, m-Tolyl, p-Tolyl, 1-Naphthyl, 2-Naphthyl, oder Hydrocarbyl, vorzugsweise mit 1 bis 20 Kohlenstoffatomen, wie 1,1'-Biphenol, 1,1'-Binaphthol in Betracht. Die Gruppen R¹, R² und R³ können miteinander direkt, also nicht allein über das zentrale Phosphor-Atom, verbunden sein. Vorzugsweise sind die Gruppen R¹, R² und R³ nicht miteinander direkt verbunden.

In einer Ausführungsform kommen als Gruppen R¹, R² und R³ Reste ausgewählt aus der Gruppe bestehend aus Phenyl, o-Tolyl, m-Tolyl und p-Tolyl in Betracht. In einer Ausführungsform sollten dabei maximal zwei der Gruppen R¹, R² und R³ Phenyl-Gruppen sein.

In einer anderen Ausführungsform sollten dabei maximal zwei der Gruppen R¹, R² und R³ o-Tolyl-Gruppen sein.

Als Verbindungen I können solche der Formel a

(o-Tolyl-O-)_{w}(m-Tolyl-O-)ₓ(p-Tolyl-O-)_{y}(Phenyl-O-)_{z} P (I a)

eingesetzt werden, wobei w, x, y und z eine natürliche Zahl bedeuten, und folgende Bedingungen gelten: w + x + y + z = 3 und w, z ≤2.

Solche VerbindungenIa sind z.B. (p-Tolyl-O-)(Phenyl-O-)₂P, (m-Tolyl-O-)(Phenyl-O-)₂P, (o-Tolyl-O-) (Phenyl-O-)₂P, (p-Tolyl-O-)₂(Phenyl-O-)P, (m-Tolyl-O-)₂(Phenyl-O-)P, (o-Tolyl-O-)₂(Phenyl-O-)P, (m-Tolyl-O-)(p-Tolyl-O)(Phenyl-O-)P, (o-Tolyl-O-)(p-Tolyl-O-)(Phenyl-O-)P, (o-Tolyl-O-)(m-Tolyl-O-)(Phenyl-O-)P, (p-Tolyl-O-)₃P, (m-Tolyl-O-)(p-Tolyl-O-)₂P, (o-Tolyl-O-)(p-Tolyl-O-)₂P, (m-Tolyl-O-)₂(p-Toluyl-O-)P, (o-Tolyl-O-)₂(p-Tolyl-O-)P, (o-Tolyl-O-)(m-Tolyl-O-)(p-Tolyl-O)P, (m-Tolyl-O-)₃P, (o-Tolyl-O-)(m-Tolyl-O-)₂P (o-Tolyl-O-)₂(m-Tolyl-O-)P, oder Gemische solcher Verbindungen.

Gemische enthaltend (m-Tolyl-O-)₃P, (m -Tolyl-O-)₂(p-Tofyl-O-)P, (m-Tolyl-O-)(p-Tolyl-O-)₂P und (p-Tolyl-O-)₃P kann man beispielsweise durch Umsetzung eines Gemisches enthaltend m-Kresol und p-Kresol, insbesondere im Molverhältnis 2 : 1, wie es bei der destillativen Aufarbeitung von Erdöl anfällt, mit einem Phosphortrihalogenid, wie Phosphortrichlorid, erhalten.

In einer Ausführungsform kommen als phosphorhaltige Liganden die in der DE-A 199 53 058 näher beschriebenen Phosphite der Formel Ib in Betracht:

P(O-R¹)ₓ(O-R²)_{y}(O-R³)_{z}(O-R⁴)ₚ (I b)

mit
- R¹:: aromatischer Rest mit einem C₁-C₁₈-Alkylsubstituenten in o-Stellung zu dem Sauerstoffatom, das das Phosphoratom mit dem aromatischen System verbindet, oder mit einem aromatischen Substituenten in o-Stellung zu dem Sauerstoffatom, das das Phosphoratom mit dem aromatischen System verbindet, oder mit einem in o-Stellung zu dem Sauerstoffatom, das das Phosphoratom mit dem aromati- schen System verbindet, anellierten aromatischen System,
- R²:: aromatischer Rest mit einem C₁-C₁₈-Alkylsubstituenten in m-Stellung zu dem Sauerstoffatom, das das Phosphoratom mit dem aromatischen System verbindet, oder mit einem aromatischen Substituenten in m-Stellung zu dem Sauerstoff- atom, das das Phosphoratom mit dem aromatischen System verbindet, oder mit einem in m-Stellung zu dem Sauerstoffatom, das das Phosphoratom mit dem a- romatischen System verbindet, anellierten aromatischen System, wobei der aro- matische Rest in o-Stellung zu dem Sauerstoffatom, das das Phosphoratom mit dem aromatischen System verbindet, ein Wasserstoffatom trägt
- R³:: aromatischer Rest mit einem C₁-C₁₈-Alkylsubstituenten in p-Stellung zu dem Sauerstoffatom, das das Phosphoratom mit dem aromatischen System verbindet, oder mit einem aromatischen Substituenten in p-Stellung zu dem Sauerstoffatom, das das Phosphoratom mit dem aromatischen System verbindet, wobei der aro- matische Rest in o-Stellung zu dem Sauerstoffatom, das das Phosphoratom mit dem aromatischen System verbindet, ein Wasserstoffatom trägt,
- R⁴:: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aromatische Rest in o-Stellung zu dem Sauerstoffatom, das das Phosphoratom mit dem aromatischen System verbindet, ein Wasserstoffatom trägt,
- x :: 1 oder 2,
- y, z, p:: unabhängig voneinander 0, 1 oder 2 mit der Maßgabe, dass x+y+z+p = 3.

Bevorzugte Phosphite der Formel Ib sind der DE-A 199 53 058 zu entnehmen. Als Rest R¹ kommen vorteilhaft o-Tolyl-, o-Ethyl-phenyl-, o-n-Propyl-phenyl-, o-Isopropyl-phenyl-, o-n-Butyl-phenyl-, o-sek-Butyl-phenyl-, o-tert-Butyl-phenyl-, (o-Phenyl)-Phenyl- oder 1-Naphthyl- Gruppen in Betracht.

Als Rest R² sind m-Tolyl-, m-Ethyl-phenyl-, m-n-Propyl-phenyl-, m-Isopropyl-phenyl-, m-n-Butyl-phenyl-, m-sek-Butyl-phenyl-, m-tert-Butyl-phenyl-, (m-Phenyl)-Phenyl- oder 2-Naphthyl- Gruppen bevorzugt.

Als Rest R³ kommen vorteilhaft p-Tolyl-, p-Ethyl-phenyl-, p-n-Propyl-phenyl-, p-Isopropyl-phenyl-, p-n-Butyl-phenyl-, p-sek-Butyl-phenyl-, p-tert-Butyl-phenyl- oder (p-Phenyl)-Phenyl-Gruppen in Betracht.

Rest R⁴ ist bevorzugt Phenyl. Vorzugsweise ist p gleich null. Für die Indizes x, y, z und p in Verbindung Ib ergeben sich folgende Möglichkeiten:

| | | | |
|---|---|---|---|
| x | y | z | p |
| 1 | 0 | 0 | 2 |
| 1 | 0 | 1 | 1 |
| 1 | 1 | 0 | 1 |
| 2 | 0 | 0 | 1 |
| 1 | 0 | 2 | 0 |
| 1 | 1 | 1 | 0 |
| 1 | 2 | 0 | 0 |
| 2 | 0 | 1 | 0 |
| 2 | 1 | 0 | 0 |

Bevorzugte Phosphite der Formel Ib sind solche, in denen p gleich null ist sowie R¹, R² und R³ unabhängig voneinander ausgewählt sind aus o-Isopropyl-phenyl, m-Tolyl und p-Tolyl, und R⁴ Phenyl ist.

Besonders bevorzugte Phosphite der Formel Ib sind solche, in denen R¹ der o-Isopropyl-phenyl-Rest, R² der m-Tolylrest und R³ der p-Tolylrest ist mit den in der vorstehenden Tabelle genannten Indizes; außerdem solche, in denen R¹ der o-Tolylrest, R² der m-Tolylrest und R³ der p-Tolylrest ist mit den in der Tabelle genannten Indizes; weiterhin solche, in denen R¹ der 1-Naphthylrest, R² der m-Tolylrest und R³ der p-Tolylrest ist mit den in der Tabelle genannten Indizes; außerdem solche, in denen R¹ der o-Tolylrest, R² der 2-Naphthylrest und R³ der p-Tolylrest ist mit den in der Tabelle genannten Indizes; und schließlich solche, in denen R¹ der o-lsopropyl-phenyl-Rest, R² der 2-Naphthylrest und R³ der p-Tolylrest ist mit den in der Tabelle genannten Indizes; sowie Gemische dieser Phosphite.

Phosphite der Formel Ib können erhalten werden, indem man
a) ein Phosphortrihalogenid mit einem Alkohol ausgewählt aus der Gruppe bestehend aus R¹OH, R²OH, R³OH und R⁴OH oder deren Gemische umsetzt unter Erhalt eines Dihalogenophosphorigsäuremonoesters,
b) den genannten Dihalogenophosphorigsäuremonoester mit einem Alkohol ausgewählt aus der Gruppe bestehend aus R¹OH, R²OH, R³OH und R⁴OH oder deren Gemische umsetzt unter Erhalt eines Monohalogenophosphorigsäurediesters und
c) den genannten Monohalogenophosphorigsäurediester mit einem Alkohol ausgewählt aus der Gruppe bestehend aus R¹OH, R²OH , R³OH und R⁴OH oder deren Gemische umsetzt unter Erhalt eines Phosphits der Formel Ib.

Die Umsetzung kann in drei getrennten Schritten durchgeführt werden. Ebenso können zwei der drei Schritte kombiniert werden, also a) mit b) oder b) mit c). Alternativ können alle der Schritte a), b) und c) miteinander kombiniert werden.

Dabei kann man geeignete Parameter und Mengen der Alkohole ausgewählt aus der Gruppe bestehend aus R¹OH, R²OH, R³OH und R⁴OH oder deren Gemische durch einige einfache Vorversuche leicht ermitteln.

Als Phosphortrihalogenid kommen grundsätzlich alle Phosphortrihalogenide, vorzugsweise solche, in denen als Halogenid Cl, Br, I, insbesondere Cl, eingesetzt wird, sowie deren Gemische in Betracht. Es können auch Gemische verschiedener gleich oder unterschiedlich halogensubstituierter Phosphine als Phosphortrihalogenid eingesetzt werden. Besonders bevorzugt ist PCI₃. Weitere Einzelheiten zu den Reaktionsbedingungen bei der Herstellung der Phosphite I b und zur Aufarbeitung sind der DE-A 199 53 058 zu entnehmen.

Die Phosphite I b können auch in Form eines Gemisches verschiedener Phosphite I b als Ligand verwendet werden. Ein solches Gemisch kann beispielsweise bei der Herstellung der Phosphite I b anfallen.

Es ist ebenfalls möglich, dass der phosphorhaltige Ligand mehrzähnig, insbesondere zweizähnig ist. Dann weist der verwendete Ligand beispielsweise die Formel II 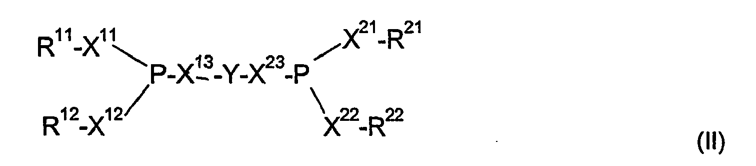 auf, worin bedeuten
- X¹¹, X¹², X¹³, X²¹, X²², X²³: unabhängig voneinander Sauerstoff oder Einzelbindung
- R¹¹, R¹²: unabhängig voneinander gleiche oder unterschiedliche, einzelne oder verbrückte organische Reste
- R²¹, R²²: unabhängig voneinander gleiche oder unterschiedliche, einzelne oder verbrückte organische Reste,
- Y: Brückengruppe.

Unter Verbindung II wird im Sinne der vorliegenden Erfindung eine einzelne Verbindung oder ein Gemisch verschiedener Verbindungen der vorgenannten Formel verstanden.

In einer Ausführungsform können X¹¹, X¹², X¹³, X²¹, X²², X²³ Sauerstoff darstellen. In einem solchen Fall ist die Brückengruppe Y mit Phosphit-Gruppen verknüpft.

In einer anderen Ausführungsform können X¹¹ und X¹² Sauerstoff und X¹³ eine Einzelbindung oder X¹¹ und X¹³ Sauerstoff und X¹² eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²¹ und X²² Sauerstoff und X²³ eine Einzelbindung oder X²¹ und X²³ Sauerstoff und X²² eine Einzelbindung oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphonits, Phosphinits oder Phosphins, vorzugsweise eines Phosphonits, sein kann.

In einer anderen Ausführungsform können X¹³ Sauerstoff und X¹¹ und X¹² eine Einzelbindung oder X¹¹ Sauerstoff und X¹² und X¹³ eine Einzelbindung darstellen, so dass das mit X¹¹, X¹² und X¹³ umgebene Phosphoratom Zentralatom eines Phosphonits ist. In einem solchen Fall können X²¹, X²² und X²³ Sauerstoff oder X²³ Sauerstoff und X²¹ und X²² eine Einzelbindung oder X²¹ Sauerstoff und X²² und X²³ eine Einzelbindung oder X²¹, X²² und X²³ eine Einzelbindung darstellen, so dass das mit X²¹, X²² und X²³ umgebene Phosphoratom Zentralatom eines Phosphits, Phosphinits oder Phosphins, vorzugsweise eines Phosphinits, sein kann.

In einer anderen Ausführungsform können X¹¹, X¹² und X¹³ eine Einzelbindung darstellen, so dass das mit X¹¹, X¹² und X¹³ umgebene Phosphoratom Zentralatom eines Phosphins ist. In einem solchen Fall können X²¹, X²² und X²³ Sauerstoff oder X²¹, X²² und X²³ eine Einzelbindung darstellen, so dass das mit X²¹, X²² und X²³ umgebene Phosphoratom Zentralatom eines Phosphits oder Phosphins, vorzugsweise eines Phosphins, sein kann.

Als Brückengruppe Y kommen vorzugsweise substituierte, beispielsweise mit C₁-C₄-Alkyl, Halogen, wie Fluor, Chlor, Brom, halogeniertem Alkyl, wie Trifluormethyl, Aryl, wie Phenyl, oder unsubstituerte Arylgruppen in Betracht, vorzugsweise solche mit 6 bis 20 Kohlenstoffatomen im aromatischen System, insbesondere Pyrocatechol, Bis(phenol) oder Bis(naphthol).

Die Reste R¹¹ und R¹² können unabhängig voneinander gleiche oder unterschiedliche organische Reste darstellen. Vorteilhaft kommen als Reste R¹¹ und R¹² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R²¹ und R²² können unabhängig voneinander gleiche oder unterscheidliche organische Reste darstellen. Vorteilhaft kommen als Reste R²¹ und R²² Arylreste, vorzugsweise solche mit 6 bis 10 Kohlenstoffatomen, in Betracht, die unsubstituiert oder einfach oder mehrfach substituiert sein können, insbesondere durch C₁-C₄-Alkyl, Halogen, wie Fluor, Chlor, Brom, halogeniertem Alkyl, wie Trifluormethyl, Aryl, wie Phenyl, oder unsubstituierte Arylgruppen.

Die Reste R¹¹ und R¹² können einzeln oder verbrückt sein. Auch die Reste R²¹ und R²² können einzeln oder verbrückt sein. Die Reste R¹¹, R¹², R²¹ und R²² können alle einzeln, zwei verbrückt und zwei einzeln oder alle vier verbrückt sein in der beschriebenen Art.

In einer Ausführungsform kommen die in US 5,723,641 genannten Verbindungen der Formel I, II, III, IV und V in Betracht. In einer Ausführungsform kommen die in US 5,512,696 genannten Verbindungen der Formel I, II, III IV, V, VI und VII, die dort in den Beispielen 1 bis 31 eingesetzten Verbindungen, in Betracht. In einer Ausführungsform kommen die in US 5,821,378 genannten Verbindungen der Formel I, II, III, IV, V, VI, VII, VIII, IX, X, XI, XII, XIII, XIV und XV, die dort in den Beispielen 1 bis 73 eingesetzten Verbindungen, in Betracht.

In einer Ausführungsform kommen die in US 5,512,695 genannten Verbindungen der Formel I, II, III, IV, V und VI, die dort in den Beispielen 1 bis 6 eingesetzten Verbindungen, in Betracht. In einer Ausführungsform kommen die in US 5,981,772 genannten Verbindungen der Formel I, II, III, IV, V, VI, VII, VIII, IX, X, XI, XII, XIII und XIV, die dort in den Beispielen 1 bis 66 eingesetzten Verbindungen, in Betracht.

In einer Ausführungsform kommen die in US 6,127,567 genannten Verbindungen und dort in den Beispielen 1 bis 29 eingesetzten Verbindungen in Betracht. In einer Ausführungsform kommen die in US 6,020,516 genannten Verbindungen der Formel I, II, III, IV, V, VI, VII, VIII, IX und X, die dort in den Beispielen 1 bis 33 eingesetzten Verbindungen, in Betracht. In einer Ausführungsform kommen die in US 5,959,135 genannten Verbindungen und dort in den Beispielen 1 bis 13 eingesetzten Verbindungen in Betracht.

In einer Ausführungsform kommen die in US 5,847,191 genannten Verbindungen der Formel I, II und III in Betracht. In einer Ausführungsform kommen die in US 5,523,453 genannten Verbindungen, die dort in Formel 1, 2, 3, 4, 5, 6, 7, 8, 9, 10, 11, 12, 13, 14, 15, 16, 17, 18, 19, 20 und 21 dargestellten Verbindungen, in Betracht. In einer Ausführungsform kommen die in WO 01/14392 genannten Verbindungen, die dort in Formel V, VI, VII, VIII, IX, X, XI, XII, XIII, XIV, XV, XVI, XVII, XXI, XXII, XXIII dargestellten Verbindungen, in Betracht.

In einer Ausführungsform kommen die in WO 98/27054 genannten Verbindungen in Betracht. In einer Ausführungsform kommen die in WO 99/13983 genannten Verbindungen in Betracht. In einer Ausführungsform kommen die in WO 99/64155 genannten Verbindungen in Betracht.

In einer Ausführungsform kommen die in der deutschen Patentanmeldung DE 100 380 37 genannten Verbindungen in Betracht. In einer Ausführungsform kommen die in der deutschen Patentanmeldung DE 100 460 25 genannten Verbindungen in Betracht. In einer Ausführungsform kommen die in der deutschen Patentanmeldung DE 101 502 85 genannten Verbindungen in Betracht.

In einer Ausführungsform kommen die in der deutschen Patentanmeldung DE 101 502 86 genannten Verbindungen in Betracht. In einer Ausführungsform kommen die in der deutschen Patentanmeldung DE 102 071 65 genannten Verbindungen in Betracht. In einer weiteren Ausführungsform der vorliegenden Erfindung kommen die in der US 2003/0100442 A1 genannten phosphorhaltigen Chelatliganden in Betracht.

In einer weiteren Ausführungsform der vorliegenden Erfindung kommen die in der nicht vorveröffentlichten deutschen Patentanmeldung Aktenzeichen DE 103 50 999.2 vom 30.10.2003 genannten phosphorhaltigen Chelatliganden in Betracht.

Die beschriebenen Verbindungen I, I a, I b und II.sowie deren Herstellung sind an sich bekannt. Als phosphorhaltiger Ligand können auch Mischungen, enthaltend mindestens zwei der Verbindungen I, I a, I b und II, eingesetzt werden.

In einer besonders bevorzugten Ausführungsform des erfindungsgemäßen Verfahrens ist der phosphorhaltige Ligand des Nickel(0)-Komplexes und/oder der freie phosphorhaltige Ligand ausgewählt aus den Phosphiten der Formel I b

P (O-R¹)ₓ(O-R²)_{y}(O-R³)_{z}(O-R⁴)ₚ (I b)

worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen, also Gemische von 2 oder mehreren, bevorzugt 2 bis 10, besonders bevorzugt 2 bis 6 der Verbindungen der Formel Ib.

Das Verfahren ist in einer Ausführungsform III durch die folgenden Verfahrensschritte gekennzeichnet:
(a*) Isomerisierung eines Eduktstroms, der 2-Methyl-3-butennitril enthält, an mindestens einem gelösten oder dispergierten Isomerisierungskatalysator zu einem Strom 1, der den mindestens einen Isomerisierungskatalysator, 2-Methyl-3-butennitril, 3-Pentennitril und (Z)-2-Methyl-2-butennitril enthält,
(b*) Destillation des Stromes 1 unter Erhalt eines Stromes 2 als Kopfprodukt, der 2-Methyl-3-butennitril, 3-Pentennitril, und (Z)-2-Methyl-2-butennitril enthält, und eines Stromes 3 als Sumpfprodukt, der den mindestens einen Isomerisierungskatalysator enthält,
(c*) Destillation des Stromes 2 unter Erhalt eines Stromes 4 als Kopfprodukt, der gegenüber dem Strom 2 an (Z)-2-Methyl-2-butennitril, bezogen auf die Summe aller Pentennitrile im Strom 2, angereichert ist, und eines Stromes 5 als Sumpfprodukt, der gegenüber dem Strom 2 an 3-Pentennitril und 2-Methyl-3-butennitril, bezogen auf die Summe aller Pentennitrile im Strom 2, angereichert ist,
(d*) Destillation des Stromes 5 unter Erhalt eines Stromes 6 als Sumpfprodukt, der 3-Pentennitril enthält, und eines Stromes 7 als Kopfprodukt, der 2-Methyl-3-butennitril enthält.
(h*) Katalysatorregenerierung zur Aufstockung der Nickel(O)-Gehaltes der Teilströme 14 aus Strom 3 bzw. 16 aus Strom 10 unter Erzeugung eunes Stroms 18,
(i*) gegebenenfalls unter Zusatz eines Verdünnungsmittels F zu Strom 18 unter Erzeugung von Strom 19,
(j*) Extraktion des Stromes 18, ggf Stromes 19, bezüglich der Katalysatorkomponenten und/oder Störkomponente(n) durch Zusatz eines Dinitrilstroms 20 und eines Kohlenwasserstoffstroms 21 unter Erzeugung zweier nichtmischbarer Phasen 22 und 23, wobei Strom 22 den überwiegenden Teil der Katalysatorkomponenten und Strom 23 den überwiegenden Teil der Störkomponente(n) enthält,
(k*) destillative Abtrennung des Kohlenwasserstoffes von den Katalysatorkomponenten aus Strom 22 unter Erzeugung eines Stroms 25 , der den überwiegenden Teil der Katalysatorkomponenten enthält und ggf. teilweise oder ganze Rückführung des Stroms 25 in die Verfahrensschritte (a*) oder (e*).

### Eduktstrom

In Verfahrensschritt (a*) findet eine Isomerisierung eines Eduktstromes, der 2-Methyl-3-butennitril enthält, an mindestens einem Isomerisierungskatalysator statt.

In einer besonderen Ausführungsform des erfindungsgemäßen Verfahrens ist der Eduktstrom durch folgende Verfahrensschritte erhältlich:
(e*) Hydrocyanierung von 1,3-Butadien an mindestens einem Hydrocyanierungskatalysator mit Cyanwasserstoff unter Erhalt eines Stromes 8, der den mindestens einen Hydrocyanierungskatalysator, 3-Pentennitril, 2-Methyl-3-butennitril, 1,3-Butadien und Reste Cyanwasserstoff enthält,
(f*) ein- oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält,
(g*) Destillation des Stromes 11 unter Erhalt eines Stromes 12 als Sumpfprodukt, der 3-Pentennitril enthält, und eines Stromes 13 als Kopfprodukt, der 2-Methyl-3-butennitril enthält.

### Verfahrensschritt e*)

In Verfahrensschritt (e*) findet zur Herstellung des Eduktstroms zunächst eine Hydrocyanierung von 1,3-Butadien an mindestens einem Hydrocyanierungskatalysator mit Cyanwasserstoff statt unter Erhalt eines Stromes 8, der den mindestens einen Hydrocyanierungskatalysator, 3-Pentennitril, 2-Methyl-3-butennitril und nicht umgesetztes 1,3-Butadien enthält.

Der Verfahrensschritt (e*) kann in jeder geeigneten, dem Fachmann bekannten Apparatur durchgeführt werden. Für die Reaktion kommen somit übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren, jeweils gegebenenfalls mit Vorrichtungen zur Abfuhr von Reaktionswärme. Die Reaktion kann in mehreren, wie zwei oder drei, Apparaten durchgeführt werden.

In einer bevorzugten Ausführungsform des erfindungsgemäßen Verfahrens haben sich Reaktoren mit Rückvermischungscharakteristik oder Kaskaden von Reaktoren mit Rückvermischungscharakteristik als vorteilhaft erwiesen. Als besonders vorteilhaft haben sich Kaskaden aus Reaktoren mit Rückvermischungscharakteristik erwiesen, die in Bezug auf die Dosierung von Cyanwasserstoff in Querstromfahrweise betrieben werden.

Die Hydrocyanierung kann in Gegenwart oder in Abwesenheit von einem Lösemittel durchgeführt werden. Wenn ein Lösemittel verwendet wird, so sollte das Lösemittel bei der gegebenen Reaktionstemperatur und dem gegebenen Reaktionsdruck flüssig und inert gegenüber den ungesättigten Verbindungen und dem mindestens einenoKatalysator sein. Im Allgemeinen werden als Lösemittel Kohlenwasserstoffe, beispielsweise Benzol oder Xylol, oder Nitrile, beispielsweise Acetonitril oder Benzonitril, verwendet. Vorzugsweise wird allerdings ein Ligand als Lösemittel verwendet.

Die Reaktion kann in Batchfahrweise, kontinuierlich oder im Semibatchbetrieb durchgeführt werden.

Die Hydrocyanierungsreaktion kann durchgeführt werden, indem die Vorrichtung mit allen Reaktanten bestückt wird. Bevorzugt ist allerdings, wenn die Vorrichtung mit dem Katalysator, der ungesättigten organischen Verbindung und gegebenenfalls dem Lösemittel gefüllt wird. Vorzugsweise schwebt der gasförmige Cyanwasserstoff über der Oberfläche der Reaktionsmischung oder wird durch die Reaktionsmischung geleitet. Eine weitere Verfahrensweise zum Bestücken der Vorrichtung ist das Befüllen der Vorrichtung mit dem Katalysator, Cyanwasserstoff und gegebenenfalls dem Lösemittel und das langsame Zuspeisen der ungesättigten Verbindung zu der Reaktionsmischung. Alternativ ist auch möglich, dass die Reaktanten in den Reaktor eingeführt werden und die Reaktionsmischung auf die Reaktionstemperatur gebracht wird, bei welcher der Cyanwasserstoff flüssig zu der Mischung gegeben wird. Darüber hinaus kann der Cyanwasserstoff auch vor dem Erwärmen auf Reaktionstemperatur zugegeben werden. Die Reaktion wird unter konventionellen Hydrocyanierungsbedingungen für Temperatur, Atmosphäre, Reaktionszeit, etc. durchgeführt.

Vorzugsweise wird die Hydrocyanierung kontinuierlich in einem oder mehreren gerührten Verfahrensschritten durchgeführt. Wenn eine Mehrzahl von Verfahrensschritten verwendet wird, so ist es bevorzugt, dass die Verfahrensschritte in Serie geschaltet sind. Dabei wird das Produkt von einem Verfahrensschritt direkt in den nächsten Verfahrensschritt überführt. Der Cyanwasserstoff kann direkt in den ersten Verfahrensschritt oder zwischen den einzelnen Verfahrensschritten zugeführt werden.

Wenn das erfindungsgemäße Verfahren im Semibatchbetrieb durchgeführt wird, so ist es bevorzugt, dass im Reaktor die Katalysatorkomponenten und 1,3-Butadien vorgelegt werden, während Cyanwasserstoff über die Reaktionszeit hinweg in die Reaktionsmischung dosiert wird.

Die Reaktion wird vorzugsweise bei absoluten Drücken von 0,1 bis 500 MPa, besonders bevorzugt 0,5 bis 50 MPa, insbesondere 1 bis 5 MPa, durchgeführt. Die Reaktion wird vorzugsweise bei Temperaturen von 273 bis 473 K, besonders bevorzugt 313 bis 423 K, insbesondere bei 333 bis 393 K, durchgeführt. Dabei haben sich durchschnittliche mittlere Verweilzeiten der flüssigen Reaktorphase im Bereich von 0,001 bis 100 Stunden, vorzugsweise 0,05 bis 20 Stunden, besonders bevorzugt 0,1 bis 5 Stunden, jeweils pro Reaktor, als vorteilhaft erwiesen.

Die Reaktion kann in einer Ausführungsform in flüssiger Phase in Gegenwart einer Gasphase und gegebenenfalls einer festen suspendierten Phase ausgeführt werden. Dabei können die Ausgangsstoffe Cyanwasserstoff und 1,3-Butadien jeweils flüssig oder gasförmig zudosiert werden.

Die Reaktion kann in einer weiteren Ausführungsform in flüssiger Phase durchgeführt werden, wobei der Druck im Reaktor so bemessen ist, dass alle Einsatzstoffe wie 1,3-Butadien, Cyanwasserstoff und der mindestens eine Katalysator flüssig zudosiert werden und in der Reaktionsmischung in flüssiger Phase vorliegen. Dabei kann eine feste suspendierte Phase im Reaktionsgemisch vorliegen, die auch zusammen mit dem mindestens einen Katalysator zudosiert werden kann, beispielsweise bestehend aus Abbauprodukten des Katalysatorsystems, enthaltend unter anderem Nickel(II)-Verbindungen.

Im Verfahrensschritt (e*) wird ein Strom 8, der 3-Pentennitril, 2-Methyl-3-butennitril, den mindestens eine Katalysator und nicht umgesetztes 1,3-Butadien enthält, erhalten.

### Verfahrensschritt f*)

Der Strom 8, der 3-Pentennitril, 2-Methyl-3-butennitril, den mindestens einen Katalysator und nicht umgesetztes 1,3-Butadien enthält, wird anschließend in Verfahrensschritt (f*) in eine Destillationsvorrichtung überführt, In dieser Destillationsvorrichtung erfolgt eine einfache oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält.

Die Destillation des Verfahrensschrittes (f*) kann zweistufig erfolgen, wie in der DE-A-102 004 004 720, Verfahrensschritte (b*) und (c*), beschrieben. Die Destillation des Verfahrensschrittes (f*) kann auch gemäß der DE-A-102 004 004729, Verfahrensschritte (b*) und (c*) erfolgen.

Die Destillation(en) des Verfahrensschrittes (f*) kann (können) in jeder geeigneten, dem Fachmann bekannten Apparatur durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die einzelnen Destillationen kann man jeweils in mehreren, wie zwei oder drei Apparaturen, vorteilhaft in jeweils einer einzigen Apparatur durchführen.

Die Destillation(en) kann/können zudem jeweils einstufig im Sinne einer Teilverdampfung des Zulaufstroms erfolgen.

Der Druck in Verfahrensschritt (f*) beträgt vorzugsweise 0,001 bis 10 bar, besonders bevorzugt 0,010 bis 1 bar, insbesondere 0,02 bis 0,5 bar. Die Destillation(en) wird/werden so durchgeführt, dass die Temperatur(en) im Sumpf der Destillationsvorrichtung(en) vorzugsweise 30 bis 200 °C, besonders bevorzugt 50 bis 150 °C, insbesondere 60 bis 120 °C, beträgt / betragen. Die Destillation(en) wird/werden so durchgeführt, dass die Kondensationstemperaturen am Kopf der Destillationsvorrichtung vorzugsweise -50 bis 150 °C, besonders bevorzugt -15 bis 60 °C, insbesondere 5 bis 45°C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung(en) eingehalten.

Der Strom 11 wird anschließend in einem weiteren Verfahrensschritt (g*) einer Destillation unterzogen. Diese Destillation kann in jeder geeigneten, dem Fachmann bekannten Apparatur durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Druck in Verfahrensschritt (g*) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250°C, besonders bevorzugt 50 bis 200°C, insbesondere 60 bis 180°C, beträgt. Die Destillation wird so durchgeführt, dass die Kondensationstemperatur am Kopf der Destillationsvorrichtung vorzugsweise -50 bis 250°C, besonders bevorzugt 0 bis 180°C, insbesondere 15 bis 160°C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

Im Verfahrensschritt (g*) wird ein Strom 12 als Sumpfprodukt, der 1,3-Pentennitril enthält, und Strom 13 als Kopfprodukt, der 2-Methyl-3-butennitril enthält, erhalten. Der Strom 13 wird vorzugsweise als Eduktstrom in dem erfindungsgemäßen Verfahren zur Herstellung von 3-Pentennitril verwendet.

In einer weiteren Ausführungsform der vorliegenden Erfindung ist es möglich, dass der in dem erfindungsgemäßen Verfahrensschritt (a*) verwendete Eduktstrom dem Strom 11 des Verfahrensschrittes (f*) entspricht, so dass auf eine Auftrennung des Stromes 11 in dem Verfahrensschritt (g*) verzichtet wird.

### Verfahrensschritt (a*)

In Verfahrensschritt (a*) findet eine Isomerisierung des Eduktstromes, der 2-Methyl-3-butennitril enthält, an mindestens einem Isomerisierungskatalysator statt. Dabei wird ein Strom 1, der den Isomerisierungskatalysator, nicht umgesetztes 2-Methyl-3-butennitril, 3-Pentennitril und (Z)-2-Methyl-2-butennitril enthält, erhalten.

Erfindungsgemäß führt man die Isomerisierung in Gegenwart eines Systems, enthaltend
- Nickel(0) und
- eine Nickel(0) als Ligand komplexierende, dreibindigen Phosphor enthaltende Verbindung
durch.

Die Herstellung von Nickel(0) enthaltenden Katalysatorsystemen kann nach an sich bekannten Verfahren erfolgen.

Als Liganden für den Isomerisierungskatalysator werden die gleichen phosphorhaltigen Liganden wie für den in Verfahrensschritt (e*) verwendeten Hydrocyanierungskatalysator verwendet. Somit ist der Hydrocyanierungskatalysator identisch zu dem Isomerisierungskatalysator.

Der Katalysator in den Verfahrensschritten (a*) und (e*) ist im wesentlichen Lewis-Säure frei, d.h. es wird dem Katalysator zu keiner Zeit Lewis-Säure zugegeben, bevorzugt enthält der Katalysator keine Lewis-Säure.

Unter Lewis-Säure versteht man hierbei anorganische oder organische Metall-Verbindungen, in denen das Kation ausgewählt ist aus der Gruppe, bestehend aus Scandium, Titan, Vanadium, Chrom, Mangan, Eisen, Kobalt, Kupfer, Zink, Bor, Aluminium, Yttrium, Zirkonium, Niob, Molybdän, Cadmium, Rhenium und Zinn. Beispiele sind ZnBr₂, ZnI₂, ZnCl₂, ZnSO₄, CuCl₂, CuCI, Cu(O₃SCF₃)₂, CoCl₂, Col₂, Fel₂, FeCl₃, FeCl₂, FeCI₂(THF)₂, TCl₄(THF)₂, TiCl₄, TBCl₃, ClTi(O-i-Propyl)₃, MnCl₂, ScCl₃, AlCl₃, (C₈H₁₇)AlCl₂, (C₈H₁₇)₂AlCl, (i-C₄H₉)₂AlCl, (C₆H₅)₂AlCl, (C₆H₅)AlCl₂, ReCl₅, ZrCl₄, NbCl₅, VCl₃, CrCl₂, MoCl₅, YCl₃, CdCl₂, LaCl₃, Er(O₃SCF₃)₃, Yb(O₂CCF₃)₃, SmCl₃, B(C₆H₅)₃, TaCl₅, RAlCl₂, R₂AlCl, RSnO₃SCF₃ und R₃B, wobei R eine Alkyl- oder Aryl-Gruppe ist, B(C₆H₅)₃ und (C₆Hₛ)₃SnX, mit X=CF₃SO₃, CH₃C₆H₄SO₃ oder (C₆H₅)₃BCN wie beispielsweise in US 6,127,567, US 6,171,996, US 6,380,421, US 3,496,217, US 3,496,218, US 4,774,353, US 3,773,809, US 3,496,217 und US 4,874,884 beschrieben.

Die Isomerisierung kann in Gegenwart eines flüssigen Verdünnungsmittels,
- beispielsweise eines Kohlenwasserstoffs, wie Hexan, Heptan, Oktan, Cyclohexan, Methylcyclohexan, Benzol, Decahydronaphthalin
- beispielsweise eines Ethers, wie Diethylether, Tetrahydrofuran, Dioxan, Glykoldimethylether, Anisol,
- beispielsweise eines Esters, wie Ethylacetat, Methylbenzoat, oder
- beispielsweise eines Nitrils, wie Acetonitril, Benzonitril, oder
- Gemischen solcher Verdünnungsmittel durchgeführt werden.

In einer besonders bevorzugten Ausführungsform kommt eine Isomerisierung in Abwesenheit eines solchen flüssigen Verdünnungsmittels in Betracht.

Weiterhin hat es sich als vorteilhaft erwiesen, wenn die Isomerisierung in Verfahrensschritt (a*) in nicht-oxidierend wirkender Atmosphäre, wie beispielsweise unter einer Schutzgasatmosphäre aus Stickstoff oder einem Edelgas, wie Argon, durchgeführt wird.

Der Verfahrensschritt (a*) kann in jeder geeigneten, dem Fachmann bekannten Apparatur durchgeführt werden. Für die Reaktion kommen hierfür übliche Apparaturen in Betracht, wie sie beispielsweise in: Kirk-Othmer, Encyclopedia of Chemical Technology, 4. Ed., Vol. 20, John Wiley & Sons, New York, 1996, Seiten 1040 bis 1055 beschrieben sind, wie Rührkesselreaktoren, Schlaufenreaktoren, Gasumlaufreaktoren, Blasensäulenreaktoren oder Rohrreaktoren. Die Reaktion kann in mehreren, wie zwei oder drei Apparaten, durchgeführt werden.

In einer bevorzugten Ausführungsform des erfindungsgemäßen Verfahrens wird die Isomerisierung in einem kompartimentiertem Rohrreaktor durchgeführt.

In einer weiteren bevorzugten Ausführungsform des erfindungsgemäßen Verfahrens wird die Isomerisierung in mindestens zwei in Serie geschalteten Reaktoren durchgeführt, wobei der erste Reaktor im Wesentlichen Rührkesselcharakteristik aufweist und der zweite Reaktor so ausgeführt ist, dass er im Wesentlichen Rohrcharakteristik aufweist.

In einer besonders bevorzugten Ausführungsform des erfindungsgemäßen Verfahrens wird die Isomerisierung in einem Reaktor durchgeführt, wobei der Reaktor die Charakteristik einer Rührkesselkaskade aufweist, die 2 bis 20 Rührkesseln, insbesondere 3 bis 10 Rührkesseln, entspricht.

Die Reaktion kann in einer Ausführungsform des erfindungsgemäßen Verfahrens in einer Destillationsapparatur durchgeführt werden, wobei die Isomerisierungsreaktion zumindest im Sumpfbereich der Destillationsapparatur stattfindet. Geeignet ist jede, dem Fachmann bekannte Destillationsapparatur, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation mit gleichzeitig stattfindender Reaktion kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (a*) des erfindungsgemäßen Verfahrens wird vorzugsweise bei einem absoluten Druck von 0,1 mbar bis 100 bar, besonders bevorzugt 1 mbar bis 16 bar, insbesondere 10 mbar bis 6 bar, durchgeführt. Die Temperatur beträgt in Verfahrensschritt (a*) vorzugsweise 25 bis 250 °C, besonders bevorzugt 30 bis 180 °C, insbesondere 40 bis 140 °C.

Die Zusammensetzung des entnommen Stroms hinsichtlich des molaren Verhältnisses von 2-Methyl-3-butennitril zu linearem Pentennitril und damit der Umsatzgrad an eingesetztem 2-Methyl-3-butennitril kann in Abhängigkeit von der Zusammensetzung des zugeführten Stroms auf technisch einfache Weise durch die Temperatur, die Katalysatorkonzentration, die Verweilzeit und die Gestaltung des Reaktors eingestellt werden. In einer bevorzugten Ausführungsform des erfindungsgemäßen Verfahrens wird der Umsatzgrad mit Hilfe dieser Maßnahmen auf Werte im Bereich 10 bis 99 %, besonders bevorzugt 30 bis 95 %, insbesondere 60 bis 90 %, eingestellt.

### Verfahrensschritt (b*)

In Verfahrensschritt (b*) wird der in Verfahrensschritt (a*) erhaltene Strom 1 destilliert. Hierbei erhält man als Kopfprodukt einen Strom 2, der 2-Methyl-3-butennitril, 3-Pentennitril und (Z)-2-Methyl-2-butennitril enthält. Darüber hinaus wird in Verfahrensschritt (b*) ein Strom 3 als Sumpfprodukt erhalten, der den mindestens einen Isomerisierungskatalysator enthält.

Der Verfahrensschritt (b*) des erfindungsgemäßen Verfahrens kann in jeder geeigneten, dem Fachmann bekannten Destillationsvorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, - Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (b*) des erfindungsgemäßen Verfahrens wird vorzugsweise bei einem absoluten Druck von 0,1 mbar bis 100 bar, besonders bevorzugt 1 mbar bis 6 bar, insbesondere 10 mbar bis 500 mbar, durchgeführt. Die Destillation wird so durchgeführt, dass die Temperatur im Sumpf der Destillationsvorrichtung vorzugsweise 25 bis 250 °C, besonders bevorzugt 40 bis 180 °C, insbesondere 60 bis 140 °C, beträgt. Die Destillation wird so durchgeführt, dass die Temperatur am Kopf der Destillationsvorrichtung vorzugsweise -15 bis 200 °C, besonders bevorzugt 5 bis 150 °C, insbesondere 10 bis 100 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In einer besonders bevorzugten Ausführungsform der vorliegenden Erfindung findet die in Verfahrensschritt (b*) durchgeführte Destillation des Stromes 1 unter Druck- und Temperaturbedingungen statt, bei welcher der in der Mischung vorhandene Isomerisierungskatalysator weniger als in Verfahrensschritt (a*) oder nicht aktiv ist.

In einer bevorzugten Ausführungsform der vorliegenden Erfindung wird der in Verfahrensschritt (b*) erhaltene Strom 3, der den mindestens einen Isomerisierungskatalysator enthält, zumindest teilweise in den Verfahrensschritt (a*) zurückgeführt.

In einer weiteren Ausführungsform des erfindungsgemäßen Verfahrens finden die Verfahrensschritte (a*) und (b*) in der gleichen Vorrichtung statt. Dabei ist es auch möglich, dass der Strom 3, der den mindestens einen Isomerisierungskatalysator enthält, aus dem Verfahrensschritt (b*) nicht entnommen wird und in der gemeinsamen Vorrichtung der Verfahrensschritte (a*) und (b*) verweilt.

### Verfahrensschritt (c*)

In Verfahrensschritt (c*) findet eine Destillation des Stromes 2 statt. Hierbei wird ein Strom 4 als Kopfprodukt erhalten, der gegenüber dem Strom 2 an (Z)-2-Methyl-2-butennitril in Bezug auf die Summe aller in Strom 2 enthaltenen Pentennitrile angereichert ist. Darüber hinaus wird ein Strom 5 als Sumpfprodukt erhalten, der gegenüber dem Strom 2 an (Z)-2-Methyl-2-butennitril in Bezug auf die Summe aller in Strom 2 enthaltenen Pentennitrile abgereichert ist.

Der Verfahrensschritt (c*) kann in jeder geeigneten, dem Fachmann bekannten Vorrichtung durchgeführt werden. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehüphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der Verfahrensschritt (c*) des erfindungsgemäßen Verfahrens wird vorzugsweise bei einem absoluten Druck von 0,1 mbar bis 100 bar, besonders bevorzugt 1 mbar bis 6 bar, insbesondere 10 mbar bis 500 mbar, durchgeführt. Die Destillation wird so durchgeführt, dass die Temperatur im Sumpf der Destillationsvorrichtung vorzugsweise 25 bis 250 °C, besonders bevorzugt 40 bis 180 °C, insbesondere 60 bis 140 °C, beträgt. Die Destillation wird so durchgeführt, dass die Temperatur am Kopf der Destillationsvorrichtung vorzugsweise -15 bis 200 °C, besonders bevorzugt 5 bis 150 °C, insbesondere 10 bis 100 °C, beträgt. In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten.

In einer besonders bevorzugten Ausführungsform des erfindungsgemäßen Verfahrens werden die Verfahrensschritte (b*) und (c*) zusammen in einer Destillationsvorrichtung durchgeführt, wobei der Strom 3, der den mindestens einen Isomerisierungskatalysator enthält, als Sumpfprodukt, der Strom 4, der (Z)-2-Methyl-2-butennitril enthält, als Kopfprodukt und der Strom 5, der 3-Pentennitril und 2-Methyl-3-butennitril enthält, an einem Seitenabzug der Kolonne erhalten werden.

In einer weiteren bevorzugten Ausführungsform des erfindungsgemäßen Verfahrens werden die Verfahrensschritte (a*), (b*) und (c*) zusammen in einer Destillationsvorrichtung durchgeführt. Dabei wird der Strom 4, der (Z)-2-Methyl-2-butennitril enthält, als Kopfprodukt erhalten. Der Strom 5, der 3-Pentennitril und 2-Methyl-3-butennitril enthält, wird an einem Seitenabzug der Destillationskolonne erhalten. Der Isomerisierungskatalysator verbleibt in dieser Ausführungsform vorzugsweise im Sumpf der Destillationskolonne.

### Verfahrensschritt (d*)

Der in Verfahrensschritt (c*) erhaltene Strom 5, der 3-Pentennitril und 2-Methyl-3-butennitril enthält, wird anschließend in eine weitere Destillationsvorrichtung überführt. In dieser Destillationsvorrichtung wird der Strom 5 in einen 3-Pentennitril-Strom, der als Sumpfprodukt entnommen wird, und einen 2-Methyl-3-butennitril-Strom, der am Kopf entnommen wird, aufgetrennt.

Der Verfahrensschritt (d*) kann in jeder geeigneten, dem Fachmann bekannten Vorrichtung durchgeführt werde. Für die Destillation geeignet sind Apparaturen, wie sie beispielsweise in: Kirk-Othmer, Encyclopedia of Chemical Technology, 4. Ed., Vol. 8, John Wiley & Sons, New York, 1996, Seite 334-348 beschrieben sind, wie Siebbodenkolonnen, Glockenbodenkolonnen, Packungskolonnen, Füllkörperkolonnen, die auch als Trennwandkolonnen betrieben werden können. Diese Destillationseinrichtungen sind jeweils mit geeigneten Vorrichtungen zur Verdampfung, wie Fallfilmverdampfer, Dünnschichtverdampfer, Mehrphasenwendelrohrverdampfer, Naturumlaufverdampfer oder Zwangsumlaufentspannungsverdampfer, sowie mit Vorrichtungen zur Kondensation des Brüdenstroms ausgerüstet. Die Destillation kann man in mehreren, wie zwei oder drei Apparaturen, vorteilhaft in einer einzigen Apparatur durchführen. Die Destillation kann zudem einstufig im Sinne einer Teilverdampfung des Zulaufstroms erfolgen.

Der absolute Druck in Verfahrensschritt (d*) beträgt vorzugsweise 0,001 bis 100 bar, besonders bevorzugt 0,01 bis 20 bar, insbesondere 0,05 bis 2 bar. Die Destillation wird so durchgeführt, dass die Temperatur im Sumpf der Destillationsvorrichtung vorzugsweise 30 bis 250 °C, besonders bevorzugt 50 bis 200 °C, insbesondere 60 bis 180 °C, beträgt. Die Destillation wird so durchgeführt, dass die Kondensationstemperatur am Kopf der Destillationsvorrichtung vorzugsweise -50 bis 250 °C, besonders bevorzugt 0 bis 180 °C, insbesondere 15 bis 160 °C, beträgt.

In einer besonders bevorzugten Ausführungsform des erfindungsgemäßen Verfahrens werden die zuvor genannten Temperaturbereiche sowohl am Kopf als auch im Sumpf der Destillationsvorrichtung eingehalten

In einer besonders bevorzugten Ausführungsform des erfindungsgemäßen Verfahrens werden der Verfahrensschritt (d*) und der Verfahrensschritt (g*) in der gleichen Destillationsvorrichtung durchgeführt. Dabei fallen die Ströme 6 und 12 sowie 7 und 13 zusammen. Ferner wird in dieser bevorzugten Ausführungsform der Strom 5 direkt in die gemeinsame Vorrichtung der Verfahrensschritte (d*) und (g*) geführt. Dabei können die Zulaufstellen der Ströme 5 und 11 im Falle einer Destillationskolonne als Destillationsvorrichtung gleich oder unterschiedlich sein.

In einer weiteren Ausführungsform des erfindungsgemäßen Verfahrens werden die Verfahrensschritte (c*) und (g*) in einer gemeinsamen Destillationskolonne durchgeführt, wobei der Verfahrensschritt (d*) entfällt, der Strom 2 aus Verfahrensschritt (b*) sowie Strom 11 aus Verfahrensschritt (f*) in Verfahrensschritt (g*) geführt werden, in Verfahrensschritt (g*) der Strom 4 als Kopfprodukt, enthaltend (Z)-2-Methyl-2-butennitril, der Strom 12 als Sumpfprodukt, enthaltend 3-Pentennitril und der Strom 13 als Seitenabzugsstrom, enthaltend 2-Methyl-3-butennitril erhalten werden.

In dem erfindungsgemäßen Verfahren gemäß Ausführungsform III ist es möglich, dass der Strom 2 direkt in den Verfahrensschritt (g*) zurückgeführt wird und der Eduktstrom direkt in den Verfahrensschritt (c*) gefahren wird, wobei ein Strom 5a aus Verfahrensschritt (c*) in die Isomerisierung von Verfahrensschritt (a*) zurückgeführt wird.

Alternativ ist es auch möglich, den Strom 2 direkt in den Verfahrensschritt (g*) zurückzuführen und den Eduktstrom in Verfahrensschritt (c*) zu fahren, wobei der Strom 5 aus Verfahrensschritt (c*) in den Verfahrensschritt (f*) zurückgeführt wird.

Alternativ ist es auch möglich, dass der Strom 2 direkt in den Verfahrensschritt (g*) zurückgeführt wird und der Eduktstrom in Verfahrensschritt (c*) gefahren wird und der Strom 5 aus Verfahrensschritt (c*) in den Verfahrensschritt (e*) zurückgeführt wird.

### Vertahrensschrüt h*):

Der Verfahrensschritt h*) beinhaltet ein Verfahren zur Herstellung von Nickel(0)-Phosphorligand-Komplexen, enthaltend mindestens ein Nickel(0)-Zentralatom und mindestens einen phosphorhaltigen Liganden.

Im folgenden sind die Begriffe reduktive Katalysator-Synthese/-Regenerierung und Redox-Katalysator-Synthese/-Regenerierung synonym.

### Verfahrensschritt h₁*):

Eine bevorzugte Ausführungsform von Verfahrensschritt h*), hier als Verfahrensschritt h₁*) bezeichnet, ist dadurch gekennzeichnet, dass ein durch Azeotropdestillation getrocknetes (zuvor nach Azeodest natürlich trocken) wasserhaltiges Nickel(II)-halogenid in Gegenwart mindestens eines phosphorhaltigen Liganden reduziert wird.

### Azeotropdestillation

In der Azeotropdestillation wird ein wasserhaltiges Nickel(II)-halogenid verwendet. Wasserhaltiges Nickel(II)-halogenid ist ein Nickelhalogenid, welches ausgewählt ist aus der Gruppe von Nickelchlorid, Nickelbromid und Nickeliodid, das mindestens 2 Gew.-% Wasser enthält. Beispiele hierfür sind Nickelchlorid-Dihydrat, Nickelchlorid-Hexahydrat, eine wässrige Lösung von Nickelchlorid, Nickelbromid-Trihydrat, eine wässrige Lösung von Nickelbromid, Nickeliodid-Hydrate oder eine wässrige Lösung von Nickeliodid. Im Fall von Nickelchlorid werden bevorzugt Nickelchlorid-Hexahydrat oder eine wässrige Lösung von Nickelchlorid eingesetzt. Im Fall von Nickelbromid und Nickeliodid werden bevorzugt die wässrigen Lösungen eingesetzt. Besonders bevorzugt ist eine wässrige Lösung von Nickelchlorid.

Im Falle einer wässrigen Lösung ist die Konzentration des Nickel(II)-halogenids in Wasser an sich nicht kritisch. Als vorteilhaft hat sich ein Anteil des Nickel(II)-halogenids an der Gewichtssumme aus Nickel(II)-halogenid und Wasser von mindestens 0,01 Gew.%, vorzugsweise mindestens 0,1 Gew.%, besonders bevorzugt mindestens 0,25 Gew.%, insbesondere bevorzugt mindestens 0,5 Gew.% erwiesen. Als vorteilhaft hat sich ein Anteil des Nickel(II)-halogenids an der Gewichtssumme aus Nickel(II)-halogenid und Wasser im Bereich von höchstens 80 Gew.%, vorzugsweise höchstens 60 Gew.%, besonders bevorzugt höchstens 40 Gew.-% erwiesen. Aus praktischen Gründen ist es von Vorteil, einen Anteil von Nickelhalogenid in der Mischung aus Nickelhalogenid und Wasser nicht zu überschreiten, der unter den gegebenen Temperatur- und Druckbedingungen eine Lösung ergibt. Im Falle einer wässrigen Lösung von Nickelchlorid ist es daher aus praktischen Gründen von Vorteil, bei Raumtemperatur einen Anteil von Nickelhalogenid an der Gewichtssumme aus Nickelchlorid und Wassers von höchstens 31 % Gew.% zu wählen. Bei höheren Temperaturen können entsprechend höhere Konzentrationen gewählt werden, die sich aus der Löslichkeit von Nickelchlorid in Wasser ergeben.

Das wasserhaltige Nickel(II)-halogenid wird vor der Reduktion durch eine Azeotropdestillation getrocknet. In einer bevorzugten Ausführungsform der vorliegenden Erfindung ist die Azeotropdestillation ein Verfahren zur Entfernung von Wasser aus dem entsprechenden wasserhaltigen Nickel(II)-halogenid, wobei dieses mit einem Verdünnungsmittel versetzt wird, dessen Siedepunkt im Falle der Nichtazeotrop-Bildung des Verdünnungsmittels mit Wasser unter den Druckbedingungen der nachfolgend genannten Destillation höher ist als der Siedepunkt von Wasser und das an diesem Siedepunkt des Wassers flüssig vorliegt oder das ein Azeotrop oder Heteroazeotrop mit Wasser unter den Druck- und Temperaturbedingungen der nachfolgend genannten Destillation bildet, und die Mischung, enthaltend das wasserhaltige Nickel(II)-halogenid und das Verdünnungsmittel, unter Abtrennung von Wasser oder des genannten Azeotrops oder des genannten Heteroazeotrops von dieser Mischung und unter Erhalt einer wasserfreien Mischung, enthaltend Nickel(II)-halogenid und das besagte Verdünnungsmittel, destilliert wird.

Die Ausgangsmischung kann neben dem wasserhaltigen Nickel(II)-halogenid weitere Bestandteile enthalten, wie ionische oder nichtionische, organische oder anorganische Verbindungen, insbesondere solche, die mit der Ausgangsmischung homogen einphasig mischbar oder in der Ausgangsmischung löslich sind.

Erfindungsgemäß versetzt man das wasserhaltige Nickel(II)-halogenid mit einem Verdünnungsmittel, dessen Siedepunkt unter den Druckbedingungen der Destillation höher ist als der Siedepunkt von Wasser und das an diesem Siedepunkt des Wassers flüssig vorliegt.

Die Druckbedingungen für die nachfolgende Destillation sind an sich nicht kritisch. Als vorteilhaft haben sich Drücke von mindestens 10⁻⁴ MPa, vorzugsweise mindestens 10⁻³ MPa, insbesondere mindestens 5*10⁻³ MPa erwiesen. Als vorteilhaft haben sich Drücke von höchstens 1 MPa, vorzugsweise höchstens 5*10⁻¹ MPa, insbesondere höchstens 1,5*10⁻¹ MPa erwiesen.

In Abhängigkeit von den Druckbedingungen und der Zusammensetzung des zu destillierenden Gemischs stellt sich dann die Destillationstemperatur ein. Bei dieser Temperatur liegt das Verdünnungsmittel vorzugsweise flüssig vor. Im Sinne der vorliegenden Erfindung wird unter dem Begriff Verdünnungsmittel sowohl ein einzelnes Verdünnungsmittel wie auch ein Gemisch solcher Verdünnungsmittel verstanden, wobei sich im Falle eines solchen Gemischs die in der vorliegenden Erfindung genannten physikalischen Eigenschaften auf dieses Gemisch beziehen.

Weiterhin weist das Verdünnungsmittel unter diesen Druck- und Temperaturbedingungen vorzugsweise einen Siedepunkt auf, der im Falle der Nichtazeotrop-Bildung des Verdünnungsmittels mit Wasser höher als der von Wasser liegt, vorzugsweise um mindestens 5 °C, insbesondere mindestens 20 °C, und vorzugsweise höchstens 200 °C, insbesondere höchstens 100 °C.

In einer bevorzugten Ausführungsform kann man Verdünnungsmittel einsetzen, die mit Wasser ein Azeotrop oder Heteroazeotrop bilden. Die Menge an Verdünnungsmittel gegenüber der Menge an Wasser in dem Gemisch ist an sich nicht kritisch. Vorteilhaft sollte man mehr flüssiges Verdünnungsmittel einsetzen als den durch die Azeotrope abzudestillierenden Mengen entspricht, so dass überschüssiges Verdünnungsmittel als Sumpfprodukt verbleibt.

Setzt man ein Verdünnungsmittel ein, das mit Wasser kein Azeotrop bildet, so ist die Menge an Verdünnungsmittel gegenüber der Menge an Wasser in dem Gemisch an sich nicht kritisch.

Das eingesetzte Verdünnungsmittel ist dabei insbesondere ausgewählt aus der Gruppe bestehend aus organischen Nitrilen, aromatischen Kohlenwasserstoffen, aliphatischen Kohlenwasserstoffen und Mischungen der zuvor genannten Lösemittel. Bezüglich der organischen Nitrile werden vorzugsweise Acetonitril, Propionitril, n-Butyronitril, n-Valeronitril, Cyanocyclopropan, Acrylnitril, Crotonitril, Allylcyanid, cis-2-Pentennitril, trans-2-Pentennitril, cis-3-Pentennitril, trans-3-Pentennitril, 4-Pentennitril, 2-Methyl-3-butennitril, Z-2-Methyl-2-butennitril, E-2-Methyl-2-butennitril, Ethylsuccinnitril, Adipodinitril, Methylglutarnitril oder Mischungen davon verwendet. Bezüglich der aromatischen Kohlenwasserstoffe können vorzugsweise Benzol, Toluol, o-Xylol, m-Xylol, p-Xylol oder Mischungen davon verwendet werden. Aliphatische Kohlenwasserstoffe können vorzugsweise aus der Gruppe der linearen oder verzweigten aliphatischen Kohlenwasserstoffe, besonders bevorzugt aus der Gruppe der Cycloaliphaten, wie Cyclohexan oder Methylcyclohexan, oder Mischungen davon gewählt werden. Besonders bevorzugt werden cis-3-Pentennitril, trans-3-Pentennitril, Adipodinitril, Methylglutarnitril oder Mischungen daraus als Lösemittel verwendet.

Setzt man als Verdünnungsmittel ein organisches Nitril bzw. Mischungen, enthaltend mindestens ein organisches Nitril ein, so hat es sich als vorteilhaft erwiesen, die Menge an Verdünnungsmittel so zu wählen, dass in der fertigen Mischung der Anteil des Nickel(II)-halogenids an der Gewichtssumme aus Nickel(II)-halogenid und Verdünnungsmittel mindestes 0,05 Gew.-%, vorzugsweise mindestens 0,5 Gew.%, besonders bevorzugt mindestens 1 Gew.% beträgt.

Setzt man als Verdünnungsmittel ein organisches Nitril bzw. Mischungen, enthaltend mindestens ein organisches Nitril ein, so hat es sich als vorteilhaft erwiesen, die Menge an Verdünnungsmittel so zu wählen, dass in der fertigen Mischung der Anteil des Nickel(II)-halogenids an der Gewichtssumme aus Nickel(II)-halogenid und Verdünnungsmittel höchstens 50 Gew.%, vorzugsweise höchstens 30 Gew.%, besonders bevorzugt höchstens 20 Gew.-% beträgt.

Erfindungsgemäß destilliert man die Mischung, enthaltend das wasserhaltige Nickel(II)-halogenid und das Verdünnungsmittel, unter Abtrennung von Wasser von dieser Mischung und unter Erhalt einer wasserfreien Mischung, enthaltend Nickel(II)-halogenid und das besagte Verdünnungsmittel. In einer bevorzugten Ausführungsform wird zunächst die Mischung hergestellt und anschließend destilliert. In einer anderen bevorzugten Ausführungsform wird das wasserhaltige Nickelhalogenid, besonders bevorzugt die wässrige Lösung des Nickelahlogenids, während der Destillation nach und nach zu dem siedenden Verdünnungsmittel zugegeben. Dadurch kann die Bildung eines verfahrenstechnisch schwer zu handhabenden, schmierigen Feststoffs im wesentlichen vermieden werden.

Im Falle von Pentennitril als Verdünnungsmittel kann man die Destillation vorteilhaft bei einem Druck von höchstens 1 Megapascal, vorzugsweise 0,5 Megapascal.

- Im Falle von Pentennitril als Verdünnungsmittel kann man die Destillation vorzugsweise bei einem Druck von mindestens 1 kPa, vorzugsweise mindestens 5 kPa, besonders bevorzugt 10 kPa, durchführen.

Die Destillation kann vorteilhaft durch einstufige Verdampfung, bevorzugt durch fraktionierende Destillation in einer oder mehreren, wie 2 oder 3 Destillationsapparaturen erfolgen. Dabei kommen für die Destillation hierfür übliche Apparaturen in Betracht, wie sie beispielsweise in: Kirk-Othmer, Encyclopedia of Chemical Technology, 3. Ed., Vol. 7, John Wiley & Sons, New York, 1979, Seite 870-881 beschrieben sind, wie Siebbodenkolonnen, Glockenbodenkolonnen, Packungskolonnen, Füllkörperkolonnen, Kolonnen mit Seitenabzug oder Trennwandkolonnen.

Die Destillation kann diskontinuierlich oder kontinuierlich erfolgen.

### Reduktion

Das Verfahren zur Herstellung von Nickel(0)-Phosphorligand-Komplexen, enthaltend mindestens ein Nickel(0)-Zentralatom und mindestens einen phosphorhaltigen Liganden, durch Reduktion wird vorzugsweise in Gegenwart eines Lösemittels durchgeführt. Das Lösemittel ist dabei insbesondere ausgewählt aus der Gruppe bestehend aus organischen Nitrilen, aromatischen Kohlenwasserstoffen, aliphatischen Kohlenwasserstoffen und Mischungen der zuvor genannten Lösemittel. Bezüglich der organischen Nitrile werden vorzugsweise Acetonitril, Propionitril, n-Butyronitril, n-Valeronitril, Cyanocyclopropan, Acrylnitril, Crotonitril, Allylcyanid, cis-2-Pentennitril, trans-2-Pentennitril, cis-3-Pentennitril, trans-3-Pentennitril, 4-Pentennitril, 2-Methyl-3-butennitril, Z-2-Methyl-2-butennitril, E-2-Methyl-2-butennitril, Ethylsuccinnitril, Adipodinitril, Methylglutarnitril oder Mischungen davon verwendet. Bezüglich der aromatischen Kohlenwasserstoffe können vorzugsweise Benzol, Toluol, o-Xylol, m-Xylol, p-Xylol oder Mischungen davon verwendet werden. Aliphatische Kohlenwasserstoffe können vorzugsweise aus der Gruppe der linearen oder verzweigten aliphatischen Kohlenwasserstoffe, besonders bevorzugt aus der Gruppe der Cycloaliphaten, wie Cyclohexan oder Methylcyclohexan, oder Mischungen davon gewählt werden. Besonders bevorzugt werden cis-3-Pentennitril, trans-3-Pentennitril, Adipodinitril, Methylglutarnitril oder Mischungen daraus als Lösemittel verwendet.

Vorzugsweise wird ein inertes Lösemittel verwendet.

Die Konzentration des Lösemittels beträgt vorzugsweise 10 bis 90 Massen-%, besonders bevorzugt 20 bis 70 Massen-%, insbesondere 30 bis 60 Massen-%, jeweils bezogen auf die fertige Reaktionsmischung.

In einer besonderen Ausführungsform der vorliegenden Erfindung ist das Lösemittel identisch zu dem Verdünnungsmittel, das in dem oben beschriebenen erfindungsgemäßen Verfahren zur Herstellung der wasserfreien Mischung, enthaltend das Nickel(II)-Halogenid und das Verdünnungsmittel, verwendet wird.

In dem erfindungsgemäßen Verfahren beträgt die Konzentration des Liganden in dem Lösemittel vorzugsweise 1 bis 90 Gew.%, besonders bevorzugt 5 bis 80 Gew.%, insbesondere 50 bis 80 Gew.-%.

Das in dem erfindungsgemäßen Verfahren verwendete Reduktionsmittel ist vorzugsweise ausgewählt aus der Gruppe bestehend aus Metallen, die elektropositiver als Nickel sind, Metallalkylen, elektrischem Strom, komplexen Hydriden und Wasserstoff.

Wenn in dem erfindungsgemäßen Verfahren als Reduktionsmittel ein Metall, das elektropositiver als Nickel ist, verwendet wird, so ist dieses Metall vorzugsweise ausgewählt aus der Gruppe bestehend aus Natrium, Lithium, Kalium, Magnesium, Calcium, Barium, Strontium, Titan, Vanadium, Eisen, Kobalt, Kupfer, Zink, Cadmium, Aluminium, Gallium, Indium, Zinn, Blei und Thorium. Besonders bevorzugt sind hierbei Eisen und Zink. Wird Aluminium als Reduktionsmittel verwendet, so ist es von Vorteil, wenn dieses durch Reaktion mit einer katalytischen Menge Quecksilber(II)-Salz oder Metallalkyl voraktiviert wird. Bevorzugt wird für die Voraktivierung Triethylaluminium in einer Menge von vorzugsweise 0,05 bis 50 Mol.-%, besonders bevorzugt 0,5 bis 10 Mol-%, verwendet. Das Reduktionsmetall ist vorzugsweise fein verteilt, wobei der Ausdruck "fein verteilt" bedeutet, dass das Metall in einer Partikelgröße von weniger als 10 mesh, besonders bevorzugt weniger als 20 mesh, verwendet wird.

Wenn in dem erfindungsgemäßen Verfahren als Reduktionsmittel ein Metall verwendet wird, das elektropositiver ist als Nickel, so beträgt die Menge an Metall vorzugsweise 0,1 bis 50 Gew.%, bezogen auf die Reaktionsmasse.

Wenn in dem erfindungsgemäßen Verfahren als Reduktionsmittel Metallalkyle verwendet werden, so handelt es sich bevorzugt um Lithiumalkyle, Natriumalkyle, Magnesiumalkyle, insbesondere Grignard-Reagenzien, Zinkalkyle oder Aluminiumalkyle. Besonders bevorzugt sind Aluminiumalkyle, wie Trimethylaluminium, Triethylaluminium, Triisopropylaluminium oder Mischungen hiervon, insbesondere Triethylaluminium. Die Metallalkyle können in Substanz oder gelöst in einem inerten organischen Lösesmittel, wie Hexan, Heptan oder Toluol, eingesetzt werden.

Wenn in dem erfindungsgemäßen Verfahren komplexe Hydride als Reduktionsmittel verwendet werden, so werden bevorzugt Metallaluminiumhydride, wie Lithiumaluminiumhydrid, oder Metallborhydride, wie Natriumborhydrid, eingesetzt.

Das molare Verhältnis der Redoxäquivalente zwischen der Nickel(II)-Quelle und dem Reduktionsmittel beträgt vorzugsweise 1 : 1 bis 1 : 100, besonders bevorzugt 1 : 1 bis 1 : 50, insbesondere 1 : 1 bis 1 : 5.

In dem e-ndungsgemäßen Verfahren kann der zu verwendende Ligand auch in einer Ligandlösung vorliegen, die bereits als Katalysatorlösung in Hydrocyanierungsreaktionen wie beispielsweise Schritt e*) oder Isomerisierungsreaktionen wie beispielsweise Schritt a*) eingesetzt wurde und an Nickel(0) abgereichert ist. Solche Ströme sind die Ströme 3 bzw. 10 entweder teilweise oder jeweils unabhängig ausgewählte Teilströme 14 (aus Teilstrom 3) bzw. Teilstrom 16 (aus Teilstrom 10) in Stufe h*) und die folgenden Stufen, ggf. i*), j*) und k*) gefahren werden. Die ggf. verbleibenden Teilströme 15 (aus Strom 3) und 17 (aus Strom 10) werden nicht durch h*), i*), j*) und k*) gefharen, sondern direkt in Stufe a*) oder e*) zurückgeführt. Diese "Rück-Katalysatorlösung" hat im Allgemeinen die folgende Zusammensetzung:
- 2 bis 60 Gew.%, insbesondere 10 bis 40 Gew.-% Pentennitrile,
- 0 bis 60 Gew.%, insbesondere 0 bis 40 Gew.-% Adipodinitril,
- 0 bis 10 Gew.%, insbesondere 0 bis 5 Gew.-% andere Nitrile,
- 10 bis 90 Gew.%, insbesondere 50 bis 90 Gew.-% phosphorhaitiger Ligand und
- 0 bis 2 Gew.%, insbesondere 0 bis 1 Gew.-% Nickel(0).

Der in der Rück-Katalysatorlösung enthaltene freie Ligand kann nach dem erfindungsgemäßen Verfahren somit wieder zu einem Nickel(0)-Komplex umgesetzt werden.

In einer besonderen Ausführungsform der vorliegenden Erfindung ist das Verhältnis der Nickel(II)-Quelle zu phosphorhaltigem Ligand 1 : 1 bis 1 : 100. Weitere bevorzugte Verhältnisse von Nickel(II)-Quelle zu phosphorhaltigem Ligand sind 1 : 1 bis 1 : 3, insbesondere 1 : 1 bis 1 : 2.

Das erfindungsgemäße Verfahren kann bei beliebigem Druck durchgeführt werden. Aus praktischen Gründen sind Drücke zwischen 0.1 bara und 5 bara, vorzugsweise 0.5 bara und 1.5 bara, bevorzugt.

Das erfindungsgemäße Verfahren kann in Batchfahrweise oder kontinuierlich durchgeführt werden.

In dem erfindungsgemäßen Verfahren ist es möglich, ohne Überschuss an Nickel(II)-halogenid oder Reduktionsmittel, beispielsweise Zink, zu arbeiten, so dass deren Abtrennung nach der Nickel(0)-Komplexbildung nicht notwendig ist.

In einer besonderen Ausführungsform der vorliegenden Erfindung umfasst das erfindungsgemäße Verfahren die folgenden Verfahrensschritte:
(1) Trocknung eines wasserhaltigen Nickel(II)-halogenids durch Azeotropdestillation,
(2) Vorkomplexierung des azeotrop getrockneten Nickel(II)-halogenids in einem Lösemittel in Gegenwart eines phosphorhaltigen Liganden,
(3) Zugabe mindestens eines Reduktionsmittels zu der aus Verfahrensschritt (2) stammenden Lösung oder Suspension bei einer Zugabetemperatur von 20 bis 120°C,
(4) Rühren der aus Verfahrensschritt (3) stammenden Suspension oder Lösung für bei einer Umsetzungstemperatur von 20 bis 120 °C.

Die Vorkomplexierungstemperaturen, Zugabetemperaturen und Umsetzungstemperaturen können, jeweils unabhängig voneinander, 20 °C bis 120 °C betragen. Besonders bevorzugt sind bei der Vorkomplexierung, Zugabe und Umsetzung Temperaturen von 30 °C bis 80 °C.

Die Vorkomplexierungszeiträume, Zugabezeiträume und Umsetzungszeiträume können, jeweils unabhängig voneinander, 1 Minute bis 24 Stunden betragen. Der Vorkomplexierungszeitraum beträgt insbesondere 1 Minute bis 3 Stunden. Der Zugabezeitraum beträgt vorzugsweise 1 Minute bis 30 Minuten. Der Umsetzungszeitraum beträgt vorzugsweise 20 Minuten bis 5 Stunden.

### Verfahrensschritt h₂*):

Eine weitere bevorzugte Ausführungsform von Verfahrensschritt h*), hier als Verfahrensschritt h₂*) beschrieben, beinhaltet die Aufstockung des Nickel(0)-Gehaltes der Ströme 14 bzw. 16 z.B. durch Einrühren von Nickelpulver. Dabei wird freier Phosphor-Ligand in den Strömen 14 bzw. 16 als Komplexbildungspartner verwendet oder frischer Ligand zugegeben.

Die Katalysatorverbindungen können aus Nickel-Pulver mit einer geeigneten Halogenidquelle als Initiator, wie z.B. ein Halogenid oder ein alkylsubstituiertes Halogenid von Phosphor, Arsen oder Antimon, wie CH₃PCl₂, CH₃AsCl₂ oder CH₃SbCl₂, oder ein geeignetes Metallhalogenid, elementares Halogen, wie Chlor, Brom oder Jod oder den entsprechenden Halogenwasserstoffen oder Thionylhalogenid hergestellt werden. Erfindungsgemäß zu verwendende Metallhalogenide sind die Halogenide von Cr , Ni, Ti, Cu, Co, Fe, Hg, Sn, Li, K, Ca, Ba, Sc, Ce, V, Mn, Be, Ru, Rh, Pd, Zn, Cd, Al, Th, Zr und Hf. Das Halogenid kann Chlorid, Bromid oder Jodid sein. Besonders geeignete Halogenidquellen sind PX₃, TiX₄, ZrX₄, HfX₄ oder HX, worin X Chlorid, Bromid oder Jodid bedeutet. Bei Durchführung der erfindungsgemäßen Reaktion können auch Mischungen von 2 oder mehr Initiatoren oder Katalysatoren verwendet werden.

Die Katalysatorregenerierung kann diskontinuierlich, z.B. in Batch-Fahrweise analog US 3,903,120, oder kontinuierlich analog US 4,416,825 bei Temperaturen von 0 bis 200°C, bevorzugt 25 bis 145°C, besonders bevorzugt 50 bis 100°C durchgeführt werden. Die Verweilzeit des Katalysators kann in weiten Grenzen variiert werden und liegt in der Regel zwischen 15 Minuten und 10h, bevorzugt 20 Minuten und 5h, besonders bevorzugt 30 Minuten und 2h.

Bei Durchführung von Verfahrensschritt h₂*) anstelle von Verfahrensschritt h₁*) können ggf. die Verfahrensschritte i*), j*) und k*) ganz oder teilweise entfallen.

### Verfahrensschritt i*):

Falls erforderlich, kann man Strom 18 vor Schritt i) durch Destillation z.B. bei Drücken von 0,1 bis 5000, bevorzugt 0,5 bis 1000 und insbesondere 1 bis 200 mbar (abs.) und Temperaturen von 10 bis 150, bevorzugt 40 bis 100°C - oder andere geeignete Maßnahmen einengen, beispielsweise auf 50 bis 95, bevorzugt 60 bis 90 % seines ursprünglichen Volumens. In einer besonders bevorzugten Ausführungsform enthält dieser Strom nach dem Einengen bis zu 10 Gew.%, also 0 bis 10 Gew.%, bevorzugt 0,01 bis 8 Gew.-% Pentennitrile.

### Schritt i*): Zufügen der unpolaren aprotischen Flüssigkeit F

In Schritt i*) fügt man dem Strom 18 eine unpolare aprotische Flüssigkeit F hinzu, wodurch man einen Strom 19 erhält. Dabei bedeutet Flüssigkeit, dass die Verbindung F zumindest unter den in Schritt i*) herrschenden Druck- und Temperaturbedingungen in flüssiger Form vorliegt; bei anderen Druck- und Temperaturbedingungen kann F auch fest oder gasförmig sein.

Als unpolare (bzw. apolare) aprotische Flüssigkeit F eignen sich alle unter den Bedingungen des Schrittes i*) flüssigen Verbindungen, die den Katalyastor, z.B. den Ni(0)-Komplex mit phosphorhaltigen Liganden und/oder die freien phosphorhaltigen Liganden, chemisch oder physikalisch nicht oder nicht wesentlich verändern. Als Flüssigkeit F geeignete Verbindungen enthalten kein ionisierbares Proton im Molekül und haben in der Regel niedrige relative Dielektrizitätskonsanten (εᵣ < 15) bzw. niedrige elektrische Dipolmomente (µ < 2,5 Debye).

Geeignet sind insbesondere Kohlenwasserstoffe, die beispielsweise unhalogeniert oder halogeniert sein können, sowie - insbesondere tertiäre - Amine, und Kohlenstoff disulfid.

In einer bevorzugten Ausführungsform ist die Flüssigkeit F ein Kohlenwasserstoff K*. Geeignet sind aliphatische, cycloaliphatische oder aromatische Kohlenwasserstoffe K*. Als aliphatische Kohlenwasserstoffe eignen sich z.B. lineare oder verzweigte Alkane oder Alkene mit 5 bis 30, bevorzugt 5 bis 16 C-Atomen, insbesondere Pentan, Hexan, Heptan, Octan, Nonan, Decan, Undecan und Dodecan (jeweils alle Isomere).

Geeignete cycloaliphatische Kohlenwasserstoffe weisen beispielsweise 5 bis 10 C-Atome auf, wie Cyclopentan, Cyclohexan, Cycloheptan, Cycloocatan, Cyclononan und Cyclodecan. Auch substitiuerte, insbesondere C₁₋₁₀-alkylsubstituierte Cycloaliphaten wie Methylcyclohexan sind geeignet. Als aromatische Kohlenwasserstoffe eigen sich vorzugsweise solche mit 6 bis 20 C-Atomen, insbesondere Benzol, Toluol, o-, m- und p-Xylol, Naphtalin und Anthracen. Man kann auch substituierte, vorzugsweise C₁₋₁₀-alkylsubstituierte Aromaten wie Ethylbenzol verwenden.

Besonders bevorzugt ist der Kohlenwasserstoff K* ausgewählt unter den weiter unten für den Kohlenwasserstoff K genannten Verbindungen. Ganz besonders bevorzugt ist der Kohlenwasserstoff K* identisch mit dem Kohlenwasserstoff K, d.h. man verwendet für die Extraktion in Schritt j*) und als Flüssigkeit F den gleichen Kohlenwasserstoff.

### Ausgestaltung des Zufügens der Flüssigkeit

Die unpolare aprotische Flüssigkeit F kann dem Strom 18 in üblichen Mischvorrichtungen zugefügt werden. Bevorzugt weil verfahrenstechnisch besonders einfach vermischt man in Schritt i*) die unpolare aprotische Flüssigkeit F mit dem Strom 18 in einem Rührbehälter oder einem Umpumpkreislauf.

Bevorzugt wird die unpolare aprotische Flüssigkeit mit dem Strom 18 innig vermischt. Als Rührbehälter eignen sich übliche Flüssigkeitsmischer, die mit intensivmischenden Mischelementen und/oder statischen bzw. beweglichen Einbauten versehen sein können.

Die Verwendung eines Umpumpkreislaufs ist ebenfalls bevorzugt. Er wird üblicherweise derart betrieben, dass das Verhältnis von Umpumpmenge zu Ausstoß aus dem Umpumpkreis, 0,1 : 1 bis 1000 : 1, bevorzugt 1 : 1 bis 100 : 1 und besonders bevorzugt 2 : 1 bis 25 : 1 beträgt. Als Umlaufpumpe eigen sich z.B. Zahnradpumpen oder andere übliche Pumpen. Bevorzugt arbeitet die Umlaufpumpe gegen einen Überströmer, der bei einem definierten Druck von z.B. 3 bis 10 bar (abs.) öffnet.

Sofern man in Schritt i*) und j*) den gleichen Kohlenwasserstoff verwendet, kann man in beiden Schritten jeweils frischen Kohlenwasserstoff einsetzen. Ebenso kann man den in Schritt i*) eingesetzten Kohlenwasserstoff in Schritt j*) weiterverwenden, oder den in Schritt j*) eingesetzten Kohlenwasserstoff nach Schritt i*) zurückführen und dort weiterverwenden.

In einer ganz besonders bevorzugten Ausführungsform ist die Flüssigkeit F ein Teilstrom des Stroms 22 (mit Katalysator angereicherter Kohlenwasserstoff K, siehe unten), der bei Schritt j*) anfällt. Dies bedeutet, dass in Schritt j*) ein Teil von Strom 22 abgezweigt wird und man den abgezweigten Teil in Schritt i*) dem Strom 18 zufügt. In dieser Ausführungsform wird demnach ein Teil des Stroms 22 im Kreis gefahren.

In einer anderen, ebenfalls bevorzugten Ausführungsform wird die unpolare aprotische Flüssigkeit F direkt in eine Verweilzeitstrecke (siehe weiter unten) dosiert, beispielsweise an deren Anfang.

Das Zufügen der Flüssigkeit F erfolgt in der Regel bei Temperaturen von 0 bis 150, bevorzugt 10 bis 100 und insbesondere 20 bis 80°C, und Drücken von 0,01 bis 100, bevorzugt 0,1 bis 10 und insbesondere 0,5 bis 5 bar (abs.).

Die erforderliche Menge der Flüssigkeit F kann in weiten Grenzen varriieren. Sie ist in der Regel geringer als die verwendete Menge des Kohlenwasserstoffs K, mit dem in Schritt j*) extrahiert wird, kann jedoch auch größer sein. Bevorzugt beträgt die Menge der Flüssigkeit F 0,1 bis 200 Vol.-%, insbesondere 1 bis 50 Vol.-% und besonders bevorzugt 5 bis 30 Vol.-%, bezogen auf die Menge des in Schritt j*) zur Extraktion eingesetzten Kohlenwasserstoffs K.

### Optionale Behandlung mit Ammoniak oder Amin

Wenn Schritt h*) eine Redox-Regenerierung enthält, so kann ggf. Strom 18 oder Strom 19 oder während Schritt i*) oder während Schritt j*) selbst Ammoniak oder ein primäres, sekundäres oder tertiäres aromatisches oder aliphatisches Amin zugesetzt werden. Aromatisch schließt alkylaromatisch, und aliphatisch schließt cycloaliphatisch ein.

Es wurde gefunden, dass sich durch diese Ammoniak- bzw. Aminbehandlung der Gehalt an Katalysator, insbesondere an Nickel(0)-Komplex bzw. Ligand bei der Extraktion (Schritt j*)) in der zweiten, mit Dinitrilen angereicherten Phase (Strom 23) vermindern lässt, d.h. bei der Extraktion wird die Verteilung des Ni(0)-Komplexes bzw. der Liganden auf die beiden Phasen zugunsten der ersten Phase (Strom 22) verschoben. Die Ammoniak- bzw. Aminbehandlung verbessert die Katalysatoranreicherung im Strom 22; dies bedeutet geringere Katalysatorverluste im Katalysatorkreislauf und verbessert die Wirtschaftlichkeit der Hydrocyanierung.

Demnach geht in dieser Ausführungsform der Extraktion eine Behandlung des Stroms 18 bzw. von Strom 19 mit Ammoniak bzw. einem Amin voraus oder erfolgt während der Extraktion. Dabei ist die Behandlung während der Extraktion weniger bevorzugt.

Besonders bevorzugt fügt man den Ammoniak bzw. das Amin zusammen mit der unpolaren aprotischen Flüssigkeit F zu. Insbesondere erfolgt die Zugabe der Flüssigkeit F und des Ammoniaks bzw. Amins in derselben Mischvorrichtung.

Als Amine verwendet man Monomamine, Diamine, Triamine oder höherfuktionelle Amine (Polyamine). Die Monoamine weisen üblicherweise Alkylreste, Arylreste oder Arylalkylreste mit 1 bis 30 C-Atomen auf; geeignete Monoamine sind z.B. primäre Amine, z.B. Monoalkylamine, sekundäre oder tertiäre Amine, z.B. Dialkylamine. Geeignete primäre Monoamine sind beispielsweise Butylamin, Cyclohexylamin, 2-Methylcyclohexylamin, 3-Methylcyclohexylamin, 4-Methylcyclohexylamin, Benzylamin, Tetrahydrofurfurylamin und Furfurylamin. Als sekundäre Monoamine kommen z.B. Diethylamin, Dibutylamin, Di-n-propylamin und N-Methylbenzylamin in Betracht. Als tertiäre Amine eignen sich beispielsweise Trialkylamine mit C₁₋₁₀-Alkylresten, wie Trimethylamin, Triethylamin oder Tributylamin.

Als Diamine eignen sich z.B. solche der Formel R¹-NH-R²-NH-R³, worin R¹, R² und R³ unabhängig voneinander Wasserstoff oder einen Alkylrest, Arylrest oder Arylalkylrest mit 1 bis 20 C-Atomen bedeuten. Der Alkylrest kann linear oder insbesondere für R² auch cyclisch sein. Geeignete Diamine sind beispielsweise Ethylendiamin, die Propylendiamine (1,2-Diaminopropan and 1,3-Diaminopropan), N-Methyl-ethylendiamin, Piperazin, Tetramethylendiamin (1,4-Diaminobutan), N,N'-Dimethylethylendiamin, N-Ethylethylendiamin, 1,5-Diaminopentan, 1,3-Diamino-2,2-diethylpropan, 1,3-Bis-(methylamino)propan, Hexamethylendiamin (1,6-Diaminohexan), 1,5-Diamino-2-methylpentan, 3-(Propylamino)-propylamin, N,N'-Bis-(3-aminopropyl)-piperazin, N,N'-Bis-(3-aminopropyl)-piperazin und Isophorondiamin (IPDA). Als Triamine, Tetramine bzw. höherfunktionelle Amine eignen sich z.B. Tris(2-aminoethyl)amin, Tris(2-aminopropyl)amin, Diethylentriamin (DETA), Triethylentetramin (TE-TA), Tetraethylenpentamin (TEPA), Isopropylentriamin, Dipropylentriamin und N,N'-bis(3-aminopropyl-ethylendiamin). Aminobenzylamine und Aminohydrazide mit 2 oder mehr Aminogruppen sind ebenfalls geeignet.

Naturgemäß kann man auch Mischungen von Ammoniak mit einem oder mehreren Aminen, oder Mischungen mehrerer Amine verwenden.

Bevorzugt verwendet man Ammoniak oder aliphatische Amine, insbesondere Trialkylamine mit 1 bis 10 C-Atomen im Alkylrest, z.B. Trimethylamin, Triethylamin oder Tributylamin, sowie Diamine wie Ethylendiamin, Hexamehtylendiamin oder 1,5-Diamino-2-methylpentan.

Besonders bevorzugt ist Ammoniak allein, d.h. besonders bevorzugt verwendet man neben Ammoniak kein Amin. Wasserfreier Ammoniak ist ganz besonders bevorzugt; dabei bedeutet wasserfrei einen Wassergehalt unter 1 Gew.-%, bevorzugt unter 1000 und insbesondere unter 100 ppm by weight.

Das Molverhältnis von Amin zu Ammoniak kann in weiten Grenzen variiert werden, liegt in der Regel bei 10000 : 1 bis 1 : 10000.

Die Menge des eingesetzten Ammoniaks bzw. Amins richtet sich u.a. nach Art und Menge des Katalysators, z.B. des Nickel(0)-Katalysators und/oder der Liganden, und - sofern mitverwendet - nach Art und Menge der Lewis-Säure, die bei der Hydrocyanierung als Promotor eingesetzt wird. Üblicherweise beträgt das Molverhältnis von Ammoniak bzw. Amin zu Lewis-Säure mindestens 1 : 1. Die Obergrenze dieses Molverhältnisses ist in der Regel unkritisch und beträgt beispielsweise 100 : 1; der Überschuss an Ammoniak bzw. Amin sollte jedoch nicht so groß sein, dass sich der Ni(0)-Komplex bzw. dessen Liganden zersetzen. Bevorzugt beträgt das Molverhältnis Ammoniak bzw. Amin zu Lewis-Säure 1 : 1 bis 10 : 1, besonders bevorzugt 1,5 : 1 bis 5 : 1, und insbesondere etwa 2 : 1. Sofern man eine Mischung aus Ammoniak und Amin verwendet, gelten diese Molverhältnisse für die Summe aus Ammoniak und Amin.

Die Temperatur bei der Behandlung mit Ammoniak bzw. Amin ist üblicherweise nicht kritisch und beträgt beispielsweise 10 bis 140, bevorzugt 20 bis 100 und insbesondere 20 bis 90°C. Auch der Druck ist in der Regel nicht kritisch.

Der Ammoniak bzw. das Amin kann dem Strom 18 gasförmig, flüssig (unter Druck stehend) oder gelöst in einem Lösungsmittel zugegeben werden. Als Lösungsmittel eignen sich z.B. Nitrile, insbesondere solche, die bei der Hydrocyanierung vorliegen, und weiterhin aliphatische, cycloaliphatische oder aromatische Kohlenwasserstoffe, wie man sie bei dem erfindungsgemäßen Verfahren als Extraktionsmittel verwendet, beispielsweise Cyclohexan, Methylcyclohexan, n-Heptan oder n-Octan.

Die Ammoniak- bzw. Aminzugabe erfolgt in üblichen Vorrichtungen, beispielsweise solchen zur Gaseinleitung oder in Flüssigkeitsmischern. Der dabei in vielen Fällen ausfallende Feststoff kann entweder im Strom 18 verbleiben, d.h. der Extraktion wird eine Suspension zugeführt, oder abgetrennt werden wie nachfolgend beschrieben.

### Optionale Abtrennung der Feststoffe

In einer bevorzugten Ausführungsform werden Feststoffe, die in Schritt i*) des Verfahrens ausfallen, vor der Extraktion (Schritt j*)) aus dem Strom 19 abgetrennt.

Dadurch lässt sich in vielen Fällen die Extraktionsleistung des erfindungsgemäßen Verfahrens weiter verbessern, denn oftmals vermindern anfallende Feststoffe die Trennleistung der Extraktionsvorrichtungen. Außerdem wurde gefunden, dass eine Feststoffabtrennung vor der Extraktion in manchen Fällen die unerwünschte Mulmbildung nochmals deutlich vermindert oder vollständig unterdrückt.

Bevorzugt wird die Feststoffabtrennung derart ausgestaltet, dass Feststoffpartikel mit einem hydraulischen Durchmesser größer 5 µm, insbesondere größer 1 µm und besonders bevorzugt größer 100 nm, abgetrennt werden.

Zur Feststoffabtrennung kann man übliche Verfahren verwenden, beispielsweise Filtration, Querstromfiltration, Zentrifugation, Sedimentation, Klassierung oder bevorzugt Dekantieren, wozu gängige Vorrichtungen wie Filter, Zentrifugen bzw. Dekanter verwendet werden können.

Temperatur und Druck bei der Feststoffabtrennung sind üblicherweise nicht kritisch. Beispielsweise kann man in den zuvor bzw. weiter unten genannten Temperatur- bzw. Druckbereichen arbeiten.

Die Feststoffabtrennung kann vor, während oder nach der - optionalen - Behandlung des Stroms 18 bzw. des Stromes 19 mit Ammoniak bzw. Amin erfolgen. Dabei ist die Abtrennung während oder nach der Ammoniak- bzw. Aminbehandlung bevorzugt, und danach besonders bevorzugt.

Sofern man die Feststoffe während oder nach der Ammoniak- bzw. Aminbehandlung abtrennt, handelt es sich bei den Feststoffen zumeist um im Strom 18 schwerlösliche Verbindungen von Ammoniak bzw. Amin mit der verwendeten Lewis-Säure bzw. dem Promotor. Verwendet man beispielsweise ZnCl₂, so fällt bei der Ammoniakbehandlung im Wesentlichen schwerlösliches ZnCl₂• 2 NH₃ aus.

Sofern man die Feststoffe vor der Ammoniak- bzw. Aminbehandlung abtrennt oder falls gar keine Behandlung mit Ammoniak oder Amin erfolgt, handelt es sich bei den Feststoffen in der Regel um Nickelverbindungen der Oxidationsstufe +II, beispielsweise Nickel(II)cyanid oder ähnliche cyanidhaltige Nickel(II)verbindungen, oder um Lewis-Säuren bzw. deren Verbindungen. Die genannten Verbindungen können beispielsweise ausfallen, weil ihre Löslichkeit z.B. durch Temperaturänderung vermindert wurde.

### Optionale Verweilzeitstrecke

Der Strom 19 als Austrag von Schritt i*) kann unmittelbar in Schritt j*) überführt werden, beispielsweise durch eine Rohrleitung. Dabei bedeutet unmittelbar, dass die mittlere Verweilzeit von Strom 19 in der Rohrleitung weniger als 1 min beträgt.

Jedoch ist in einer bevorzugten Ausführungsform das erfindungsgemäße Verfahren dadurch gekennzeichnet, dass der Strom 19 nach Schritt i*) und vor Schritt j*) durch eine Verweilzeitstrecke geführt wird. Die Verweilzeitstrecke befindet sich folglich nach der Zugabe der Flüssigkeit F und vor der Extraktion.

Als Verweilzeitstrecke eignen sich z.B. Rohrleitungen, statische Mischer, gerührte oder ungerührte Behälter oder Behälterkaskaden, sowie Kombinationen dieser Elemente. Die Verweilzeitstrecke wird vorzugsweise derart bemessen und ausgestaltet, dass die mittlere Verweilzeit des Stroms 19 in der Verweilzeitstrecke mindestens 1 min, bevorzugt mindestens 5 min beträgt.

Die weiter oben beschriebene optionale Feststoffabtrennung kann auch in der Verweilzeitstrecke erfolgen. Dabei dient die Verweilzeitstrecke als Beruhigungszone, in der sich der Feststoff absetzen kann. Auf diese Weise wirkt die Verweilzeitstrecke wie ein Dekanter oder Querstromfilter. Sie kann mit Vorrichtungen zur Förderung und/oder zum Austrag von Feststoffen versehen sein.

Wie erwähnt wird in einer bevorzugten Ausführungsform die unpolare aprotische Flüssigkeit F direkt in die Verweilzeitstrecke dosiert, beispielsweise an deren Anfang. Besonders bevorzugt wählt man in dieser Ausführungsform eine Verweilzeitstrecke, die eine innige Durchmischung von Strom 18 und Flüssigkeit F gewährleistet. Wie ebenfalls bereits beschrieben kann die Verweilzeitstrecke eine Phasentrennung des Stroms 19 bewirken.

Die Verweilzeitstrecke wird in der Regel bei Temperaturen von 0 bis 200, bevorzugt 10 bis 150 und insbesondere 20 bis 100°C, und Drücken von 0,01 bis 100, bevorzugt 0,1 bis 10 und insbesondere 0,5 bis 5 bar (abs.), betrieben.

In einer bevorzugten Ausgestaltung der Erfindung beträgt die Strömungsgeschwindigkeit des Stroms 19 in allen in dem erfindungsgemäßen Verfahren verwendeten Rohrleitungen mindestens 0,5, insbesondere mindestens 1 und besonders bevorzugt mindestens 2 m/s.

Der in Schritt a) erhaltene Strom 19 wird, ggf. nach der Behandlung mit Ammoniak oder Aminen, und/oder nach der Feststoffabtrennung und/oder nach Durchlaufen der Verweilzeitstrecke, in Schritt j*) extrahiert.

### Verfahrensschritt j*):

### Verfahrensprinzip

Das erfindungsgemäße Verfahren eignet sich zur extraktiven Reinigung von Ni(0)-Komplexen, die phosphorhaltige Liganden und/oder freie phosphorhaltige Liganden enthalten, in Strom 19 oder ggf. Strom 18, wenn Schritt i*) nicht ausgeführt wird, durch Zugabe eines C6-Dinitrils wie Adipodinitril (ADN), 2-Methylglutarsäuredinitril (MGN) oder 2-Ethylbemsteinsäuredinitril (ESN) bezüglich der Störkomponente(n), die eine vermehrte Bildung von der Hydrocyanierung nicht zugänglichen C5-Mononitrilen wie E-2-Methyl-2-butennitril und/oder Z-2-Methyl-2-butennitril auslösen.

Zudem werden die Katalysatorverluste in der Extraktion vermindert, in dem ein Kohlenwasserstoff K im Strom 21 an einer Zulaufstelle aufgegeben wird, die näher zur Ablaufstelle des Extrakts als zur Zulaufstelle des Feedstroms 18 bzw. 19 liegt. Die Zulaufstelle des Dinitrils (Strom 20) liegt näher zur Ablaufstelle des Raffinats als die Zulaufstelle des Feedstroms 18 bzw. 19 liegt. Hierbei ist unter näher im Sinn der Anzahl der theoretischen Trennstufen zwischen zwei Punkten zu verstehen. Zwischen den Zulaufstellen der Ströme 18 bzw. 19 und 21 liegen in der Regel 0 bis 10, bevorzugt 1 bis 7 theoretische Extraktions-(Trenn-)stufen (Rückextraktionszone für den Katalysator); zwischen den Zulaufstellen der Ströme 18 bzw. 19 und 20 liegen in der Regel 1. bis 10, bevorzugt 1 bis 5 theoretische Extraktions-(Trenn-)stufen (Reinigungszone bzgl. Störkomponente(n)).

In der Regel bildet sich bei einer Temperatur T (in °C) eine erste Phase [Raffinat; Strom 22], die gegenüber dem Strom 18 an den genannten Ni(0)-Komplexen bzw. Liganden angereichert ist, und eine zweite Phase [Extrakt; Strom 23; angereicherte Störkomponente(n)], die gegenüber dem Strom 18 an Dinitrilen angereichert ist. Zumeist ist die erste Phase die leichtere Phase, also die Oberphase, und die zweite Phase die schwerere Phase, also die Unterphase.

Die Oberphase enthält nach der Phasentrennung vorzugsweise zwischen 50 und 99 Gew.-%, besonders bevorzugt zwischen 60 und 97 Gew.-%, insbesondere zwischen 80 und 95 Gew.-%, des zur Extraktion eingesetzten Kohlenwasserstoffes.

Die Lewis-Säure, die gegebenenfalls (nämlich bei Erzeugung der Redox-KatalysatorRegenerierung in Verfahrensschritt h₁*)) im Zulaufstrom der Extraktion enthalten ist, verbleibt vorzugsweise größtenteils und besonders bevorzugt vollständig in der Unterphase. Hier bedeutet vollständig, dass die Restkonzentration der Lewis-Säure in der Oberphase vorzugsweise kleiner als 1 Gew.-%, besonders bevorzugt kleiner als 0,5 Gew.-%, insbesondere kleiner als 500 ppm by weight ist.

Die Ausschleusung der Störkomponente(n) verbessert die Verfahrensselektivität da weniger der Hydrocyanierung nicht zugängliche C5-Mononitrile gebildet werden (Verminderung von Fehlisomerisierungen).

Ein besonderer Vorteil der Ausführungsform III besteht darin, dass Dinitrile wie ADN, MGN, ESN, die sich in geringen Mengen im Verfahrensschritt e*) bilden und damit im Strom 10 anreichern, zumindest teilweise mit der Unterphase der Extraktion ausgetragen werden.

Ein weiterer besonderer Vorteil der Anwendung von Verfahrensschritt j*) besteht darin, dass als Edukt im Verfahrensschritt e*) stabiliastorhaltiges Butadien verwendet werden kann. Ein solcher Stabilisator kann beispielsweise tert.-Butylbrenzkatechin sein. Dieser Stabilisator wird über die Unterphase der Extraktion ausgetragen. Somit können keine katalysatorschädigenden Konzentrationen des Stabilisators im Katalysatorkreislauf aufgepegelt werden.

Ein weiterer besonderer Vorteil besteht darin, dass in Verfahrensschritt h*) eine Redox-Regenerierung des Katalysators zur Aufstockung des Ni(0)-Wertes gemäß h₁*) vorgenommen werden kann, da die hierbei entstehende Lewis-Säure über die Unterphase der Extraktion ausgetragen wird. Diese Lewis-Säure würde anderenfalls in der ersten Hydrocyanierung (Verfahrensschritt e*)) zu verstärkter Dinitrilbildung führen.

Die Unterphase der Extraktion kann in geeigneter Weise aufbereitet werden, so dass die darin enthaltenen Dinitrile wieder als Zulauf zur Extraktion eingesetzt werden können. Eine solche Aufarbeitung kann z.B. destillativ erfolgen (DE-A-10 2004 004683; Strom 7 aus Schritt c)).

### Ausgestaltung der Extraktion

Die extraktiven Aufgaben können bevorzugt gelöst werden, indem eine Gegenstromextraktionskolonne mit einer Rückextraktionszone verwendet wird. Es sind jedoch auch gleichartig wirkende Kombinationen von jeder geeigneten, dem Fachmann bekannten Vorrichtungen, wie Gegenstrom-Extraktionskolonnen, Mixer-Settler-Kaskaden oder Kombinationen von Mixer-Settler-Kaskaden mit Kolonnen, beispielsweise eine Serienschaltung von zwei Gegenstrom-Extraktionskolonnen (beispielsweise eine für die Reinigung bzgl. Störkomponente(n), die andere für die Rückextraktion des Katalysators). Besonders bevorzugt ist die Verwendung von Gegenstrom-Extraktionskolonnen, die insbesondere mit Blechpackungen als dispergierenden Elemente ausgestattet sind. In einer weiteren besonders bevorzugten Ausführungsform wird die Extraktion im Gegenstrom in kompartimentierten, gerührten Extraktionskolonnen ausgeführt.

Betreffend die Dispergierrichtung wird in einer bevorzugten Ausführungsform des Verfahrens der Kohlenwasserstoff als kontinuierliche Phase und der Strom 18 der Hydrocyanierung als disperse Phase eingesetzt. Dies verkürzt in der Regel die Phasentrennzeit und vermindert die Mulmbildung. Jedoch ist auch die umgekehrte Dispergierrichtung, also Strom 18 als kontinuierliche und Kohlenwasserstoff als disperse Phase, möglich. Letzteres gilt insbesondere dann, wenn die Mulmbildung durch vorherige Feststoffabtrennung (siehe weiter unten), höhere Temperatur bei der Extraktion bzw. Phasentrennung oder Verwendung eines geeigneten Kohlenwasserstoffs reduziert oder vollständig unterdrückt wird. Üblicherweise wählt man die für die Trennleistung der Extraktionsvorrichtung günstigere Dispergierrichtung.

In der Extraktion werden folgende Verhältnisse der Zuläufe eingestellt:
Strom 20 zu der Summe aus Strom 18 oder 19 und Strom 21 im Bereich von 0,01 bis 10 kg/kg, bevorzugt 0,05 bis 5 kg/kg. Strom 21 zu Strom 20 im Bereich von 0,05 bis 20 kg/kg, bevorzugt 1 bis 10 kg/kg. Strom 21 zu Strom 18 bzw. 19 im Bereich von 0,05 bis 20 kg/kg, bevorzugt 0,5 bis 8 kg/kg.

Der absolute Druck während der Extraktion beträgt vorzugsweise 10 kPa bis 1 MPa, besonders bevorzugt 50 kPa bis 0,5 MPa, insbesondere 75 kPa bis 0,25 MPa (absolut).

Die Extraktion wird vorzugsweise bei einer Temperatur von -15 bis 120°C, insbesondere 20 bis 100°C und besonders bevorzugt 30 bis 80°C durchgeführt. Es wurde gefunden, dass bei höherer Temperatur der Extraktion die Mulmbildung geringer ist.

### Ausgestaltung der Phasentrennung

Die Phasentrennung kann räumlich und zeitlich je nach apparativer Ausgestaltung auch als letzter Teil der Extraktion betrachtet werden. Zur Phasentrennung kann üblicherweise ein weiter Druck-, Konzentrations- und Temperaturbereich gewählt werden, wobei die für die jeweilige Zusammensetzung der Reaktionsmischung optimalen Parameter leicht durch wenige einfache Vorversuche ermittelt werden können.

Die Temperatur T bei der Phasentrennung beträgt üblicherweise mindestens 0°C, vorzugsweise mindestens 10°C, besonders bevorzugt mindestens 20°C. Üblicherweise beträgt sie höchstens 120°C, vorzugsweise höchstens 100°C, besonders bevorzugt höchstens 95°C. Beispielsweise führt man die Phasentrennung bei 0 bis 100°C, bevorzugt 60 bis 95°C durch. Es wurde gefunden, dass bei höherer Temperatur der Phasentrennung die Mulmbildung geringer ist.

Der Druck bei der Phasentrennung liegt in der Regel bei mindestens 1 kPa, vorzugsweise mindestens 10 kPa, besonders bevorzugt 20 kPa. In der Regel beträgt er höchstens 2 MPa, vorzugsweise höchstens 1 MPa, besonders bevorzugt höchstens 0,5 MPa absolut.

Die Phasentrennzeit, also die Zeitspanne von der Vermischung des Stroms 18 mit dem Kohlenwasserstoff (Extraktionsmittel) bis zur Ausbildung einer einheitlichen Oberphase und einer einheitlichen Unterphase, kann in weiten Grenzen variieren. Die Phasentrennzeit beträgt in der Regel 0,1 bis 60, bevorzugt 1 bis 30 und insbesondere 2 bis 10 min. Bei großtechnischer Durchführung des erfindungsgemäßen Verfahrens ist üblicherweise eine Phasentrennzeit von maximal 15, insbesondere maximal 10 min technisch und ökonomisch sinnvoll.

Es wurde gefunden, dass sich die Phasentrennzeit insbesondere bei Verwendung langkettiger aliphatischer Alkane wie n-Heptan oder n-Octan als Kohlenwasserstoff K in vorteilhafter Weise vermindert.

Die Phasentrennung kann in einer oder mehreren dem Fachmann für solche Phasentrennungen bekannten Vorrichtungen durchgeführt werden. In einer vorteilhaften Ausführungsform kann man die Phasentrennung in der Extraktionsvorrichtung durchführen, beispielsweise in einer oder mehreren Mixer-Settler-Kombinationen oder durch Ausstattung einer Extraktionskolonne mit einer Beruhigungszone.

Bei der Phasentrennung erhält man zwei flüssige Phasen, von denen eine Phase einen höheren Anteil an dem Nickel(0)-Komplex mit phosphorhaltigen Liganden und/oder freien phosphorhaltigen Liganden, bezogen auf das Gesamtgewicht dieser Phase, aufweist als die andere Phase oder anderen Phasen. Die andere Phase ist angereichert an der/den Störkomponente(n).

### Dinitril

Strom 20, der als Feedstrom zur Extraktion geführt wird, beinhaltet überwiegend Dinitrile, bevorzugt C6-Dinitrile, insbesondere bevorzugt Adipodinitril (ADN), 2-Methylglutarsäuredinitril (MGN), 2-Ethylbernsteinsäuredinitril (ESN) oder Gemische derer. Der Gehlat dieses Stroms an Dinitrilen ist bevorzugt größer als 50 Gew.-%, besonders bevorzugt größer als 70 Gew.-%, insbesondere bevorzugt größer als 90 Gew.-%. Verfahren zur Herstellung von Dinitrilen, insbesondere C6-Dinitrilen sind an sich bekannt. Ein mögliches solches Verfahren ist in der DE-A-10 2004 004683 beschrieben. Derartig hergestellte Ströme von C6-Dinitrilen, insbesondere die Ströme 15, 16 und 17 aus Verfahrensschritt h) aus der DE-A-10 2004 004683 sind generell geeignet, um hier als Strom 20 verwendet zu werden.

Der Zusatz von Dinitrilen erfolgt bevorzugt in dem Maße, dass eine Phasentrennung in der Extraktionsstufe k*) erfolgt.

### Kohlenwasserstoff

Der Kohlenwasserstoff ist das Extraktionsmittel. Er weist bevorzugt einen Siedepunkt von mindestens 30°C, besonders bevorzugt mindestens 60°C, insbesondere mindestens 90°C, und bevorzugt höchstens 140°C, besonders bevorzugt höchstens 135°C, insbesondere höchstens 130°C auf, jeweils bei einem Druck von 10⁵ Pa absolut.

Besonders bevorzugt kann ein Kohlenwasserstoff, wobei im Sinne der vorliegenden Erfindung hierunter ein einzelner Kohlenwasserstoff, wie auch ein Gemisch solcher Kohlenwasserstoffe verstanden wird, zur Abtrennung, insbesondere durch Extraktion, von Adipodinitril aus einer Mischung, enthaltend Adipodinitril und den Ni(0) enthaltenden Katalysator, eingesetzt werden, der einen Siedepunkt im Bereich zwischen 90°C und 140°C aufweist. Aus der nach der Abtrennung gemäß diesem Verfahren enthaltenen Mischung kann das Adipodinitril vorteilhaft durch destillative Abtrennung des Kohlenwasserstoffs erhalten werden, wobei der Einsatz eines Kohlenwasserstoffs mit einem Siedepunkt in dem genannten Bereich eine besonders wirtschaftliche und technisch einfache Abtrennung durch die Möglichkeit der Kondensierung des abdestillierten Kohlenwasserstoffs mit Flusswasser gestattet.

Geeignete Kohlenwasserstoffe sind beispielsweise in US 3,773,809, Spalte 3, Zeile 50-62, beschrieben. Vorzugsweise kommt ein Kohlenwasserstoff, ausgewählt aus Cyclohexan, Methylcyclohexan, Cycloheptan, n-Hexan, n-Heptan, isomeren Heptanen, n-Octan, iso-Octan, isomeren Octanen wie 2,2,4-Trimethylpentan, cis- und trans-Decalin oder deren Gemische, insbesondere aus Cyclohexan, Methylcyclohexan, n-Heptan, isomeren Heptanen, n-Octan, isomeren Octanen wie 2,2,4-Trimethylpentan, oder deren Gemische, in Betracht. Besonders bevorzugt verwendet man Cyclohexan, Methylcyclohexan, n-Heptan oder n-Octan.

Ganz besonders bevorzugt sind n-Heptan oder n-Octan. Bei diesen Kohlenwasserstoffen ist die unerwünschte Mulmbildung besonders gering. Unter Mulm wird ein Bereich unvollständiger Phasentrennung zwischen Ober- und Unterphase verstanden, meist ein flüssig/flüssig-Gemisch, in dem auch Feststoffe dispergiert sein können. Übermäßige Mulmbildung ist unerwünscht, da sie die Extraktion behindert und u.U. die Extraktionsvorrichtung vom Mulm geflutet werden kann, wodurch sie ihre Trennaufgabe nicht mehr erfüllen kann.

Der verwendete Kohlenwasserstoff ist vorzugsweise wasserfrei, wobei wasserfrei einen Wassergehalt von unter 100, vorzugsweise unter 50, insbesondere unter 10 ppm by weight bedeutet. Der Kohlenwasserstoff kann durch geeignete, dem Fachmann bekannte Verfahren getrocknet werden, beispielsweise durch Adsorption oder Azeotropdestillation. Die Trocknung kann in einem dem erfindungsgemäßen Verfahren vorgeschalteten Schritt erfolgen.

### Verfahrensschritt k*):

In ***Verfahrensschritt (k*)*** findet eine Destillation des Stromes 22 unter Erhalt eines den mindestens einen Katalysator enthaltenden Stromes 25 und eines das Extraktionsmittel enthaltenden Stromes 24 statt.

Dieser Verfahrensschritt dient im Wesentlichen zum Zurückgewinnen des Katalysators und des Extraktionsmittels.

Der Verfahrensschritt (k*) kann in jeder geeigneten, dem Fachmann bekannten Apparatur durchgeführt werden. Die Destillation des Verfahrensschrittes k*) findet vorzugsweise in einer oder mehreren Verdampfungsstufen sowie Rektifikationskolonnen/Destillationskolonnen statt.

Als Einbauten für die Rektifikationskolonnen/Destillationskolonnen werden vorzugsweise strukturierte Blechpackung, strukturierte Gewebepackung, Glockenböden, Dualflow-Böden oder Schüttungen aus Füllkörpern oder Kombinationen von zwei oder mehreren dieser Klassen von trennwirksamen Einbauten verwendet. Die Rektifikationskolonne Destillationskolonne des Verfahrensschrittes k*) kann mit einem oder mehreren flüssigen oder gasförmigen Seitenabzüge ausgeführt sein. Die Rektifikationskolonne Destillationskolonne aus Verfahrensschritt k*) kann als Trennwandkolonne mit einem oder mehreren vorhandenen gasförmigen oder flüssigen Seitenabzügen ausgeführt werden.

Die eine oder mehrere Verdampferstufen beziehungsweise die Rektifikationskolonne/Destillationskolonne des Verfahrensschrittes k*) können insbesondere mit Fallfilmverdampfer, Dünnschichtverdampfer, Naturumlaufverdampfer, Zwangsumlaufentspannungsverdampfer und Mehrphasenwendelrohrverdampfer ausgerüstet sein.

In einer weiteren Ausführungsform des erfindungsgemäßen Verfahrens wird mindestens eine der Verdampfereinheiten von Verfahrensschritt k*) mit einem geteilten Sumpf betrieben, wobei man aus einem ersten Sumpf der betreffenden Verdampferstufe den im Verhältnis zum Sumpfabzugsstrom im Allgemeinen großen Umlaufstrom zum Verdampfer fährt, den flüssigen Ablaufstrom aus dem Verdampfer jedoch nicht direkt in den Sumpf zurückgibt, sondern in einen zweiten Sumpf, der vom ersten Sumpf getrennt ist, auffängt, aus dem zweiten Sumpf den Sumpfabzugsstrom erhält und den verbleibenden Überschuss vom Verdampferumlaufstrom in den ersten Sumpf überlaufen lässt, wobei als Sumpfabzugsstrom aus dem zweiten Sumpf eine Mischung erhalten wird, die gegenüber dem Abzug aus dem ersten Sumpf an Leichtsiedern abgereichert ist.

Der absolute Druck in Verfahrenschritt k*) beträgt vorzugsweise 0,001 bis 2 bar(a), besonders bevorzugt 0,01 bis 0,5 bar(a), insbesondere 0,09 bis 0,12 bar(a). Die Destillation wird so durchgeführt, dass die Temperatur im Sumpf der Destillationsapparatur vorzugsweise 40 bis 150°C, besonders bevorzugt 70 bis 120 °C, insbesondere 80 bis 100 °C, beträgt. Die Destillation wird so durchgeführt, dass die Temperatur am Kopf der Destillationsapparatur vorzugsweise -15 bis 100°C, besonders bevorzugt 0 bis 60 °C, insbesondere 20 bis 50 °C, beträgt. In einer besonders bevorzugten Ausführungsform des erfindungsgemäßen Verfahrens werden die zuvor genannten Temperaturbereiche sowohl am Kopf als auch im Sumpf eingehalten.

Bei der Abtrennung des Extraktionsmittels zur Rückgewinnung des Katalysators gemäß Verfahrensschritt k*) kann ggf. 3-Pentennitril als Zwischensieder zur Destillation gegeben werden. Dieser Lösemittelwechsel hat ggf. den Vorteil, dass eine effektive Abreicherung des Extraktionsmittels aus dem hochsiedenden Katalysatorstrom bei Verdampfertemperaturen möglich wird, die niedrig genug sind, um den jeweilig eingesetzten Nickelkatalysator, insbesondere bei Verwendung von Chelatliganden nicht thermisch zu schädigen und bei Verwendung von monodentaten Liganden thermisch zu schonen, wobei der Druck noch hoch genug ist, um das im Vergleich zu den Katalysatorbestandteilen vergleichsweise leichtsiedende Extraktionsmittel am Kopf der Verdampferstufe oder Destillationskolonne noch bei üblichen Kühlwassertemperaturen von 25 bis 50 °C kondensieren zu können. Der Lösungsmittelwechsel hat zudem gegebenenfalls den Vorteil, dass die Fließfähigkeit und die Einphasigkeit der Katalysatorlösung sichergestellt wird, da, abhängig von Temperatur und Restgehalt an Extraktionsmittel - ohne den Zusatz von 3-Pentennitril - gegebenenfalls Katalysatorbestandteile auskristallisieren können. Dabei wirkt sich 3-Pentennitril, das beispielsweise von den Extraktionsmitteln Cyclohexan oder Methylcyclohexan oder Heptan oder n-Heptan je nach Druckverhältnissen nur schwer oder wegen Minimumsdampfdruckazeotropbildung gar nicht vollständig abtrennbar ist, bei einem Anteil von vorzugsweise bis 10 Gew.-%, besonders bevorzugt bis 5 Gew.-%, insbesondere bis 1 Gew.-%, bezogen auf die Gesamtmenge des Extraktionsmittelzulaufstroms zur Extraktionskolonne in Verfahrensschritt j*), nicht störend auf das erfindungsgemäße Verfahren aus.

Der in Verfahrensschritt k*) erhaltene Strom 24, enthaltend das Extraktionsmittel, wird in einer bevorzugten Ausführungsform des erfindungsgemäßen Verfahrens zumindest teilweise in den Extraktionsschritt j*) zurückgeführt. Dabei wird der recyclierte Strom 24 gegebenenfalls vor dem Extraktionsschritt j*) getrocknet, so dass der Wassergehalt in diesem Strom vorzugsweise weniger als 100 Gew.-ppm, besonders bevorzugt weniger als 50 Gew.-ppm, insbesondere weniger als Gew.-10 ppm, beträgt.

Der in Verfahrensschritt k*) erhaltene Strom 25, enthaltend den Katalysator, wird in einer weiteren bevorzugten Ausführungsform des erfindungsgemäßen Verfahrens zumindest teilweise in die Hydrocyanierung des Verfahrensschritts e*) bzw. in die Isomerisierung des Verfahrensschritts a*) zurückgeführt. In einer bevorzugten Ausführungsform des erfindungsgemäßen Verfahrens beträgt der Anteil an Extraktionsmittel in dem Strom 25 vorzugsweise weniger als 10 Gew.-%, besonders bevorzugt weniger als 5 Gew.-%, insbesondere weniger als 1 Gew.-%, bezogen auf die Gesamtmenge von Strom 25.

Im folgenden werden anhand der Schemata 1 bis 5 bevorzugte Ausführungsformen der Katalysatorstromführung durch die Verfahrensstufen a*) bis k*) beschrieben. Der Verfahrensschritt i*) ist in den Schemata 1 bis 5 nicht enthalten, kann jedoch jeweils zwischen h*) und j*) durchgeführt werden. In den Schemata 1 bis 5 wird exemplarisch der Verfahrensschritt h*) in der Ausführungsform als Verfahrensschritt h₁*) beschrieben. Alternativ kann auch die Ausführungsform als Verfahrensschritt h₂*) durchgeführt werden. In diesen Fällen wären die Nickel(II)chloride durch Nickelpulver ersetzt, das Reduktionsmittel (Red.) entfällt ebenso wie die Lewissäure (LS). ADN steht synonym für Dinitrilströme; Heptan synonym für Kohlenwasserstoffe als Extraktionsmittel. Kat. Bedeutet jeweils Katalysatorkomplexe plus freier Ligand. Die gestrichelten Linien in den Schemata 1 bis 5 bezeichnen optionale bypass Teilströme der Katalysatorströme.

Eine besondere Ausführungsform des Verfahrens bzgl. der Katalysatorstromführung ist in Schema 1 dargestellt. Demnach sind die katalysatorführenden Stufen in einem einzigen Katalysatorkreis zusammengefasst. Die Reihenfolge der Verfahrensstufen lautet ausgehend von der ersten Hydrocyanierung e*), f*), a*), b*), c*), h*), ggf. i*), j*), k*), ehe wieder e*) gespeist wird. Dabei kann gegebenenfalls ein Teilstrom des Katalysators an den Stufen h*), i*), j*) und k*) vorbei direkt in e*) zurückgeführt werden. Die Verfahrensstufe a*) wird hier mit nicht aufgetrenntem Gemisch aus 2-Methyl-3-butennitril und 3-Pentennnitril gespeist, d.h. d*) bzw. g*) wird nach a*) ausgeführt.

Eine weitere besondere Ausführungsform des Verfahrens bzgl. der Katalysatorstromführung ist in Schema 2 dargestellt. Demnach sind die katalysatorführenden Stufen in einem einzigen Katalysatorkreis zusammengefasst. Die Reihenfolge der Verfahrensstufen lautet ausgehend von der ersten Hydrocyanierung e*), f*), a*), b*), c*), h*), ggf. i*), j*), k*), ehe wieder e*) gespeist wird. Dabei kann gegebenenfalls ein Teilstrom des Katalysators an den Stufen h*), i*), j*) und k*) vorbei direkt in e*) zurückgeführt werden. Die Verfahrensstufe a*) wird hier mit bzgl. 3-Pentennnitril abgereichertem 2-Methyl-3-butennitril gespeist, d.h. d*) bzw. g*) wird vor a*) ausgeführt.

Eine weitere besondere Ausführungsform des Verfahrens bzgl. der Katalysatorstromführung ist in Schema 3 dargestellt. Demnach wird ein Katalysatorstrom über die Stufen e*) und f*) im Kreis gefahren. Aus diesem Strom wird ein Teilstrom ausgeschleust und als Katalysatorfeed für die Stufe a*) verwendet. Anschließend wird dieser Strom vollständig, gegebenenfalls teilweise, durch die Stufen b*), c*), h*), gegebenenfalls i*), j*) und k*) wieder in e*) zurückgespeist. Die Isomerisierungsstufe a*) wird mit bzgl. 3-Pentennnitril abgereichertem 2-Methyl-3-butennitril gespeist, d.h. d*) bzw. g*) wird vor a*) ausgeführt. Ebenso kann aber auch d* bzw. g* nach a* ausgeführt werden.

Eine weitere besondere Ausführungsform des Verfahrens bzgl. der Katalysatorstromführung ist in Schema 4 dargestellt. Demnach werden zwei Katalysatorkreise aufgebaut. Katalysatorkreis 1 beinhaltet die Stufen e*) und f*), Katalysatorkreis 2 die Stufen a*), b*), c*). Aus beiden Strömen werden Teilströme, gegebenenfalls auch die jeweils gesamten Katalysatorströme über die Stufen h*), gegebenenfalls i*), j*) und k*) gefahren, um den Katalysator von Störkompontene(n) zu reinigen und/oder den Ni(0)-Gehalt aufzustocken. Dabei ist der über die Extraktion gefahrene Anteil aus Katalysatorkreis 2 bevorzugt größer als der aus Katalysatorkreis 1. Gegebenenfalls wird der gesamte Strom aus Katalysatorkreis 2 durch die Extraktion gefahren. Die beiden Katalysatorkreise sind nur über die Stufen h*), gegebenenfalls i*), j*) und k*) miteinander gekoppelt. Die Aufteilung des Stroms aus k*) zur Speisung von a*) bzw. e*) entspricht in der Regel dem Verhältnis der Feedströme zu h*) aus a*) und e*). Die Isomerisierungsstufe a*) wird mit bzgl. 3-Pentennnitril abgereichertem 2-Methyl-3-butennitril gespeist, d.h. d*) bzw. g*) wird vor a*) ausgeführt. Ebenso kann aber auch d* bzw. g* nach a* ausgeführt werden.

Eine weitere besondere Ausführungsform des Verfahrens bzgl. der Katalysatorstromführung ist in Schema 5 dargestellt. Demnach wird ein Katalysatorkreis über die Stufen a*), b*), c*), h*), gegebenenfalls i*), j*) und k*) betrieben. Aus diesem Katalysatorkreis wird vor h*) ein Teilstrom abgezogen und damit die erste Hydrocyanierung e*) betrieben. Über f*) wird der Strom zu h*) zurückgeführt. Gegebenenfalls kann die Rückführung auch direkt zu a*) erfolgen. Auch ein Teilstrom des Isomerisierungskatalysatorkreises kann aus c*) direkt in a*) zurückgeführt werden. Die Isomerisierungsstufe a*) wird mit bzgl. 3-Pentennnitril abgereichertem 2-Methyl-3-butennitril gespeist, d.h. d*) bzw. g*) wird vor a*) ausgeführt. Ebenso kann aber auch d* bzw. g* nach a* ausgeführt werden.

Die vorliegende Erfindung wird anhand der nachfolgend dargestellten Beispiele näher erläutert.

### Ausführungsbeispiele

In den Beispielen werden folgende Abkürzungen verwendet:
- Cyanwasserstoff :: Cyanwasserstoff
- T3PN:: trans-3-Pentennitril
- C3PN:: cis-3-Pentennitril
- 4PN:: 4-Pentennitril
- E2M2BN:: (E)-2-Methyl-2-butennitril
- T2PN:: trans-2-Pentennitril
- C2PN:: cis-2-Pentennitril
- ADN:: Adipodinitril
- MGN:: Methylglutarnitril
- VSN :: Valeriansäurenitril
- VCH :: 4-Vinylcyclohexen
- BD:: 1,3-Butadien
- TBC:: tert.-Butylbrenzkatechin
- C2BU:: cis-2-Buten
- LS:: Lewis-Säure

In den Beispielen werden die Verfahrensschritte in einer chronologischen Reihenfolge angegeben und weichen somit von der Bezeichnung in der Beschreibung und in den Ansprüchen ab. Nicht näher charakterisierte Angaben in % bzw. ppm sind Gew.-% bzw. Gew.-ppm.

### Beispiel 1:

Beispiel 1 wird anhand von Figur 3 verdeutlicht.

In Beispiel 1 wird für die Hydrocyanierung von Butadien ein Katalysatorsystem auf Basis von Nickel(0)-Komplexen mit einem Gemisch von Liganden eingesetzt. Die Ligandmischung zur Hydrocyanierung enthält ca. 60 Mol% Tri(m/p-tolyl)phosphit und 40 Mol% des Chelatphosphonits 1:

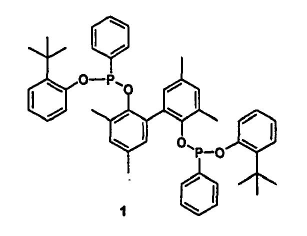

In einem Schritt (1) werden folgende Ströme in einen Schlaufenreaktor R1 von 25 I Volumen gefahren, der mit einer Düse, Impulsaustauschrohr, externen Umpumpkreislauf und im einem im Umpumpkreislauf befindlichen Wärmetauscher zur Abfuhr der Reaktionsenergie ausgestattet ist und auf 357 K temperiert ist:
(1) 10 kg/h flüssiger unstabilisierter, durch Destillation von Wasser befreiter Cyanwasserstoff,
(2) 22 kg/h handelsübliches BD, enthaltend 0,25 % C2BU, das durch Kontakt mit Aluminiumoxid behandelt wurde, um Wasser und Stabilisator TBC zu entfernen,
(3) 8 kg/h rückgeführtes BD aus K2a in Schritt (2) (Strom 9), so dass als gesamter BD-Zulauf zum Reaktor R1 ein Strom von 30 kg/h enthaltend 90 % BD, 5 % C2BU sowie 5 % 1-Buten erhalten wird,
(4) 21 kg/h Nickel(0)-Katalysatorlösung, erhalten wie in diesem Beispiel weiter unten beschrieben als Strom 10a aus der Kolonne K2b

Der aus dem Reaktor R1 abgezogene Strom 8 (63 kg/h) enthält in Summe 11 % BD und C2BU, entsprechend einem Umsatz von 79 % BD, sowie in Summe 63 % Pentennitrile, 31% T3PN, 29 % 2M3BN und geringe Mengen Z2M2BN und E2M2BN sowie weitere Pentennitril-Isomere (T2PN, C2PN, C3PN, 4PN), sowie die Katalysatorbestandteile und Katalysatorabbauprodukte und MGN.

Strom 8 wird in einem Schritt (2) einer Destillationskolonne K2a zugeführt, die mit Verstärkungs- und Abtriebsteil betrieben wird und mit einem Fallfilmverdampfer und getrennten Sumpf ausgestattet ist, sowie Kolonneneinbauten mit strukturierter Packung enthält, die 10 theoretische Trennstufen erzeugen. Kolonne K2a wird am Kopf mit einem Direktkondensator betrieben der aus einem mit strukturierter Packung bestückten Kolonnenschuss mit Totalfangtasse, Umpumpkreis und externem Wärmetauscher besteht. Die Kolonne K2a wird bei einem absoluten Kopfdruck von 2,0 bar, 288 K Kopftemperatur und 363 K Sumpfabzugstemperatur betrieben.

Über Kopf der Kolonne K2a wird der Strom 9 erhalten, der wie eingangs beschrieben als Rückführstrom in den Reaktor R1 dosiert wird. Das Rücklaufverhältnis am Kopf der Kolonne K2a wird so eingestellt, dass der Strom 9 ca. 100 ppm 2M3BN enthält.

Über Sumpf der Kolonne K2a werden 59 kg/h eines Stromes 1 b erhalten, der 2,9 % BD, 4,6 % C2BU, 67 % Pentennitrile sowie zusätzlich die Katalysatorbestandteilen enthält. C2BU ist im Verhältnis zu BD gegenüber dem Zulauf deutlich angereichert.

Strom 1b wird innerhalb des Schrittes (2) in eine Destillationskolonne K2b gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 10 theoretische Trennstufen erzeugen. Die Kolonne wird bei einem absoluten Kopfdruck von 150 mbar, 329 K Kopftemperatur und 373 K Sumpfabzugstemperatur betrieben. Der Brüdenstrom der Kolonne wird bei 308 K teilkondensiert und mit einem Nachkondensator bei 263 K behandelt. Der so von 2M3BN und anderen Pentennitrilen abgereicherte BD-Strom 2c wird in einem Verdichter V2 auf einen absoluten Druck von 1,2 bar verdichtet. Der verdichtete Gasstrom wird bei 279 K zum großen Teil unter Erhalt eines Stromes 2e (5 kg/h) kondensiert, wobei ein Teilstrom 2d (47 NI/h, enthaltend 44 % C2BU) gasförmig entsorgt wird. Strom 2e wird flüssig in den Kondensatsammelbehälter der Kolonne K2a zurückgefahren.

An der Kolonne K2b wird in einem gasförmigen Seitenabzug der Strom 11 gewonnen (40 kg/h), enthaltend ca. 100 ppm BD, 46 % 2M3BN und 48 % T3PN sowie in geringerem Umfang E2M2BN und Z2M2BN neben anderen Pentennitril-Isomeren. Die Position des Seitenabzugs ist so gewählt, dass unter dem Seitenabzug in einem Abtriebsteil die Komponente 2M3BN in dem über Sumpf gewonnen Strom 10 im Verhältnis zu T3PN abgereichert wird.

In die Kolonne K2b werden 13 kg/h eines Katalysatorstromes gefahren, der wie in Beispiel 1 gemäß der deutschen Patentanmeldung mit dem Titel "Verfahren zur Herstellung von Dinitrilen" der BASF AG (B03/0525) beschrieben als Sumpfabzug der Kolonne K4 aus Schritt (4) erhalten wird, enthaltend in Summe 73 % Pentennitrile, 0,5 % Ni(0), 18 % Ligandmischung sowie ca. 5 % ADN.

An der Kolonne K2b wird über Sumpf der Katalysator-Strom 10 erhalten, enthaltend 0,5 % Ni(0), ca. 100 ppm 2M3BN und 73 % restliche Pentennitrile. Der Strom 10 wird aufgespalten in Teilstrom 10a (21 kg/h), der in den Reaktor R1 zurückgefahren wird. Der andere Teil (10b) (5,4 kg/h) wird einer Regenerierung gemäß der DE-A-103 51 002 zugeführt, um nach Regenerierung beispielsweise in der Hydrocyanierung von 3-Pentennitril wie in Beispiel 1 gemäß der DE-A-102 004 004 683 beschrieben eingesetzt zu werden.

Der Strom 11 wird in einem Schritt (3) zu einer Destillationskolonne K3 gefahren, die mit Umlaufverdampfer und Kopfkondensator sowie mit strukturierter Packung ausgestattet ist, die 30 theoretische Trennstufen erzeugen. Die Kolonne K3 wird bei einem absoluten Kopfdruck von 180 mbar, 345 K Kopftemperatur und 363 K Sumpfabzugstemperatur betrieben.

In die Kolonne K3 werden 39 kg/h Rückführstrom 5 aus der Kolonne K5 in Schritt (5) gefahren, enthaltend 54 % T3PN, 23 % 2M3BN und 16 % Z2M2BN sowie in geringen Mengen weitere Pentennitril-Isomere.

Über Kopf der Kolonne K3 werden 40 kg/h eines Strom 13 erhalten, enthaltend 10 % T3PN, 68 % 2M3BN, 16 % Z2M2BN sowie in Summe 0,1 % BD und C2BU und geringe Mengen anderer Pentennitril-Isomere (T2PN, C2PN, C3PN, 4PN).

Über Sumpf der Kolonne K3 werden 39 kg/h des Stromes 12 erhalten, enthaltend in Summe 97 % T3PN, C3PN und 4PN und geringe Mengen anderer Pentennitril-Isomere (T2PN, C2PN) sowie ca. 100 ppm 2M3BN und ca. 1 % E2M2BN.

In Beispiel 1 wird für die Isomerisierung von 2M3BN zu T3PN ein Katalysatorsystem auf Basis von Nickel(0)-Komplexen mit einem Gemisch von Liganden eingesetzt. Die Ligandmischung zur Isomerisierung (im Folgenden als Isomerisierungsligand bezeichnet) enthält gemischte Phosphitliganden der Klasse P(OR)(OR')(OR") mit statistisch verteilten R, R', R" aus der Gruppe m-Tolyl, p-Tolyl, o-Isopropylphenyl, wobei ca 40 Mol% der Summe der Reste R, R', R" o-Isopropylphenyl-Reste sind. Solche Ligandmischungen werden bei der Umsetzung eines Gemisches von m- und p-Kresol mit einem Verhältnis von 2:1 von m-Kresol gegenüber p-Kresol und einer stöchiometrisch angepassten Menge o-Isopropylphenol mit einem Phosphortrihalogenid erhalten.

Der Strom 13 wird in einem Schritt (4) zusammen mit einem Katalysator-Rückführstrom 3a und einem Katalysatorergänzungsstrom in einen Reaktor R2 gefahren, ausgeführt als Rohrreaktor, der auf 393 K temperiert wird. Als Summe aus Rückführkatalysator und Frischkatalysator werden in den Reaktor R2 56 kg/h einer Mischung mit 20 % T3PN, 5 % 2M3BN und anderen Pentennitrilisomeren, 55 % Isomerisierungsligand und 0,5 % Nickel(0) sowie einem geringen Gehalt an Katalysatorabbauprodukten gefahren.

Als Produkt aus dem Reaktor R2 werden 96 kg/h Strom 1 erhalten, enthaltend 34 % T3PN, 12,3 % 2M3BN und geringe Mengen anderer Pentennitril-Isomere (T2PN, C2PN, C3PN, 4PN), entsprechend einem Umsatz von 60 % 2M3BN.

Strom 1 wird in einem Schritt (5) in eine Destillationskolonne K5 gefahren, die als Verstärkungskolonne betrieben wird und mit Fallfilmverdampfer, Kopfkondensator, Rücklaufteiler, gasförmigem Seitenabzug im Sumpfbereich der Kolonne sowie Kolonneneinbauten mit strukturierter Packung ausgestattet ist, die 30 theoretische Trennstufen erzeugen. Die Kolonne wird bei einem absoluten Kopfdruck von 250 mbar, 353 K Kopftemperatur und 373 K Sumpfabzugstemperatur betrieben.

In Kolonne K5 wird über Sumpf der zurück gewonnene Katalysatorstrom 3 (56 kg/h) erhalten, enthaltend 20 % T3PN neben anderen Pentennitrilen, ca. 5 % MGN sowie 0,5 % Ni(0) und 54 % Isomerisierungsligand. Ein geringer Teil des Stromes 3 wird zur Begrenzung der Aufpegelung von Katalysatordesaktivierungskomponenten und MGN als Strom 3b ausgeschleust. Zur Ergänzung der ausgeschleusten Katalysatormenge wird so viel Frischkatalysator, enthaltend 15 % T3PN neben anderen Pentennitril-Isomeren, 1 % Ni(0) und 80 % Isomerisierungsligand, zudosiert, dass der Ni(0)-Gehalt im Katalysatorzulauf zum Reaktor R2 bei 0,5 % gehalten wird.

In Kolonne K5 wird über Kopf ein Strom 4 erhalten (0.8 kg/h), enthaltend in Summe 0,5 % BD und C2BU, 50 % 2M3BN, 41 % Z2M2BN, sowie geringe Mengen Vinylcyclohexen (VCH), das einerseits in Spuren im Einsatzmaterial BD enthalten ist und andererseits in geringen Mengen in der Hydrocyanierung von Butadien gebildet wird und sich letztendlich im 2M3BN-Kreislauf der Isomerisierung aufpegelt und zusammen mit 2M3BN ausgeschleust werden muss, da die Dampfdrücke von 2M3BN und VCH so nahe beieinander liegen, dass eine Trennung durch gewöhnliche Destillation nicht möglich ist. Das Rücklaufverhältnis der Kolonne K5 wird so eingestellt, dass 10 ppm T3PN im Strom 4 enthalten sind. Die Abzugsmenge von Strom 4 vom Kopf der Kolonne K5 wird so eingestellt, dass im Kopfabzugsstrom 13 der Destillationskolonne K3 in Summe 20 % Z2M2BN und VCH enthalten sind.

In Kolonne K5 wird über den gasförmigen Seitenabzug ein Strom 5 erhalten (39 kg/h), der neben 3-Pentennitrilen im Wesentlichen das in der Isomerisierung nicht umgesetzte 2M3BN enthält und nach Kondensation flüssig in die Kolonne K3 wie oben beschrieben zurückgefahren wird.

### Beispiel 2:

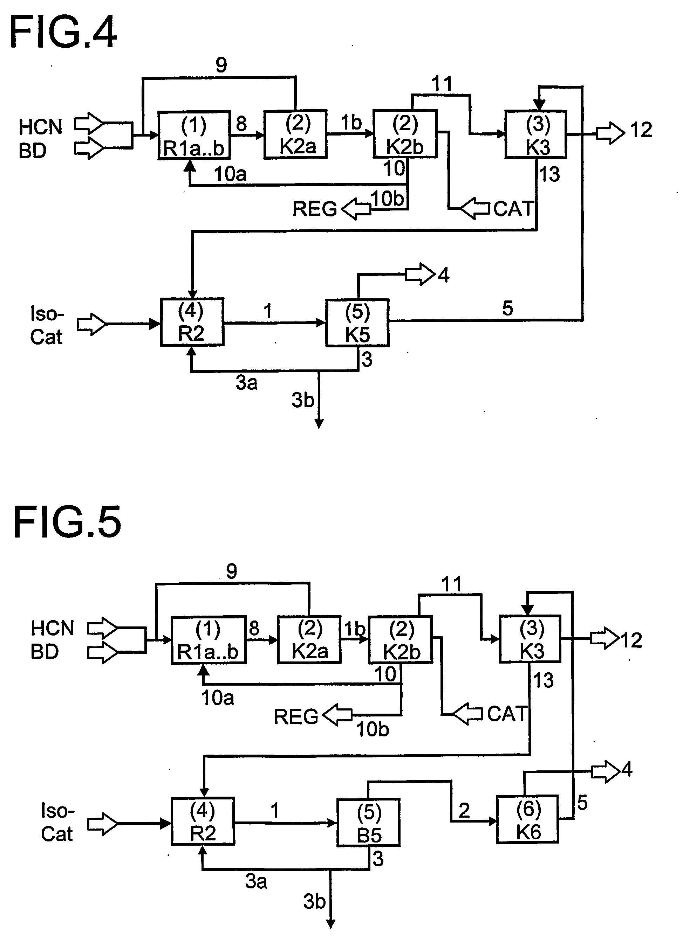

Beispiel 2 wird anhand von Figur 4 verdeutlicht.

In Beispiel 2 wird für die Hydrocyanierung von BD ein Katalysatorsystem auf Basis von Nickel(0)-Komplexen mit Chelatphosphit **2** als Ligand verwendet:

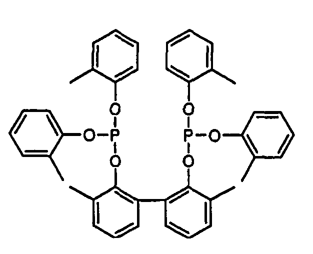

In einem Schritt (1) werden folgende Ströme in ein System aus zwei Reaktoren R1a und R1b von je 12 I Volumen gefahren, die jeweils mit einer Düse, Impulsaustauschrohr, externen Umpumpkreislauf und im einem im Umpumpkreislauf befindlichen Wärmetauscher zur Abfuhr der Reaktionsenergie ausgestattet sind und auf 363 K temperiert sind:
(1) 6 kg/h flüssiger, unstabilisierter, durch Destillation von Wasser befreiter Cyanwasserstoff zu R1a,
(2) 6 kg/h flüssiger, unstabilisierter, durch Destillation von Wasser befreiter Cyanwasserstoff zu R1b,
(3) 25 kg/h BD zu R1a, enthaltend 0,25 % C2BU, das durch Kontakt mit Aluminiumoxid behandelt wurde, um Wasser und Stabilisator TBC zu entfernen,
(4) 2 kg/h rückgeführtes BD aus Kolonne K2a in Schritt (2) zu R1a (Strom 9), so dass als gesamter BD-Zulauf zum Reaktor R1 ein Strom von 27 kg/h enthaltend 98 % BD und in Summe 2 % C2BU und 1-Buten erhalten wird,
(5) 14 kg/h Nickel(0)-Katalysatorlösung zu R1a, erhalten wie in diesem Beispiel weiter unten beschrieben als Strom 10a aus der Kolonne K2b

Der aus dem Reaktor R1b abgezogene Strom 8 (54 kg/h) enthält in Summe 4 % BD und C2BU, entsprechend einem Umsatz von 94 % BD, sowie in Summe 74 % Pentennitrile, davon 33 % T3PN, 37 % 2M3BN und geringe Mengen Z2M2BN und E2M2BN, neben anderen Pentennitril-Isomeren sowie die Katalysatorbestandteile und Katalysatorabbauprodukte und MGN.

Strom 8 wird in einem Schritt (2) in einer Destillationskolonne K2a zugeführt, die als Verstärkungskolonne betrieben wird und mit einem Fallfilmverdampfer ausgestattet ist, sowie Kolonneneinbauten mit strukturierter Packung enthält, die 4 theoretische Trennstufen erzeugen. Kolonne K2a wird am Kopf mit einem Direktkondensator betrieben der aus einem mit Füllkörperschüttung bestückten Kolonnenschuss mit Totalfangtasse, Umpumpkreis und externem Wärmetauscher besteht. Die Kolonne K2a wird bei einem absoluten Kopfdruck von 0,8 bar, 263 K Kopftemperatur und 393 K Sumpfabzugstemperatur betrieben.

Über Kopf der Kolonne K2a wird der Strom 9 erhalten, der wie eingangs beschrieben als Rückführstrom in den Reaktor R1a dosiert wird. Das Rücklaufverhältnis am Kopf der Kolonne K2a wird so eingestellt, dass der Strom 9 0,1 % 2M3BN enthält.

Über Sumpf der Kolonne K2a werden 52 kg/h eines Stromes 1b erhalten, der 0,3 % BD, 0,1 % C2BU, 76 % Pentennitrile sowie zusätzlich die Katalysatorbestandteile enthält.

Strom 1b wird innerhalb des Schrittes (2) in eine Destillationskolonne K2b gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 4 theoretische Trennstufen erzeugen. Die Kolonne wird bei einem absoluten Kopfdruck von 70 mbar, 333 K Kopftemperatur und 373 K Sumpfabzugstemperatur betrieben.

An der Kolonne K2b wird der gasförmige Kopfabzug Strom 11 gewonnen (40 kg/h), enthaltend 0,4 % BD, 54 % 2M3BN und 42 % T3PN sowie in geringerem Umfang E2M2BN und Z2M2BN neben anderen Pentennitril-Isomeren.

In die Kolonne K2b werden 3 kg/h eines Katalysatorstromes gefahren, enthaltend in Summe 45 % Pentennitrile, 1,5 % Ni(0) und den Chelatligand, erhalten beispielsweise durch Umsetzung von Nickel(0)(Cyclooctadienyl)₂-Komplex mit dem Chelatphosphit 2. An der Kolonne K2b wird über Sumpf der Katalysator-Strom 10 erhalten, enthaltend 1,2 % Ni(0), 0,3 % 2M3BN und 17 % restliche Pentennitrile. Der Strom 10 wird teilweise in den Reaktor R1 zurückgefahren (14 kg/h) (Strom 10a). Ein anderer Teil (Strom 10b) (3,8 kg/h) wird einer Regenerierung gemäß der DE A-103 51 002 zugeführt, um in der Hydrocyanierung von 3-Pentennitril gemäß der DE-A-102 004 004 683 eingesetzt oder gegebenenfalls in die Hydrocyanierung von BD gemäß dem erfindungsgemäßem Verfahren zurückgeführt zu werden.

Der Strom 11 wird in einem Schritt (3) zu einer Destillationskolonne K3 gefahren, die mit Umlaufverdampfer und Kopfkondensator sowie mit strukturierter Packung ausgestattet ist, die 45 theoretische Trennstufen erzeugen. Die Kolonne K3 wird bei einem absoluten Kopfdruck von 1,0 bar, 395 K Kopftemperatur und 416 K Sumpfabzugstemperatur betrieben.

In die Kolonne K3 werden 24 kg/h Rückführstrom 5 aus der Kolonne K5 in Schritt (5) gefahren, enthaltend 70 % T3PN, 14 % 2M3BN und 7 % Z2M2BN sowie in geringen Mengen weitere Pentennitril-Isomere.

Über Kopf der Kolonne K3 werden 30 kg/h eines Strom 13 erhalten, enthaltend 1 % T3PN, 85 % 2M3BN, 8 % Z2M2BN sowie in Summe 3 % BD und C2BU neben anderen Pentennitril-Isomeren und VCH. Das Rücklaufverhältnis der Kolonne K3 wird so eingestellt, dass über Kopf 1 % T3PN erhalten werden.

Über Sumpf der Kolonne K3 werden 38 kg/h des Stromes 12 erhalten, enthaltend in Summe 97 % T3PN, C3PN und 4PN sowie ca. 10 ppm 2M3BN und ca. 2 % E2M2BN und in geringen Mengen MGN sowie andere Pentennitril-Isomere.

In Beispiel 2 wird für die Isomerisierung der Chelatphosphit basierte Nickel(0)-Komplex als Katalysator eingesetzt, wie für die Hydrocyanierung von BD in diesem Beispiel beschrieben.

Der Strom 13 wird in einem Schritt (4) zusammen mit einem Katalysator-Rückführstrom 3a und einem Katalysatorergänzungsstrom in einen Reaktor R2 gefahren, ausgeführt als kompartimentierter Reaktor mit Rohrcharakteristik und ausgestattet mit einem Vorerhitzer, mit dem die Reaktionsmischung auf 383 K erwärmt wird. Als Summe aus Rückführkatalysator und Frischkatalysator werden in den Reaktor R2 12 kg/h einer Mischung mit 20 % T3PN, 3 % 2M3BN und anderen Pentennitrilisomeren, 71 % Ligandgemisch und 0,6 % Nickel(0) sowie einem geringen Gehalt an Katalysatorabbauprodukten gefahren.

Als Produkt aus dem Reaktor R2 werden 43 kg/h Strom 1 erhalten, enthaltend 53 % T3PN, 12 % 2M3BN, entsprechend einem Umsatz von 80 % 2M3BN.

Strom 1 wird in einem Schritt (5) in eine Destillationskolonne K5 gefahren, die mit Fallfilmverdampfer, Kopfkondensator, Rücklaufteiler, gasförmigem Seitenabzug im Sumpfbereich der Kolonne sowie Kolonneneinbauten ausgestattet ist, die 30 theoretische Trennstufen erzeugen. Die Kolonne wird bei einem absoluten Kopfdruck von 377 mbar, 355 K Kopftemperatur und 368 K Sumpfabzugstemperatur betrieben.

In Kolonne K5 wird über Sumpf der zurückgewonnene Katalysatorstrom 3 (11 kg/h) erhalten, enthaltend 20 % T3PN neben anderen Pentennitrilen, ca. 1 % MGN sowie 0,6 % Ni(0) und 54 % Ligand. Ein geringer Teil (Strom 3b) wird zur Begrenzung der Aufpegelung von Katalysatordesaktivierungskomponenten und MGN ausgeschleust. Zur Ergänzung der ausgeschleusten Katalysatormenge wird so viel Frischkatalysator, enthaltend 40 % Pentennitrilisomere, 1,2 % Ni(0) und 55 % Ligandgemisch, zudosiert, dass der Ni(0)-Gehalt im Katalysatorzulauf zum Reaktor R2 bei 0,6 % gehalten wird.

In Kolonne K5 wird über Kopf ein Strom 4 erhalten (1,4 kg/h), enthaltend in Summe 18 % BD und C2BU, 45 % 2M3BN, 28 % Z2M2BN, sowie geringe Mengen Vinylcyclohexen (VCH). Das Rücklaufverhältnis der Kolonne K5 wird so eingestellt, dass 10 ppm T3PN im Strom 4 enthalten sind. Die Abzugsmenge von Strom 4 vom Kopf der Kolonne K8 wird so eingestellt, dass im Kopfabzugsstrom 13 der Destillationskolonne K3 in Summe 10 % Z2M2BN und VCH enthalten sind.

In Kolonne K5 wird über den gasförmigen Seitenabzug ein Strom 5 erhalten (24 kg/h), der neben 3-Pentennitrilen im Wesentlichen das in der Isomerisierung nicht umgesetzte 2M3BN enthält und nach Kondensation flüssig in die Kolonne K3 wie oben beschrieben zurückgefahren wird.

### Beispiel 3:

Beispiel 3 wird anhand von Figur 5 verdeutlicht.

In Beispiel 3 wird für die Hydrocyanierung von Butadien ein Katalysatorsystem auf Basis von Nickel(0)-Komplexen mit einem Gemisch von Liganden eingesetzt. Die Ligandmischung zur Hydrocyanierung enthält ca. 60 Mol% Tri(m/p-tolyl)phosphit und 40 Mol% des Chelatphosphts 2.

In einem Schritt (1) werden folgende Ströme in ein System aus zwei Reaktoren R1a und R1b von je 12 I Volumen gefahren, die jeweils mit einer Düse, Impulsaustauschrohr, externen Umpumpkreislauf und im einem im Umpumpkreislauf befindlichen Wärmetauscher zur Abfuhr der Reaktionsenergie ausgestattet sind und auf 363 K temperiert sind:
(1) 6 kg/h flüssiger, unstabilisierter, durch Destillation von Wasser befreiter Cyanwasserstoff zu R1a,
(2) 6 kg/h flüssiger, unstabilisierter, durch Destillation von Wasser befreiter Cyanwasserstoff zu R1 b,
(3) 25 kg/h handelsübliches BD zu R1a, enthaltend 0,25 % C2BU, das durch Kontakt mit Aluminiumoxid behandelt wurde, um Wasser und Stabilisator TBC zu entfernen,
(4) 2 kg/h rückgeführtes BD aus Kolonne K2a in Schritt (2) zu R1a (Strom 9), so dass als gesamter BD-Zulauf zum Reaktor R1 ein Strom von 27 kg/h enthaltend 98 % BD und in Summe 2 % C2BU und 1-Buten erhalten wird,
(5) 14 kg/h Nickel(0)-Katalysatorlösung zu R1a, erhalten wie in diesem Beispiel weiter unten beschrieben als Strom 10a aus der Kolonne K2b

Der aus dem Reaktor R1b abgezogene Strom 8 (54 kg/h) enthält in Summe 4 % BD und C2BU, entsprechend einem Umsatz von 94 % BD, sowie in Summe 74 % Pentennitrile, davon 33 % T3PN, 37 % 2M3BN und geringe Mengen Z2M2BN und E2M2BN, andere Pentennitril-Isomere, sowie die Katalysatorbestandteile und Katalysatorabbauprodukte und MGN.

Strom 8 wird in einem Schritt (2) in einer Destillationskolonne K2a zugeführt, die als Verstärkungskolonne betrieben wird und mit einem Fallfilmverdampfer ausgestattet ist, sowie Kolonneneinbauten mit strukturierter Packung enthält, die 4 theoretische Trennstufen erzeugen. Kolonne K2a wird am Kopf mit einem Direktkondensator betrieben der aus einem mit Füllkörperschüttung bestückten Kolonnenschuss mit Totalfangtasse, Umpumpkreis und externen Wärmetauscher besteht. Die Kolonne K2a wird bei einem absoluten Kopfdruck von 0,8 bar, 263 K Kopftemperatur und 393 K Sumpfabzugstemperatur betrieben.

Über Kopf der Kolonne K2a wird der Strom 9 erhalten, der wie eingangs beschrieben als Rückführstrom in den Reaktor R1a dosiert wird. Das Rücklaufverhältnis am Kopf der Kolonne K2a wird so eingestellt, dass der Strom 9 0,1 % 2M3BN enthält.

Über Sumpf der Kolonne K2a werden 52 kg/h eines Stromes 1b erhalten, der 0,3 % BD, 0,1 % C2BU, 76 % Pentennitrile sowie zusätzlich die Katalysatorbestandteile enthält.

Strom 1b wird innerhalb des Schrittes (2) in eine Destillationskolonne K2b gefahren, die in Abtriebsfahrweise betrieben wird und mit Fallfilmverdampfer, Kopfkondensator mit Nachkondensator sowie Kolonneneinbauten mit strukturierter Packung ausgestattet ist, die 4 theoretische Trennstufen erzeugen. Die Kolonne wird bei einem absoluten Kopfdruck von 70 mbar, 333 K Kopftemperatur und 373 K Sumpfabzugstemperatur betrieben.

An der Kolonne K2b wird der gasförmige Kopfabzug Strom 11 gewonnen (40 kg/h), enthaltend 0,4 % BD, 54 % 2M3BN und 42 % T3PN sowie in geringerem Umfang E2M2BN und Z2M2BN neben anderen Pentennitril-Isomeren.

In die Kolonne K2b werden 5 kg/h eines Katalysatorstromes gefahren, der wie in Beispiel 1 gemäß der DE A-102 004 004 683 beschrieben als Sumpfabzug der Kolonne K4 aus Schritt (4) von Beispiel 2 erhalten wird, enthaltend in Summe 45 % Pentennitrile, 1,1 % Ni(0), 38 % Ligandmischung sowie ca. 12 % ADN.

An der Kolonne K2b wird über Sumpf der Katalysator-Strom 10 erhalten, enthaltend 1,2 % Ni(0), 0,3 % 2M3BN und 17 % restliche Pentennitrile. Der Strom 10 wird teilweise in den Reaktor R1 zurückgefahren (14 kg/h) (Strom 10a). Ein anderer Teil (Strom 10b) (3,8 kg/h) wird einer Regenerierung gemäß der DE-A-103 51 002 zugeführt, um in der Hydrocyanierung von 3-Pentennitril gemäß der DE-A-102 004 004 683 eingesetzt zu werden.

Der Strom 11 wird in einem Schritt (3) zu einer Destillationskolonne K3 gefahren, die mit Umlaufverdampfer und Kopfkondensator sowie mit strukturierter Packung ausgestattet ist, die 45 theoretische Trennstufen erzeugen. Die Kolonne K3 wird bei einem absoluten Kopfdruck von 1,0 bar, 395 K Kopftemperatur und 416 K Sumpfabzugstemperatur betrieben.

In die Kolonne K3 werden 28 kg/h Rückführstrom 5 aus der Kolonne K6 in Schritt (6) gefahren, enthaltend 72 % T3PN, 15 % 2M3BN und 8 % Z2M2BN sowie in geringen Mengen weitere Pentennitril-Isomere.

Ober Kopf der Kolonne K3 werden 30 kg/h eines Strom 13 erhalten, enthaltend 1 % T3PN, 85 % 2M3BN, 8 % Z2M2BN sowie in Summe 3 % BD und C2BU und weitere Pentennitril-Isomere. Das Rücklaufverhältnis der Kolonne K3 wird so eingestellt, dass über Kopf 1 % 3PN erhalten werden.

Ober Sumpf der Kolonne K3 werden 38 kg/h des Stromes 12 erhalten, enthaltend in Summe 97 % T3PN, C3PN und 4PN sowie ca. 10 ppm 2M3BN und ca. 2 % E2M2BN und in geringen Mengen MGN und weitere Pentennitril-Isomere.

In Beispiel 3 wird für die Isomerisierung von 2M3BN zu T3PN ein Katalysatorsystem auf Basis von Nickel(0)-Komplexen mit einem Gemisch von Liganden eingesetzt. Die Ligandmischung zur Isomerisierung (im Folgenden als Isomerisierungsligand bezeichnet) enthält gemischte Phosphitliganden der Klasse P(OR)(OR')(OR") mit statistisch verteilten R, R', R" aus der Gruppe Phenyl, m-Tolyl, p-Tolyl, o-Tolyl, wobei mehr als 80 Mol% der Summe der Reste R, R', R" m-Tolyl und p-Tolyl-Reste sind. Solche Ligandmischungen werden bei der Umsetzung eines Gemisches von m- und p-Kresol (mit einem Mischungsverhältnis von 2:1) von m- gegenüber p-Kresol mit einem Phosphortrihalogenid erhalten. Als Promotor für die Isomerisierungsreaktion wird Zinkchlorid eingesetzt wie in US 3,676,481, US 3,852,329 und US4,298,546 beschrieben.

Der Strom 13 wird in einem Schritt (4) zusammen mit einem Katalysator-Rückführstrom 3a und einem Katalysatorergänzungsstrom in einen Reaktor R2 gefahren, ausgeführt als kompartimentierter Reaktor mit Rohrcharakteristik und ausgestattet mit einem Vorerhitzer, mit dem die Reaktionsmischung auf 383 K erwärmt wird. Als Summe aus Rückführkatalysator und Frischkatalysator werden in den Reaktor R2 12 kg/h einer Mischung mit 20 % T3PN, 3 % 2M3BN und anderen Pentennitrilisomeren, 71 % Isomerisierungsligand und 0,6 % Nickel(0) sowie einem geringen Gehalt an Katalysatorabbauprodukten gefahren.

Als Produkt aus dem Reaktor R2 werden 43 kg/h Strom 1 erhalten, enthaltend 53 % T3PN, 12 % 2M3BN, entsprechend einem Umsatz von 80 % 2M3BN.

Strom 1 wird in einem Schritt (5) in eine Verdampferstufe B5 gefahren, die mit Zwangsumlaufverdampfer und Kopfkondensator ausgestattet ist. Die Verdampferstufe B5 wird bei einem absoluten Druck von 510 mbar, 403 K Sumpfabzugstemperatur und 366 K Kondensationstemperatur betrieben.

In Verdampferstufe B5 wird über Sumpf der zurück gewonnene Katalysatorstrom 3 (11 kg/h) erhalten, enthaltend 20 % T3PN neben anderen Pentennitrilen, ca. 10 % MGN sowie 0,5 % Ni(0) und 61 % Ligandgemisch. Ein geringer Teil (Strom 3b) wird zur Begrenzung der Aufpegelung von Katalysatordesaktivierungskomponenten und MGN ausgeschleust. Zur Ergänzung der ausgeschleusten Katalysatormenge wird so viel Frischkatalysator zudosiert, enthaltend ca. 15 % Pentennitrilisomere, ca. 2,0 % Ni(0), ca. 70 % Isomerisierungsligand und den Promtor Zinkchlorid in einer Konzentration, die einem molaren Verhältnis ZnCl₂ zu Nickel(0) von ca. 5 entspricht, dass der Ni(0)-Gehalt im Katalysatorzulauf zum Reaktor R2 bei 0,6 % gehalten wird.

In der Verdampferstufe B5 wird am Kopfkondensator der Strom 2 erhalten (25 kg/h) , enthaltend 1 % BD, 68 % T3PN, 16 % 2M3BN und weitere Pentennitrile sowie geringe Mengen VCH.

Strom 2 wird in einem Schritt (6) in die Destillationskolonne K6 gefahren, die als Verstärkungskolonne betrieben wird und mit Umlaufverdampfer, Kopfkondensator, sowie Kolonneneinbauten ausgestattet ist, die 30 theoretische Trennstufen erzeugen. Die Kolonne wird bei einem absoluten Kopfdruck von 340 mbar, 357 K Kopftemperatur, 313 K im Kondensator und 373 K Sumpfabzugstemperatur betrieben.

Am Kondensator der Kolonne K6 wird als Gasphase ca. 100 Norm-I/h eines Stromes erhalten, der im Wesentlichen aus BD besteht.

In Kolonne K6 wird am Kopfkondensator als flüssige Phase ein Strom 4 erhalten (1,1 kg/h), enthaltend in Summe 5 % BD und C2BU, 50 % 2M3BN, 30 % Z2M2BN, sowie geringe Mengen Vinylcyclohexen (VCH). Das Rücklaufverhältnis der Kolonne K6 wird so eingestellt, dass 1 ppm T3PN im Strom 4 enthalten sind. Die Abzugsmenge von Strom 4 vom Kopf der Kolonne K6 wird so eingestellt, dass im Zulauf zum Reaktor R2 in Summe 10 % Z2M2BN und VCH enthalten sind.

In Kolonne K6 wird über Sumpf ein Strom 5 erhalten (24 kg/h), der neben 3-Pentennitrilen im Wesentlichen das in der Isomerisierung nicht umgesetzte 2M3BN enthält und in die Kolonne K3 wie oben beschrieben zurückgefahren wird.

### Beispiel 4:

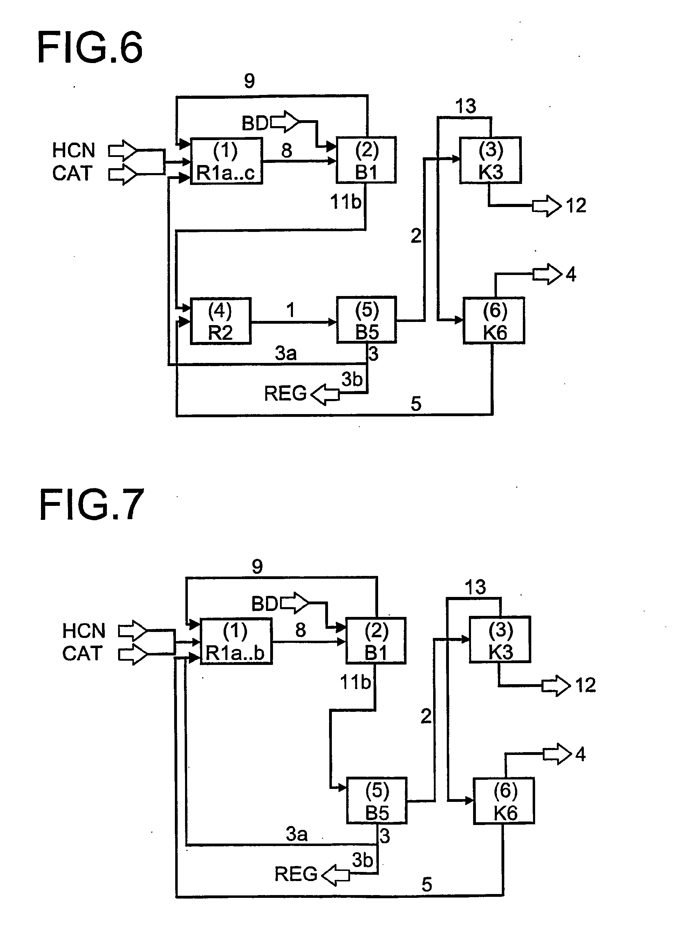

Beispiel 4 wird anhand Figur 6 verdeutlicht.

In Beispiel 3 wird für die Hydrocyanierung von Butadien ein Katalysatorsystem auf Basis von Nickel(0)-Komplexen mit einem Gemisch von Liganden eingesetzt. Die Ligandmischung zur Hydrocyanierung enthält ca. 80 Mol% Tri(m/p-tolyl)phosphit und 20 Mol% des Chelatphosphits 2 (siehe Beispiel 2).

In einem Schritt (1) werden folgende Ströme in ein System aus drei hintereinandergeschalteten kontinuierlich betriebenen Rührkesseln R1 a, R1 b und R1 c von je 10 I Volumen gefahren, die auf 373 K temperiert sind:
(1) 5,2 kg/h flüssiger, unstabilisierter, durch Destillation von Wasser befreiter Cyanwasserstoff zu R1a,
(2) 4.0 kg/h flüssiger, unstabilisierter, durch Destillation von Wasser befreiter Cyanwasserstoff zu R1b,
(3) 20 kg/h 1 BD als Strom 9 vom Kondensator des Verdampfers B1 in Schritt (2), enthaltend 92 % BD, 2 % T3PN, 4 % 2M3BN und ca. 2% C2BU zu R1a,
(4) 4,1 kg/h Nickel(0)-Katalysatorlösung zu R1a, erhalten wie in diesem Beispiel weiter unten beschrieben als Strom 3a aus der Verdampferstufe B5 in Schritt (5),
(5) 3,7 kg/h Nickel(0)-Katalysatorlösung zu R1a, erhalten wie in Beispiel 3 gemäß der deutschen Patentanmeldung mit dem Titel "Verfahren zur Herstellung von Dinitrilen" der BASF AG (B03/0525) beschrieben als Sumpfabzug der Kolonne K4 aus Schritt (4) von Beispiel 2 erhalten wird, enthaltend in Summe 45 % Pentennitrile, 1,1 % Ni(0), 38 % Ligandmischung sowie ca. 12 % ADN.

Der Reaktor R1c wird als Nachreaktor mit dem Ablauf aus Reaktor R1b bei 353 K betrieben.

Der aus dem Reaktor R1c abgezogene Strom 8 (37 kg/h) enthält 1 % BD, entsprechend einem Umsatz von 98 % BD, sowie in Summe 82 % Pentennitrile, davon 36 % T3PN, 44 % 2M3BN und geringe Mengen Z2M2BN und E2M2BN, sowie die Katalysatorbestandteile und Katalysatorabbauprodukte und MGN und weitere Pentennitril-Isomere.

Strom 8 wird in einem Schritt (2) in einer Verdampferstufe B1 zugeführt, die mit einem Umlaufverdampfer ausgestattet ist. Die Verdampferstufe B1 wird am Kopf mit einem Kondensator betriebenen, der mit kondensiertem Material aus dem Rücklaufbehälter gespült wird. Die Verdampferstufe B1 wird bei einem absoluten Kopfdruck von 0,6 bar, 253 K Kondensationstemperatur und 363 K Sumpfabzugstemperatur betrieben.

In den Kondensatsammelbehälter der Verdampferstufe B1 werden 19,5 kg/h handelsübliches BD dosiert, enthaltend 0,25 % C2BU, das durch Kontakt mit Molsieb behandelt wurde, wobei der Wassergehalt vom eingesetzten BD auf kleiner 10 Gew.-ppm Wasser entfernt wurde.

Aus dem Kondensatsammelbehälter der Verdampferstufe B1 wird Strom 9 als Summe von rückgeführtem und frisch zudosiertem Butadien abgezogen und zum Reaktor R1a wie vorher beschrieben zurückgeführt.

Über Sumpf der Verdampferstufe B1 werden 37 kg/h eines Stromes 11b erhalten, der 1 % BD, 82 % Pentennitrile sowie zusätzlich die Katalysatorbestandteile enthält.

Strom 11 b wird in einem Schritt (4) in einen auf 383K temperierten Reaktor R2, ausgeführt als Rührkessel mit nachgeschalteter Verweilzeitstrecke gefahren, wobei 2M3BN in Gegenwart des Nickelkatalysators zu T3PN isomerisiert wird.

In den Reaktor R2 wird ein Pentennitril-Rückführstrom 5 gefahren (10 kg/h), der in Schritt (6) in Kolonne 6 als Sumpfprodukt erhalten wird, enthaltend 60 % 2M3BN, in Summe 10 % T3PN mit weiteren Pentennitrilisomeren sowie VCH und in geringen Mengen BD.

Aus Reaktor R2 wird ein Strom 1 erhalten (45 kg/h), enthaltend 62 % T3PN und 14 % 2M3BN, entsprechend einem Umsatz von 70 % 2M3BN zu T3PN, sowie die Katalysatorkomponenten.

Strom 1 wird in einem Schritt (5) in eine Verdampferstufe B5 gefahren, die mit Fallfilmverdampfer und Kondensator ausgestattet ist und bei einem absoluten Druck von 50 mbar und 393 K Sumpfabzugstemperatur betrieben wird.

Aus dem Kondensator der Verdampferstufe B5 wird ein Strom 2 gewonnen (38 kg/h), enthaltend 91 % Pentennitrilisomere sowie ca. 1 % BD und in geringerem Umfang E2M2BN, Z2M2BN und VCH.

An der Verdampferstufe B5 wird über Sumpf der Katalysator-Strom 3 erhalten (7,2 kg/h), enthaltend 1,2 % Ni(0), 0,1 % 2M3BN und 15 % restliche Pentennitrile. Der Strom 3 wird teilweise (Strom 3a) in den Reaktor R1 zurückgefahren (4,1 kg/h). Der Rest (Strom 3b) wird einer Regenerierung gemäß der DE-A-103 51 002 zugeführt, und kann nach der Regenerierung beispielsweise in einer Hydrocyanierung von 3-Pentennitril wie in Beispiel 2 gemäß der DE-A-102 004 004 683 eingesetzt werden oder als Katalysator im erfindungsgemäßen Verfahren zur Hydrocyanierung von Butadien, gegebenenfalls nach Abtrennung von Zinkchlorid wieder eingesetzt werden.

Der Strom 2 wird in einem Schritt (3) zu einer Destillationskolonne K3 gefahren, die mit Zwangsumlaufverdampfer und Kopfkondensator sowie mit Kolonneneinbauten ausgestattet ist, die 30 theoretische Trennstufen erzeugen. Die Kolonne K3 wird bei einem absoluten Kopfdruck von 120 mbar, 334 K Kopftemperatur und 352 K Sumpfabzugstemperatur betrieben.

Über Kopf der Kolonne K3 werden 10 kg/h eines Strom 13 erhalten, enthaltend 5 % T3PN, 60 % 2M3BN, 4 % Z2M2BN sowie in Summe 4 % BD und C2BU und im Rest überwiegend VCH. Das Rücklaufverhältnis der Kolonne K3 wird so eingestellt, dass über Kopf 5 % T3PN erhalten werden.

Über Sumpf der Kolonne K3 werden 27 kg/h des Stromes 12 erhalten, enthaltend in Summe 98 % T3PN, C3PN und 4PN sowie ca. 1000 ppm 2M3BN und ca. 2 % E2M2BN.

Strom 13 wird in einem Schritt (6) in eine Destillationskolonne K6 gefahren, die als Verstärkungskolonne betrieben wird und mit Zwangsumlaufverdampfer, Kopfkondensator, Rücklaufteiler, sowie Kolonneneinbauten mit strukturierter Packung ausgestattet ist, die 15 theoretische Trennstufen erzeugen. Die Kolonne K6 wird bei einem absoluten Kopfdruck von 380 mbar, 361 K Kopftemperatur und 365 K Sumpfabzugstemperatur betrieben.

In Kolonne K6 wird über Kopf ein flüssiger Strom 4 erhalten (0,6 kg/h), enthaltend in Summe 4 % BD und C2BU, 54 % 2M3BN, 38 % Z2M2BN, sowie 2,5 % Vinylcyclohexen (VCH). Die Abzugsmenge von Strom 4 vom Kopf der Kolonne K6 wird so eingestellt, dass am Kopfabzugsstrom 13 der Kolonne K3 in Summe 30 % Z2M2BN und VCH enthalten sind. In Kolonne K6 wird am als Teilkondensator betriebenen Kopfkondensator ein gasförmiger Strom erhalten (195 Norm-I/h), der im Wesentlichen BD enthält.

In Kolonne K6 wird über Sumpf der Strom 5 erhalten (9,4 kg/h), der neben 3-Pentennitrilen im Wesentlichen das in der Isomerisierung nicht umgesetzte 2M3BN enthält und in den Isomerisierungsreaktor R2 zurückgefahren wird.

### Beispiel 5:

Beispiel 5 wird anhand Figur 7 verdeutlicht.

In Beispiel 5 wird für die Hydrocyanierung von BD ein Katalysatorsystem auf Basis von Nickel(0)-Komplexen mit einem Gemisch von Liganden eingesetzt. Die Ligandmischung zur Hydrocyanierung enthält ca. 80 Mol% Tri(m/p-tolyl)phosphit und 20 Mol% des Chelatphosphonits 1 (siehe Beispiel 1).

In einem Schritt (1) werden folgende Ströme in ein System aus zwei hintereinandergeschalteten kontinuierlich betriebenen Rührkesseln R1a und R1b von jeweils 50 I Volumen gefahren, die auf 363 K temperiert sind:
(1) 18 kg/h flüssiger, unstabilisierter, durch Destillation von Wasser befreiter Cyanwasserstoff zugleichen Teilen auf die Reaktoren R1 a und R1 b,
(2) 62 kg/h BD als Strom 9 vom Kopf des Verdampfers B1 in Schritt (2), enthaltend 87 % BD, 3 % T3PN, 6 % 2M3BN und ca. 2 % C2BU zu Reaktor R1a,
(3) 61 kg/h Nickel(0)-Katalysatorlösung, erhalten wie in diesem Beispiel weiter unten beschrieben als Strom 3a aus der Verdampferstufe B5 in Schritt (5) zu Reaktor R1a,
(4) 6,7 kg/h Nickel(0)-Katalysatorlösung zu R1a, erhalten wie in Beispiel 1 gemäß der deutschen Patentanmeldung mit dem Titel "Verfahren zur Herstellung von Dinitrilen" der BASF AG (B03/0525) 1 beschrieben als Sumpfabzug der Kolonne K4 aus Schritt (4) von Beispiel 2 erhalten wird, enthaltend in Summe 45 % Pentennitrile, 1,1 % Ni(0), 38 % Ligandmischung sowie ca. 12 % ADN zu Reaktor R1a, wobei der Butadien-Strom und der Katalysatorstrom vor dem Kontaktieren mit Cyanwasserstoff vorgemischt werden.

Der aus dem Reaktor R1b abgezogene Strom 8 (177 kg/h) enthält 11 % BD, entsprechend einem Umsatz von 66 % BD, sowie in Summe 64 % Pentennitrile, davon 32 % T3PN, 30 % 2M3BN und geringe Mengen Z2M2BN und E2M2BN und weitere Pentennitril-Isomere, sowie die Katalysatorbestandteile und Katalysatorabbauprodukte.

Strom 8 wird in einem Schritt (2) in einer Verdampferstufe B1 zugeführt, die mit einem Fallfilmverdampfer ausgestattet ist. Die Verdampferstufe B1 wird am Kopf mit einem Kondensator betriebenen, der mit kondensiertem Material aus dem Rücklaufbehälter gespült wird. Die Verdampferstufe B1 wird bei einem absoluten Kopfdruck von 1,3 bar, 278 K Kondensationstemperatur und 403 K Sumpfabzugstemperatur betrieben.

In den Kondensatsammelbehälter der Verdampferstufe B1 werden 37 kg/h handelsübliches BD dosiert, enthaltend 0,25 % C2BU, das durch Kontakt mit Molsieb behandelt wurde, wobei der Wassergehalt vom eingesetzten BD auf kleiner 5 Gew.-ppm Wasser entfernt wurde und wobei der im eingesetzten BD enthaltene Stabilisator TBC in Konzentrationen im ppm-Maßstab im Kondensatsammelbehälter und den Kondensator-Spülkreislauf gelangt.

Aus dem Kondensatsammelbehälter der Verdampferstufe B1 wird Strom 9 als Summe von rückgeführtem und frisch zudosiertem BD abgezogen und zum Reaktor R1a wie vorher beschrieben zurückgeführt.

Über Sumpf der Verdampferstufe B1 werden 152 kg/h eines Stromes 11 b erhalten, der 0,9 % BD, 16 % 2M3BN, 51 % T3PN und weitere Pentennitrilisomere sowie zusätzlich die Katalysatorbestandteile enthält. Die Zusammensetzung des Sumpfaustrags der Verdampferstufe lässt auf einen Umsatzgrad von 50 % 2M3BN zu T3PN im Sumpf des Verdampfers B1 schliessen.

Strom 11 b wird in einem Schritt (5) in eine Verdampferstufe B5 gefahren, die mit Fallfilmverdampfer und Kondensator ausgestattet ist und bei einem absoluten Druck von 260 mbar und 383 K Sumpfabzugstemperatur betrieben wird.

Aus der Verdampferstufe B5 wird ein Strom 2 gasförmig gewonnen (83 kg/h), enthaltend 93 % Pentennitrilisomere sowei ca. 1 % BD und in geringerem Umfang E2M2BN, Z2M2BN und VCH. Strom 2 wird in die Destillationskolonne K3 in Schritt (3) gefahren.

An der Verdampferstufe B5 wird über Sumpf der Katalysator-Strom 3 erhalten (69 kg/h), enthaltend 0,6 % Ni(0), 2 % 2M3BN und 42 % restliche Pentennitrile. Der Strom 3 wird größtenteils in den Reaktor R1 zurückgefahren (61,4 kg/h) (Strom 3a). Der Rest (Strom 3b) wird einer Regenerierung gemäß der DE-A-103 51 002 zugeführt, und kann beispielsweise in der Hydrocyanierung von 3-Pentennitril, wie in Beispiel 1 gemäß der DE-A-102 004 004 683 beschrieben, eingesetzt werden.

Der Strom 2 wird in einem Schritt (3) gasförmig zu einer Destillationskolonne K3 gefahren, die mit Zwangsumalufentspannungsverdampfer und Kopfkondensator sowie mit strukturierter Packung ausgestattet ist, die 30 theoretische Trennstufen erzeugen. Die Kolonne K3 wird bei einem absoluten Kopfdruck von 80 mbar, 375 K Kopftemperatur und 343 K Sumpfabzugstemperatur betrieben.

Über Kopf der Kolonne K3 werden 36 kg/h eines Strom 13 erhalten, enthaltend 15 % T3PN, 64 % 2M3BN, 3 % Z2M2BN sowie in Summe 4 % BD und C2BU, der Rest enthält überwiegend VCH. Das Rücklaufverhältnis der Kolonne K3 wird so eingestellt, dass über Knopf 15 % T3PN erhalten werden.

Über Sumpf der Kolonne K3 werden 47 kg/h des Stromes 12 erhalten, enthaltend in Summe 98 % T3PN, C3PN und 4PN sowie 100 ppm 2M3BN und ca. 1 % E2M2BN.

Strom 13 wird in einem Schritt (6) in eine Destillationskolonne K6 gefahren, die als Verstärkungskolonne betrieben wird und mit Zwangsumlaufverdampfer, Kopfkondensator, Rücklaufteiler, sowie Kolonneneinbauten mit strukturierter Packung ausgestattet ist, die 45 theoretische Trennstufen erzeugen. Die Kolonne wird bei einem absoluten Kopfdruck von 320 mbar, 288 K Kondensationstemperatur und 363 K Sumpfabzugstemperatur betrieben.

In Kolonne K6 wird über Kopf ein flüssiger Strom 4 erhalten (6,8 kg/h), enthaltend in Summe 10 % BD und C2BU, 80 % 2M3BN, 8 % Z2M2BN, sowie 0,5 % Vinylcyclohexen (VCH). Die Abzugsmenge von Strom 4 vom Kopf der Kolonne K6 wird so eingestellt, dass am Kopfabzugsstrom 3 der Kolonne K3 in Summe 15 % Z2M2BN und VCH enthalten sind. In Kolonne K6 wird am als Teilkondensator betriebenen Kopfkondensator ein gasförmiger Strom erhalten (263 Norm-I/h), der im Wesentlichen BD enthält.

In Kolonne K6 wird über Sumpf der Strom 5 erhalten (28,7 kg/h), der neben 3-Pentennitrilen im Wesentlichen das in der Isomerisierung nicht umgesetzte 2M3BN enthält und in den Hydrocyanierungsreaktor R1 zurückgefahren wird.

### Beispiel 6:

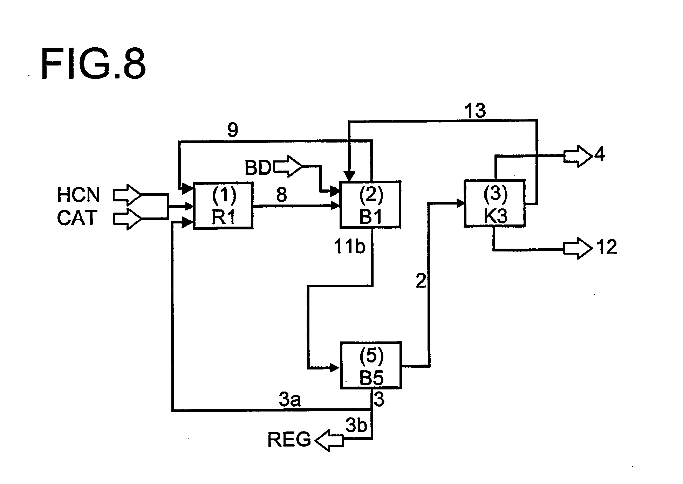

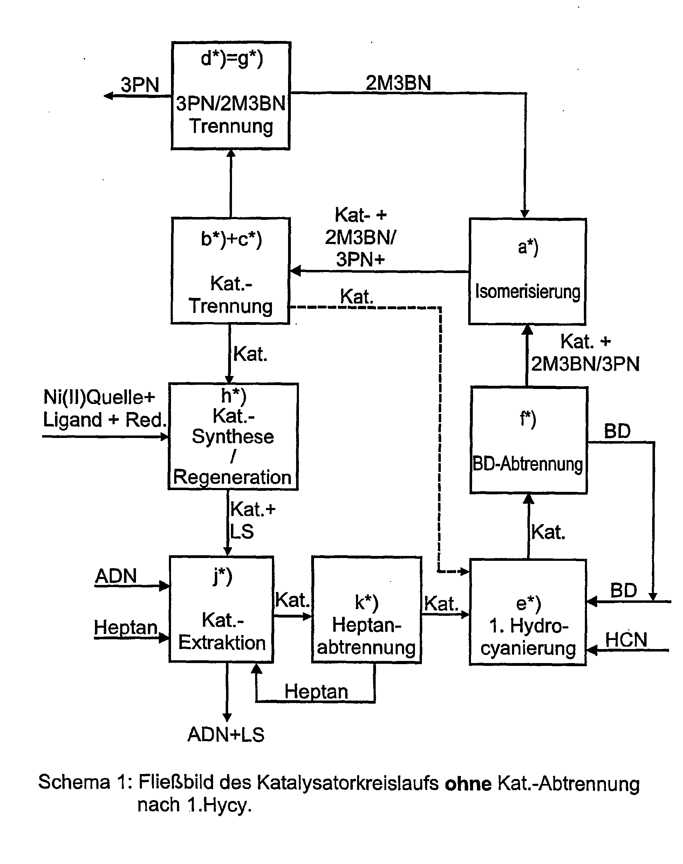

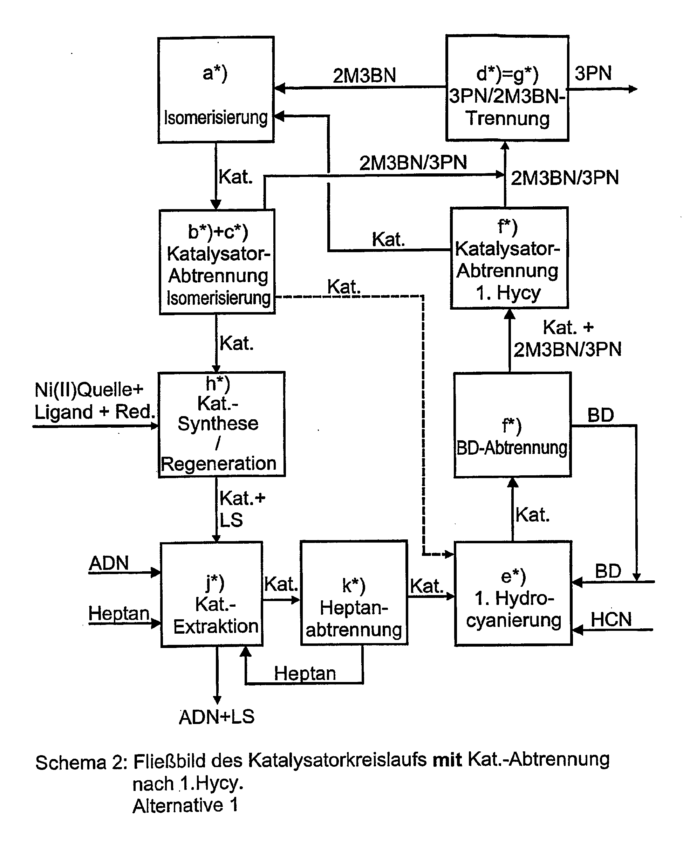

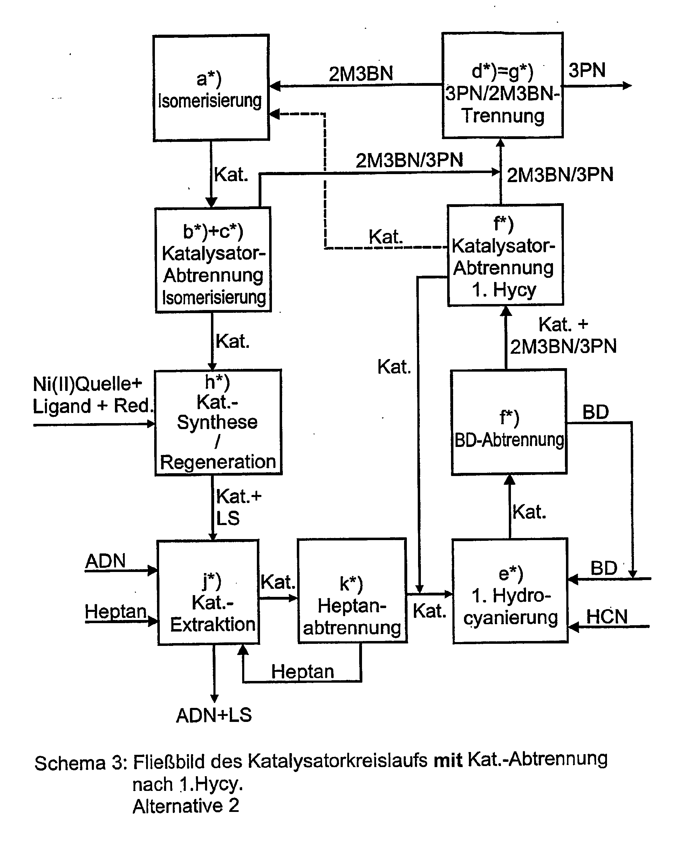

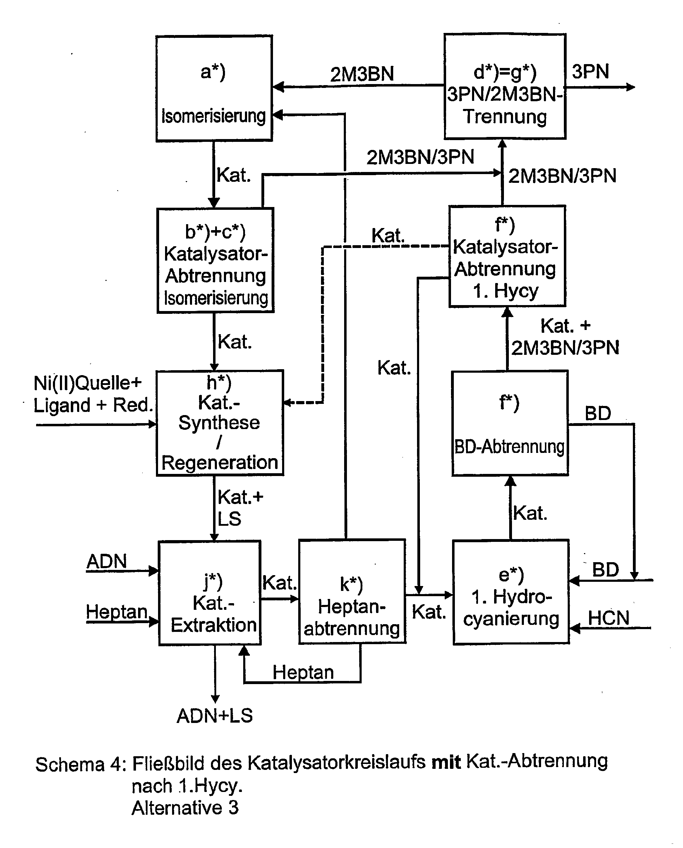

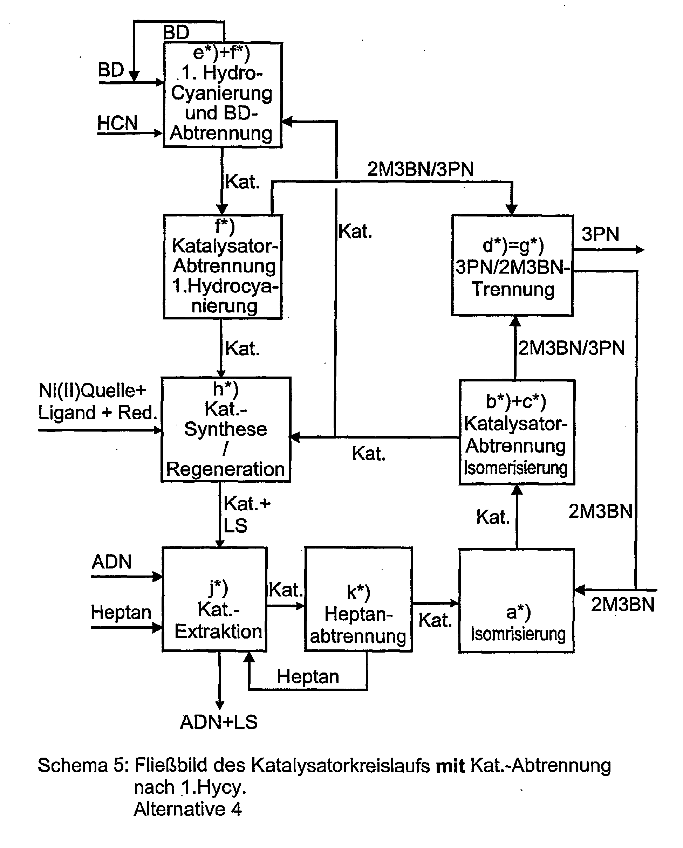

Beispiel 6 wird anhand Figur 8 verdeutlicht.

In Beispiel 8 wird für die Hydrocyanierung von BD ein Katalysatorsystem auf Basis von Nickel(0)-Komplexen mit Chelatphosphonit 1 als Ligand eingesetzt (siehe Beispiel 1).

In einem Schritt (1) werden folgende Ströme in ein einen kontinuierlich betriebenen Rührkessel R1 von 30 I Volumen gefahren, der auf 363 K temperiert ist:
(1) 16 kg/h flüssiger, unstabilisierter, durch Destillation von Wasser befreiter Cyanwasserstoff,
(2) 55 kg/h BD als Strom 9 vom Kopf des Verdampfers B1 in Schritt (2), enthaltend 87 % BD, 3 % T3PN, 6 % 2M3BN und ca. 2 % C2BU,
(3) 10 kg/h Nickel(0)-Katalysatorlösung, erhalten wie in diesem Beispiel weiter unten beschrieben als Strom 3a aus der Verdampferstufe B5 in Schritt (5), enthaltend in Summe 42 % Pentennitrile, 23 % Ligand, 0,9 % Nickel(0), sowie jeweils ca. 10 % ADN und MGN,
(4) 4 kg/h Nickel(0)-Katalysatorlösung zu R1, enthaltend in Summe 45 % Pentennitrile, 1,5 % Ni(0) und 48 % Ligand.

Der aus dem Reaktor R1 abgezogene Strom 8 (89 kg/h) enthält 17 % BD, entsprechend einem Umsatz von 71 % BD, sowie in Summe 73 % Pentennitrile, davon 32 % T3PN, 36 % 2M3BN und geringe Mengen Z2M2BN und E2M2BN, sowie die Katalysatorbestandteile und Katalysatorabbauprodukte.

Strom 8 wird in einem Schritt (2) in einer Verdampferstufe B1 zugeführt, die mit einem Fallfilmverdampfer ausgestattet ist. Die Verdampferstufe B1 wird am Kopf mit einem Kondensator betriebenen, der mit kondensiertem Material aus dem Rücklaufbehälter gespült wird. Die Verdampferstufe B1 wird bei einem absoluten Kopfdruck von 1,3 bar, 278 K Kondensationstemperatur und 403 K Sumpfabzugstemperatur betrieben.

In den Kondensatsammelbehälter der Verdampferstufe B1 werden 34 kg/h handelsübliches BD dosiert, enthaltend 0,25 % C2BU, das durch Kontakt mit Aluminiumoxid behandelt wurde, wobei der Wassergehalt vom eingesetzten BD auf kleiner 10 Gew.-ppm Wasser und der TBC-Gehalt kleiner 10 ppm verringert wurde.

Aus dem Kondensatsammelbehälter der Verdampferstufe wird Strom 9 als Summe von rückgeführtem und frisch zudosiertem Butadien abgezogen und zum Reaktor R1a, wie vorher beschrieben, zurückgeführt.

Über Sumpf der Verdampferstufe B1 werden 76 kg/h eines Stromes 5 erhalten, der 0,8 % BD, 12 % 2M3BN, 69 % T3PN und weitere Pentennitrilisomere sowie zusätzlich die Katalysatorbestandteile enthält. Die Zusammensetzung des Sumpfaustrags der Verdampferstufe entspricht einen Umsatzgrad von 75 % 2M3BN zu T3PN im Sumpf der Verdampferstufe B1.

Strom 5 wird in einem Schritt (5) in eine Verdampferstufe B5 gefahren, die mit Fallfilmverdampfer und Kondensator ausgestattet ist und bei einem absoluten Druck von 220 mbar und 381 K Sumpfabzugstemperatur betrieben wird.

Aus der Verdampferstufe B5 wird ein Strom 2 gasförmig gewonnen (58 kg/h), enthaltend 97 % Pentennitrilisomere sowie ca. 1 % BD und in geringerem Umfang E2M2BN, Z2M2BN und VCH.

An der Verdampferstufe B5 wird über Sumpf der Katalysator-Strom 3 erhalten (17 kg/h), enthaltend 0,9 % Ni(0), 0,3 % 2M3BN und 42 % restliche Pentennitrile. Der Strom 3 wird größtenteils in den Reaktor R1 zurückgefahren (10 kg/h) (Strom 3a). Der Rest (Strom 3b) wird einer Regenerierung gemäß US 2003 / 0100442 zugeführt und kann nach der Regenerierung in einer Hydrocyanierung von 3-Pentennitril verwendet werden oder in das erfindungsgemäße Verfahren in den Schritt zur Hydrocyanierung von BD zurückgefahren werden.

Der Strom 2 wird kondensiert und flüssig in einem Schritt (3) zu einer Destillationskolonne K3 gefahren, die mit Zwangsumlaufverdampfer und Kopfkondensator sowie mit strukturierter Packung ausgestattet ist, die 50 theoretische Trennstufen erzeugen. Die Kolonne K3 wird bei einem absoluten Kopfdruck von 200 mbar, 342 K Kopftemperatur und 366 K Sumpfabzugstemperatur betrieben.

Am Kopf der Kolonnen K3 wird ein Strom 4 erhalten, enthaltend 10 % BD, 18 % Z2M2BN, 68 % 2M3BN sowie weitere Pentennitril-Isomere und VCH. Das Rücklaufverhältnis der Kolonne K3 wird so eingestellt, dass der Kopfabzugsstrom 18 % Z2M2BN enthält.

An einem flüssigen Seitenabzug der Kolonne K3 werden 8 kg/h eines Stromes 13 erhalten, enthaltend 0,5 % T3PN, 85 % 2M3BN, 5 % Z2M2BN, 10 % BD Strom 13 wird in die Verdampferstufe B1 zurückgefahren.

Über Sumpf der Kolonne K3 werden 47 kg/h des Stromes 12 erhalten, enthaltend in Summe 98 % T3PN, C3PN und 4PN sowie 100 ppm 2M3BN und ca. 1 % E2M2BN.

Alle nachfolgenden Versuche wurden in einer Schutzgasatmosphäre durchgeführt.

Nickel(0)-[o-Isopropylphenyl_{0.8}-m-/p-Tolyl_{3.2}-phosphit]₁₈, (kurz: Isopropylkatalysator); entspricht einer Lösung aus 1,0 Gew.% Nickel(0) mit 19 Gew.% 3PN und 80 Gew.% o-Isopropylphenyl_{0.8}-m-/p-Tolyl_{3.2}-phosphit.

### Beispiele zur kontinuierlichen Hydrocyanierung von BD zu 2M3BN/3PN

### Beispiel 7 (Vergleich): (Verhältnis BD/HCN = 1.4:1)

2,11 mol feuchtes und stabilisiertes Butadien (100 ppm Wasser, 100 ppm TBC), 1,55 mol HCN und 14 mmol Ni in Form des Isopropylkatalysators werden pro Stunde in einen Druckreaktor eingespeist (Druck: 15 bar, Temperatur innen 105°C, Verweilzeit: ca. 40 Min/Reaktor). Der HCN-Umsatz ist nach Maßanalyse quantitativ (Titration nach Vollhard). Man bestimmt das Verhältnis 2M3BN/3PN des Reaktionsaustrages GC-chromatographisch (GC-Flächenprozent). Das Verhältnis 2M3BN/3PN betrug 1.95/1. Der Verlust an Ni(0) bezogen auf gebildetes Wertprodukt betrug: 0.58 kg Ni(0)It Wertprodukt (3PN/2M3BN).

### Beispiel 8: (Verhältnis BD/HCN =1.4:1)

2,13 mol über eine Schüttung aus Molsieb 4Å getrocknetes Butadien, 1,53 mol HCN und 14 mmol Ni in Form des Isopropylkatalysators werden pro Stunde in einen Druckreaktor eingespeist (Druck: 15 bar, Temperatur innen 105°C, Verweilzeit: ca. 40 Min/Reaktor). Der HCN-Umsatz ist nach Maßanalyse quantitativ (Titration nach Vollhard). Man bestimmt das Verhältnis 2M3BN/3PN des Reaktionsaustrages GC-chromatographisch (GC-Flächenprozent). Das Verhältnis 2M3BN/3PN betrug 1.95/1. Der Verlust an Ni(0) bezogen auf gebildetes Wertprodukt betrug:0.14 kg Ni(0)/t Wertprodukt (3PN/2M3BN).

### Beispiel 9: (Verhältnis BD/HCN =1.2:1)

2,09 mol über eine Schüttung aus Aluminiumoxid getrocknetes Butadien, 1,67 mol HCN und 14 mmol Ni in Form des Isopropylkatalysators werden pro Stunde in einen Druckreaktor eingespeist (Druck: 15 bar, Temperatur innen 105°C, Verweilzeit: ca. 45 Min/Reaktor). Der HCN-Umsatz ist nach Maßanalyse quantitativ (Titration nach Vollhard). Man bestimmt das Verhältnis 2M3BN/3PN des Reaktionsaustrages GC-chromatographisch (GC-Flächenprozent). Das Verhältnis 2M3BN/3PN betrug 1.95/1. Der Verlust an Ni(0) bezogen auf gebildetes Wertprodukt betrug: < 0.10 kg Ni(0)/t Wertprodukt (3PN/2M3BN).

### Beispiele zur kontinuierliche Isomerisierung von 2M3BN zu 3PN

### Beispiel 10:

Ein gemäß Beispiel 8 hergestellter Hydrocyanierungsaustrag wird gesammelt und destillativ von überschüssigem BD befreit. Die so erhaltene Mischung wird auf 130°C für eine Stunde erhitzt. Nach 0, 30 Min und nach 1 h werden GC-Proben aus dem Reaktionsgemisch entnommen und GC-chromatographisch (GC-Flächenprozent) untersucht.

| Zeit | 2M3BN | E,Z-2M2BN | c,t-2PN | 4PN | c,t-3PN | 3PN/2M3BN |
|---|---|---|---|---|---|---|
| 0 h | 15.62 | 0.20 | 0.50 | 0.50 | 38.33 | 2.45 |
| 30 Min | 10.21 | 0.21 | 0.51 | 0.49 | 42.36 | 4.15 |
| 1 h | 5.69 | 0.27 | 0.54 | 0.51 | 47.12 | 8.28 |

### Beispiele zur Fehlisomerisierung von 2M3BN zu 2M2BN durch rückgeführten Hydrocyanierungskatalysator

### Beispiel 11:

Aus einem Katalysatorreservoir, zu t = 0 h gefüllt mit 649 g frischem Isopropylkatalysator, werden kontinuierlich 100g Isopropylkatalysator entnommen und zusammen mit 2,14 mol über eine Schüttung aus Aluminiumoxid getrocknetes Butadien, sowie 1,67 mol HCN pro Stunde in einen Druckreaktor eingespeist (Druck: 15 bar, Temperatur innen 105°C, Verweilzeit: ca. 45 Min/Reaktor). Der HCN-Umsatz ist nach Maßanalyse quantitativ (Titration nach Vollhard). Das Wertprodukt wird kontinuierlich mittels einer Sambaydestillation vom Katalysator abgetrennt und der so erhaltene Rückkatalysator in das Reservoir zurückgefahren. Die Reaktion wird 50 h betrieben und der noch hydrocyanierungsaktive Katalysator aufgrund beginnender Bildung von der Nebenkomponente 2M2BN, ausgetragen. Der so erhaltene Katalysator wird Isomerisierungsversuchen unterzogen:

### Beispiel 12 (Vergleich):

10 g Isomerisierungskatalysator werden mit 2M3BN (15 g) aufgestockt und bei 120 °C für 5 h getempert. Bei einem Umsatz von 89% 2M3BN (GC-Flächenprozent) zeigen sich 8,6% Fehlisomere (2M2BN).

### Beispiel 13:

Zu dem Isomerisierungskatalysator aus Beispiel 11 (100 g) werden n-Heptan (100 g) und Adipodinitril (50 g) gegeben und die Mischung wird gerührt (15 min). Nach einer Phasentrennung (30 min) wird die untere Phase abgelassen. Ein Teil der oberen Phase (50 g, Heptan+Isomerisierungskatalysator) wird am Rotationsverdampfer eingeengt. Der Rückstand (14 g, Isomerisierungskatalysator) wird mit 2M3BN (21 g) aufgestockt und 5 h bei 120°C getempert. Bei einem Umsatz von 95% 2M3BN (GC-Flächenprozent) zeigen sich 2,0% Fehlisomere (2M2BN).

### Beispiel 14:

Die Reste der Oberphase aus der ersten Extraktion (Beispiel 12) werden erneut mit Adipodinitril (37,5 g) versetzt und gerührt. Nach der Phasentrennung wird wieder ein Teil der Oberphase am Rotationsverdampfer eingeengt und der Rückstand (9,3 g) mit 2M3BN (14 g) aufgestockt. Nach 5 h bei 120°C zeigt sich ein 2M3BN Umsatz von 94% (GC-Flächenprozent) und eine Fehlisomerisierung von 0,7%.

### Beispiele zur Fehlisomerisierung von 2M3BN zu 2M2BN durch kontinuierlich eingesetzten Isomerisierungskatalysator

### Beispiel 15:

In einen 21 Reaktor werden 300 g Isopropylkatalysator eingefüllt und kontinuierlich mit 450g/h 2M3BN versetzt und auf 130°C erwärmt. Bei einer Verweilzeit von 60 Min wird kontinuierlich Reaktorinhalt entnommen und destillativ aufgearbeitet und der im Sumpf verbleibende Isomerisierungskatalysator rückgeführt. Die Reaktion wird 50 h betrieben und der noch isomerisierungsaktive Katalysator aufgrund beginnender Fehlisomerisierung von 2M3BN, ausgetragen. Der so erhaltene Katalysator wird Isomerisierungsversuchen unterzogen:

### Beispiel 16:

10 g Isomerisierungskatalysator werden mit 2M3BN (15 g) aufgestockt und bei 120°C für 5 h getempert. Bei einem Umsatz von 90% 2M3BN (GC-Flächenprozent) zeigen sich 9,8% Fehlisomere (2M2BN).

### Beispiel 17:

Zu dem Isomerisierungskatalysator aus Beispiel 16 (100 g) werden n-Heptan (100 g) und Adipodinitril (50 g) gegeben und die Mischung wird gerührt (15 min). Nach einer Phasentrennung (30 min) wird die untere Phase abgelassen. Ein Teil der oberen Phase (50 g, Heptan+Isomerisierungskatalysator) wird am Rotationsverdampfer eingeengt. Der Rückstand (14 g, Isomerisierungskatalysator) wird mit 2M3BN (21 g) aufgestockt und 5 h bei 120°C getempert. Bei einem Umsatz von 93% 2M3BN (GC-Flächenprozent) zeigen sich 2,4% Fehlisomere (2M2BN).

### Beispiel 18:

Die Reste der Oberphase aus der ersten Extraktion (Beispiel 17) werden erneut mit Adipodinitril (37,5 g) versetzt und gerührt. Nach der Phasentrennung wird wieder ein Teil der Oberphase am Rotationsverdampfer eingeengt und der Rückstand (9,3 g) mit 2M3BN (14 g) aufgestockt. Nach 5 h bei 120°C zeigt sich ein 2M3BN Umsatz von 93% (GC-Flächenprozent) und eine Fehlisomerisierung von 0,6%.

## Patentansprüche

1. Verfahren zur Herstellung von 3-Pentennitril, **gekennzeichnet durch** die folgenden Verfahrensschritte:
(a) Isomerisierung eines Eduktstroms, der 2-Methyl-3-butennitril enthält, an mindestens einem gelösten oder dispergierten Isomerisierungskatalysator zu einem Strom 1, der den mindestens einen Isomerisierungskatalysator, 2-Methyl-3-butennitril, 3-Pentennitril und (Z)-2-Methyl-2-butennitril enthält,
(b) Destillation des Stromes 1 unter Erhalt eines Stromes 2 als Kopfprodukt, der 2-Methyl-3-butennitril, 3-Pentennitril und (Z)-2-Methyl-2-butennitril enthält, und eines Stromes 3 als Sumpfprodukt, der den mindestens einen Isomerisierungskatalysator enthält,
(c) Destillation des Stromes 2 unter Erhalt eines Stromes 4 als Kopfprodukt, der gegenüber dem Strom 2 an (Z)-2-Methyl-2-butennitril, bezogen auf die Summe aller Pentennitrile im Strom 2, angereichert ist, und eines Stromes 5 als Sumpfprodukt, der gegenüber dem Strom 2 an 3-Pentennitril und 2-Methyl-3-butennitril, bezogen auf die Summe aller Pentennitrile im Strom 2, angereichert ist,
(d) Destillation des Stromes 5 unter Erhalt eines Stromes 6 als Sumpfprodukt, der 3-Pentennitril enthält und eines Stromes 7 als Kopfprodukt, der 2-Methyl-3-butennitril enthält,
wobei man das an (Z)-2-Methyl-2-butennitril abgereicherte 2-Methyl-3-butennitril zurückführt.

2. Verfahren nach Anspruch 1, **dadurch gekennzeichnet, dass** der Eduktstrom durch folgende Verfahrensschritte erhalten wird:
(e) Hydrocyanierung von 1,3-Butadien an mindestens einem Hydrocyanierungskatalysator mit Cyanwasserstoff unter Erhalt eines Stromes 8, der den mindestens einen Hydrocyanierungskatalysator, 3-Pentennitril, 2-Methyl-3-butennitril , 1,3-Butadien und Reste Cyanwasserstoff enthält,
(f) ein- oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält,
(g) Destillation des Stromes 11 unter Erhalt eines Stromes 12 als Sumpfprodukt, der 3-Pentennitril enthält, und eines Stromes 13 als Kopfprodukt, der 2-Methyl-3-butennitril enthält.

3. Verfahren nach Anspruch 2, **dadurch gekennzeichnet, dass** Verfahrensschritt (d) und (g) in derselben Destillationsvorrichtung durchgeführt werden, wobei die Ströme 6 und 12 und die Ströme 7 und 13 zusammenfallen.

4. Verfahren nach einem der Ansprüche 2 oder 3, dadaurch **gekennzeichnet**, dass die Verfahrensschritte (c) und (g) in einer gemeinsamen Destillationskolonne durchgeführt, wobei der Verfahrensschritt (d) entfällt, der Strom 2 aus Verfahrensschritt (b) sowie Strom 11 aus Verfahrensschritt (f) in Verfahrensschritt (g) geführt werden, in Verfahrensschritt (g) der Strom 4 als Kopfprodukt, enthaltend (Z)-2-Methyl-2-butennitril, der Strom 12 als Sumpfprodukt, enthaltend 3-Pentennitril und der Strom 13 als Seitenabzugsstrom, enthaltend 2-Methyl-3-butennitril erhalten werden.

5. Verfahren nach einem der Ansprüche 1 bis 4, **dadurch gekennzeichnet, dass** der in Verfahrensschritt (b) in Strom 3 erhaltene mindestens eine Isomerisierungskatalysator in den Verfahrensschritt (a) zurückgeführt wird.

6. Verfahren nach einem der Ansprüche 1 bis 5, **dadurch gekennzeichnet, dass** die Verfahrensschritte (b) und (c) zusammen in einer Destillationsvorrichtung durchgeführt werden, wobei der Strom 3, der den mindestens einen Isomerisierungskatalysator enthält, als Sumpfprodukt, der Strom 4, der (Z)-2-Methyl-2-butennitril enthält, als Kopfprodukt und der Strom 5, der 3-Pentennitril und 2-Methyl-3-butennitril enthält, an einem Seitenabzug der Kolonne erhalten werden.

7. Verfahren nach einem der Ansprüche 1 bis 5, **dadurch gekennzeichnet, dass** die Verfahrensschritte (a), (b) und (c) zusammen in einer Destillationsvorrichtung durchgeführt werden, wobei der Strom 4, der (Z)-2-Methyl-2-butennitril enthält, als Kopfprodukt, der Strom 5, der 3-Pentennitril und 2-Methyl-3-butennitril enthält, an einem Seitenabzug der Destillationsvorrichtung erhalten werden und der Isomerisierungskatalysator im Sumpf der Destillationskolonne verbleibt.

8. Verfahren nach einem der Ansprüche 1 bis 7, **dadurch gekennzeichnet, dass** der Isomerisierungskatalysator Nickel(0), eine Nickel(0) als Ligand komplexierende, dreibindigen Phosphor enthaltende Verbindung und gegebenenfalls eine Lewis-Säure enthält.

9. Verfahren nach einem der Ansprüche 1 bis 8, **dadurch gekennzeichnet, dass** Druck und Temperatur in dem Verfahrensschritt (b) so eingestellt werden, dass der Isomerisierungskatalysator weniger aktiv als in Verfahrensschritt (a) oder nicht aktiv ist.

10. Verfahren nach einem der Ansprüche 1 bis 8, **dadurch gekennzeichnet, dass** der Hydrocyanierungskatalysator und der Isomerisierungskatalysator identisch sind.

11. Verfahren nach einem der Ansprüche 1 bis 10, **dadurch gekennzeichnet, dass** der Eduktstrom durch folgende Verfahrensschritte erhalten wird:
(a*) Isomerisierung eines Eduktstroms, der 2-Methyl-3-butennitril enthält, an mindestens einem gelösten oder dispergierten Isomerisierungskatalysator zu einem Strom 1, der den mindestens einen Isomerisierungskatalysator, 2-Methyl-3-butennitril, 3-Pentennitril und (Z)-2-Methyl-2-butennitril enthält,
(b*) Destillation des Stromes 1 unter Erhalt eines Stromes 2 als Kopfprodukt, der 2-Methyl-3-butennitril, 3-Pentennitril und (Z)-2-Methyl-2-butennitril enthält, und eines Stromes 3 als Sumpfprodukt, der den mindestens einen Isomerisierungskatalysator enthält,
(c*) Destillation des Stromes 2 unter Erhalt eines Stromes 4 als Kopfprodukt, der gegenüber dem Strom 2 an (Z)-2-Methyl-2-butennitril, bezogen auf die Summe aller Pentennitrile im Strom 2, angereichert ist, und eines Stromes 5 als Sumpfprodukt, der gegenüber dem Strom 2 an 3-Pentennitril und 2-Methyl-3-butennitril, bezogen auf die Summe aller Pentennitrile im Strom 2, angereichert ist,
(d*) Destillation des Stromes 5 unter Erhalt eines Stromes 6 als Sumpfprodukt, der 3-Pentennitril enthält, und eines Stromes 7 als Kopfprodukt, der 2-Methyl-3-butennitril enthält.
(h*) Katalysatorregenerierung zur Aufstockung der Nickel(0)-Gehaltes der Teilströme 14 aus Strom 3 bzw. 16 aus Strom 10 unter Erzeugung eunes Stroms 18,
(i*) gegebenenfalls unter Zusatz eines Verdünnungsmittels F zu Strom 18 unter Erzeugung von Strom 19,
(j*) Extraktion des Stromes 18, ggf Stromes 19, bezüglich der Katalysatorkomponenten und/oder Störkomponente(n) durch Zusatz eines Dinitrilstroms 20 und eines Kohlenwasserstoffstroms 21 unter Erzeugung zweier nichtmischbarer Phasen 22 und 23, wobei Strom 22 den überwiegenden Teil der Katalysatorkomponenten und Strom 23 den überwiegenden Teil der Störkomponente enthält,
(k*) destillative Abtrennung des Kohlenwasserstoffes von den Katalysatorkomponenten aus Strom 22 unter Erzeugung eines Stroms 25 , der den überwiegenden Teil der Katalysatorkomponenten enthält und ggf. teilweise oder ganze Rückführung des Stroms 25 in die Verfahrensschritte (a*) oder (e*),
(e*) Hydrocyanierung von 1,3-Butadien an mindestens einem Hydrocyanierungskatalysator mit Cyanwasserstoff unter Erhalt eines Stromes 8, der den mindestens einen Hydrocyanierungskatalysator, 3-Pentennitril, 2-Methyl-3-butennitril, 1,3-Butadien und Reste Cyanwasserstoff enthält,
(f*) ein- oder mehrfache Destillation des Stromes 8 unter Erhalt eines Stromes 9, der 1,3-Butadien enthält, eines Stromes 10, der den mindestens einen Hydrocyanierungskatalysator enthält, und eines Stromes 11, der 3-Pentennitril und 2-Methyl-3-butennitril enthält,
(g*) Destillation des Stromes 11 unter Erhalt eines Stromes 12 als Sumpfprodukt, der 3-Pentennitril enthält, und eines Stromes 13 als Kopfprodukt, der 2-Methyl-3-butennitril enthält.

12. Verfahren nach Anspruch 11, **dadurch gekennzeichnet, dass** man in Verfahrensstufe h*) die Aufstockung des Nickel(0)-Katalysatorgehaltes durch reduktive Katalysatorregenerierung durchführt.

13. Verfahren nach einem der Ansprüche 11 oder 12, **dadurch gekennzeichnet, dass** man die Katalysatorführung als zwei getrennte Katalysatorkreisläufe betreibt, wobei der eine Kreislauf die Stufen e*) und f*) und der andere Kreislauf die Stufen a*), b*) und c*) beinhaltet.

14. Verfahren nach einem der Ansprüche 11 bis 13, **dadurch gekennzeichnet, dass** man als Feedstrom zu e*) stabilisatorhaltiges Butadien einsetzt.

15. Verfahren nach einem der Ansprüche 11 bis 14, **dadurch gekennzeichnet, dass** man als Katalysatoren Phosphite der Formel I b
P (O-R¹)ₓ (O-R²)_{y} (O-R³)_{z} (O-R⁴)ₚ (I b)
mit
R¹: aromatischer Rest mit einem C₁-C₁₈-Alkylsubstituenten in o-Stellung zu dem Sauerstoffatom, das das Phosphoratom mit dem aromatischen Sys- tem verbindet, oder mit einem aromatischen Substituenten in o-Stellung zu dem Sauerstoffatom, das das Phosphoratom mit dem aromatischen Sys- tem verbindet, oder mit einem in o-Stellung zu dem Sauerstoffatom, das das Phosphoratom mit dem aromatischen System verbindet, anellierten aromatischen System,
R²: aromatischer Rest mit einem C₁-C₁₈-Alkylsubstituenten in m-Stellung zu dem Sauerstoffatom, das das Phosphoratom mit dem aromatischen Sys- tem verbindet, oder mit einem aromatischen Substituenten in m-Stellung zu dem Sauerstoffatom, das das Phosphoratom mit dem aromatischen System verbindet, oder mit einem in m-Stellung zu dem Sauerstoffatom, das das Phosphoratom mit dem aromatischen System verbindet, anellier- ten aromatischen System, wobei der aromatische Rest in o-Stellung zu dem Sauerstoffatom, das das Phosphoratom mit dem aromatischen Sys- tem verbindet, ein Wasserstoffatom trägt,
R³: aromatischer Rest mit einem C₁-C₁₈-Alkylsubstituenten in p-Stellung zu dem Sauerstoffatom, das das Phosphoratom mit dem aromatischen Sys- tem verbindet, oder mit einem aromatischen Substituenten in p-Stellung zu dem Sauerstoffatom, das das Phosphoratom mit dem aromatischen Sys- tem verbindet, wobei der aromatische Rest in o-Stellung zu dem Sauer- stoffatom, das das Phosphoratom mit dem aromatischen System verbin- det, ein Wasserstoffatom trägt,
R⁴: aromatischer Rest, der in o-, m- und p-Stellung zu dem Sauerstoffatom, das das Phosphoratom mit dem aromatischen System verbindet, andere als die für R¹, R² und R³ definierten Substituenten trägt, wobei der aroma- tische Rest in o-Stellung zu dem Sauerstoffatom, das das Phosphoratom mit dem aromatischen System verbindet, ein Wasserstoffatom trägt,
x : 1 oder 2,
y, z, p: unabhängig voneinander 0, 1 oder 2 mit der Maßgabe, dass x+y+z+p = 3,
einsetzt.

16. Verfahren nach einem der Ansprüche 11 bis 15, **dadurch gekennzeichnet, dass** man als Katalysatoren Phosphite der Formel I b
P (O-R¹)ₓ (O-R²)_{y} (O-R³)_{z} (O-R⁴)ₚ (I b)
worin R¹, R² und R³ unabhängig voneinander ausgewählt sind aus o-Isopropyl-phenyl, m-Tolyl und p-Tolyl, R⁴ Phenyl ist; x gleich 1 oder 2 ist, und y, z, p unabhängig voneinander 0, 1 oder 2 sind mit der Maßgabe, dass x+y+z+p = 3 ist; und deren Mischungen, also Gemische von 2 oder mehreren, bevorzugt 2 bis 10, besonders bevorzugt 2 bis 6 der Verbindungen der Formel Ib, einsetzt.

## Claims

1. A process for preparing 3-pentenenitrile, **characterized by** the following process steps:
(a) isomerizing a reactant stream which comprises 2-methyl-3-butenenitrile over at least one dissolved or dispersed isomerization catalyst to give a stream 1 which comprises the at least one isomerization catalyst, 2-methyl-3-butenenitrile, 3-pentenenitrile and (Z)-2-methyl-2-butenenitrile,
(b) distilling stream 1 to obtain a stream 2 as the top product which comprises 2-methyl-3-butenenitrile, 3-pentenenitrile and (Z)-2-methyl-2-butenenitrile, and a stream 3 as the bottom product which comprises the at least one isomerization catalyst,
(c) distilling stream 2 to obtain a stream 4 as the top product which, compared to stream 2, is enriched in (Z)-2-methyl-2-butenenitrile, based on the sum of all pentenenitriles in stream 2, and a stream 5 as the bottom product which, compared to stream 2, is enriched in 3-pentenenitrile and 2-methyl-3-butenenitrile, based on the sum of all pentenenitriles in stream 2,
(d) distilling stream 5 to obtain a stream 6 as the bottom product which comprises 3-pentenenitrile and a stream 7 as the top product which comprises 2-methyl-3-butenenitrile,
the 2-methyl-3-butenenitrile depleted of (z)-2-methyl-2-butenenitrile being recycled.

2. The process according to claim 1, wherein the reactant stream is obtained by the following process steps:
(e) hydrocyanating 1,3-butadiene over at least one hydrocyanation catalyst using hydrogen cyanide to obtain a stream 8 which comprises the at least one hydrocyanation catalyst, 3-pentenenitrile, 2-methyl-3-butenenitrile, 1,3-butadiene and residues of hydrogen cyanide,
(f) distilling stream 8 once or more than once to obtain a stream 9 which comprises 1,3-butadiene, a stream 10 which comprises the at least one hydrocyanation catalyst, and a stream 11 which comprises 3-pentenenitrile and 2-methyl-3-butenenitrile,
(g) distilling stream 11 to obtain a stream 12 as the bottom product which comprises 3-pentenenitrile, and a stream 13 as the top product which comprises 2-methyl-3-butenenitrile.

3. The process according to claim 2, wherein process step (d) and (g) are carried out in the same distillation apparatus, in which case streams 6 and 12 and streams 7 and 13 coincide.

4. The process according to either of claims 2 and 3, wherein process steps (c) and (g) are carried out in a common distillation column, in which case process step (d) is dispensed with, stream 2 from process step (b) and stream 11 from process step (f) are conducted into process step (g), and, in process step (g), stream 4 is obtained as the top product comprising (Z)-2-methyl-2-butenenitrile, stream 12 as the bottom product comprising 3-pentenenitrile and stream 13 as a side draw stream comprising 2-methyl-3-butenenitrile.

5. The process according to any of claims 1 to 4, wherein the at least one isomerization catalyst obtained in stream 3 in process step (b) is recycled into process step (a).

6. The process according to any of claims 1 to 5, wherein process steps (b) and (c) are carried out together in one distillation apparatus, in which case stream 3 which comprises the at least one isomerization catalyst is obtained as the bottom product, stream 4 which comprises (Z)-2-methyl-2-butenenitrile as the top product, and stream 5 which comprises 3-pentenenitrile and 2-methyl-3-butenenitrile at a side draw of the column.

7. The process according to any of claims 1 to 5, wherein process steps (a), (b) and (c) are carried out together in one distillation apparatus, in which case stream 4 which comprises (Z)-2-methyl-2-butenenitrile is obtained as the top product, and stream 5 which comprises 3-pentenenitrile and 2-methyl-3-butenenitrile at a side draw of the distillation apparatus, and the isomerization catalyst remains in the bottom of the distillation column.

8. The process according to any of claims 1 to 7, wherein the isomerization catalyst contains nickel(0), a trivalent phosphorus-containing compound which complexes nickel(0) as a ligand and, if appropriate, a Lewis acid.

9. The process according to any of claims 1 to 8, wherein pressure and temperature in process step (b) are set in such a way that the isomerization catalyst is less active than in process step (a) or is inactive.

10. The process according to any of claims 1 to 8, wherein the hydrocyanation catalyst and the isomerization catalyst are identical.

11. The process according to any of claims 1 to 10, wherein the reactant stream is obtained by the following process steps:
(a*) isomerizing a reactant stream which comprises 2-methyl-3-butenenitrile over at least one dissolved or dispersed isomerization catalyst to give a stream 1 which comprises the at least one isomerization catalyst, 2-methyl-3-butenenitrile, 3-pentenenitrile and (Z)-2-methyl-2-butenenitrile,
(b*) distilling stream 1 to obtain a stream 2 as the top product which comprises 2-methyl-3-butenenitrile, 3-pentenenitrile and (Z)-2-methyl-2-butenenitrile, and a stream 3 as the bottom product which comprises the at least one isomerization catalyst,
(c*) distilling stream 2 to obtain a stream 4 as the top product which, compared to stream 2, is enriched in (Z)-2-methyl-2-butenenitrile, based on the sum of all pentenenitriles in stream 2, and a stream 5 as the bottom product which, compared to stream 2, is enriched in 3-pentenenitrile and 2-methyl-3-butenenitrile, based on the sum of all pentenenitriles in stream 2,
(d*) distilling stream 5 to obtain a stream 6 as the bottom product which comprises 3-pentenenitrile and a stream 7 as the top product which comprises 2-methyl-3-butenenitrile,
(h*) catalyst regeneration to replenish the nickel(0) content of the substreams 14 from stream 3 and 16 from stream 10 to generate a stream 18,
(i*) if appropriate with addition of a diluent F to stream 18 to generate stream 19,
(j*) extracting stream 18, if appropriate stream 19, with regard to the catalyst components and/or disruptive component(s) by adding a dinitrile stream 20 and hydrocarbon stream 21 to generate two nonmiscible phases 22 and 23, stream 22 comprising the predominant proportion of the catalyst components and stream 23 the predominant proportion of the disruptive component,
(k*) distillatively removing the hydrocarbon from the catalyst components from stream 22 to generate a stream 25 which comprises the predominant proportion of the catalyst components and, if appropriate, partly or fully recycling stream 25 into process steps (a*) or (e*),
(e*) hydrocyanating 1,3-butadiene over at least one hydrocyanation catalyst using hydrogen cyanide to obtain a stream 8 which comprises the at least one hydrocyanation catalyst, 3-pentenenitrile, 2-methyl-3-butenenitrile, 1,3-butadiene and residues of hydrogen cyanide,
(f*) distilling stream 8 once or more than once to obtain a stream 9 which comprises 1,3-butadiene, a stream 10 which comprises the at least one hydrocyanation catalyst, and a stream 11 which comprises 3-pentenenitrile and 2-methyl-3-butenenitrile,
(g*) distilling stream 11 to obtain a stream 12 as the bottom product which comprises 3-pentenenitrile, and a stream 13 as the top product which comprises 2-methyl-3-butenenitrile.

12. The process according to claim 11, wherein the replenishment of the nickel(0) catalyst content is carried out in process stage h*) by reductive catalyst regeneration.

13. The process according to either of claims 11 and 12, wherein the catalyst system is operated as two separate catalyst circuits, one of the circuits including the stages e*) and f*) and the other circuit the stages a*), b*) and c*).

14. The process according to any of claims 11 to 13, wherein stabilizer-containing butadiene is used as the feed stream to e*).

15. The process according to any of claims 11 to 14, wherein the catalysts used are phosphites of the formula I b
P (O-R¹)ₓ (O-R²)_{y} (O-R³)_{z} (O-R⁴)ₚ (I b)
where
R¹: aromatic radical having a C₁-C₁₈-alkyl substituent in the o-position to the oxygen atom which joins the phosphorus atom to the aromatic system, or having an aromatic substituent in the o-position to the oxygen atom which joins the phosphorus atom to the aromatic system, or having a fused aromatic system in the o-position to the oxygen atom which joins the phosphorus atom to the aromatic system,
R²: aromatic radical having a C₁-C₁₈-alkyl substituent in the m-position to the oxygen atom which joins the phosphorus atom to the aromatic system, or having an aromatic substituent in the m-position to the oxygen atom which joins the phosphorus atom to the aromatic system, or having a fused aromatic system in the m-position to the oxygen atom which joins the phosphorus atom to the aromatic system, the aromatic radical bearing a hydrogen atom in the o-position to the oxygen atom which joins the phosphorus atom to the aromatic system,
R³: aromatic radical having a C₁-C₁₈-alkyl substituent in the p-position to the oxygen atom which joins the phosphorus atom to the aromatic system, or having an aromatic substituent in the p-position to the oxygen atom which joins the phosphorus atom to the aromatic system, the aromatic radical bearing a hydrogen atom in the o-position to the oxygen atom which joins the phosphorus atom to the aromatic system,
R⁴_{:} aromatic radical which bears substituents other than those defined for R¹, R² and R³ in the o-, m- and p-position to the oxygen atom which joins the phosphorus atom to the aromatic system, the aromatic radical bearing a hydrogen atom in the o-position to the oxygen atom which joins the phosphorus atom to the aromatic system,
x: 1 or 2,
y, z, p: each independently 0, 1 or 2, with the proviso that x+y+z+p=3.

16. The process according to any of claims 11 to 15, wherein the catalysts used are phosphites of the formula I b
P (O-R¹)ₓ (O-R²)_{y} (O-R³)_{z} (O-R⁴)ₚ (I b)
where R¹, R² and R³ are each independently selected from o-isopropylphenyl, m-tolyl and p-tolyl, R⁴ is phenyl; x is 1 or 2, and y, z, p are each independently 0, 1 or 2, with the proviso that x+y+z+p = 3; and mixtures thereof, i.e. mixtures of 2 or more, preferably from 2 to 10, more preferably from 2 to 6, of the compounds of the formula Ib.

## Revendications

1. Procédé de production de 3-pentène-nitrile, **caractérisé par** les étapes de procédé suivantes:
(a) isomérisation d'un flux d'éduit, qui contient du 2-méthyle-3-butène-nitrile, sur au moins un catalyseur d'isomérisation dissous ou dispersé pour obtenir un flux 1 qui contient ledit au moins un catalyseur d'isomérisation, du 2-méthyle-3-butène-nitrile, 3-pentène-nitrile et du (Z)-2-méthyle-2-butène-nitrile;
(b) distillation du flux 1 avec obtention, comme produit de tête, d'un flux 2 qui contient du 2-méthyle-3-butène-nitrile, du 3-pentène-nitrile et du (Z)-2-méthyle-2-butène-nitrile et, comme produit de bas de colonne, d'un flux 3 qui contient ledit au moins un catalyseur d'isomérisation;
(c) distillation du flux 2 avec obtention, comme produit de tête, d'un flux 4 qui, par rapport au flux 2, est enrichi en (Z)-2-méthyle-2-butène-nitrile par rapport à la somme de tous les pentène-nitriles du flux 2 et, comme produit de bas de colonne, d'un flux 5 qui, par rapport au flux 2, est enrichi en 3-pentène-nitrile et en 2-méthyle-3-butène-nitrile par rapport à la somme de tous les pentène-nitriles du flux 2;
(d) distillation du flux 5 avec obtention, comme produit de bas de colonne, d'un flux 6 qui contient du 3-pentène-nitrile et, comme produit de tête, d'un flux 7 qui contient du 2-méthyle-3-butène-nitrile,
dans lequel on renvoie le 2-méthyle-3-butène-nitrile appauvri en (Z)-2-méthyle-2-butène-nitrile.

2. Procédé selon la revendication 1, **caractérisé en ce que** le flux d'éduit est obtenu par les étapes de procédé suivantes:
(e) hydrocyanation de 1,3-butadiène sur au moins un catalyseur d'hydrocyanation avec l'acide cyanhydrique avec obtention d'un flux 8, qui contient ledit au moins un catalyseur d'hydrocyanation, du 3-pentène-nitrile, du 2-méthyle-3-butène-nitrile, du 1,3-butadiène et des radicaux d'acide cyanhydrique,
(f) distillation simple ou multiple du flux 8 avec obtention d'un flux 9, qui contient du 1,3-butadiène, d'un flux 10 qui contient ledit au moins un catalyseur d'hydrocyanation, et d'un flux 11, qui contient du 3-pentène-nitrile et du 2-méthyle-3-butène-nitrile,
(g) distillation du flux 11 avec obtention, comme produit de bas de colonne, d'un flux 12 qui contient du 3-pentène-nitrile, et comme produit de tête, d'un flux 13 qui contient du 2-méthyle-3-butène-nitrile.

3. Procédé selon la revendication 2, **caractérisé en ce que** les étapes de procédé (d) et (g) sont exécutées dans le même dispositif de distillation, dans lequel les flux 6 et 12 et les flux 7 et 13 coïncident.

4. Procédé selon l'une quelconque des revendications 2 ou 3, **caractérisé en ce que** les étapes de procédé (c) et (g) sont exécutées dans une colonne de distillation commune, dans lequel l'étape de procédé (d) est supprimée, le flux 2 provenant de l'étape de procédé (b) ainsi que le flux 11 provenant de l'étape de procédé (f) sont conduits dans l'étape de procédé (g), on obtient à l'étape de procédé (g) le flux 4 comme produit de tête qui contient du (Z)-2-méthyle-2-butène-nitrile, le flux 12 comme produit de bas de colonne qui contient du 3-pentène-nitrile, et le flux 13 comme flux de soutirage latéral qui contient du 2-méthyle-3-butène-nitrile.

5. Procédé selon l'une quelconque des revendications 1 à 4, **caractérisé en ce que** ledit au moins un catalyseur d'isomérisation obtenu dans le flux 3 à l'étape de procédé (b) est renvoyé dans l'étape de procédé (a).

6. Procédé selon l'une quelconque des revendications 1 à 5, **caractérisé en ce que** les étapes de procédé (b) et (c) sont exécutées ensemble dans un dispositif de distillation, dans lequel on obtient le flux 3 comme produit de bas de colonne qui contient ledit au moins un catalyseur d'isomérisation, le flux 4 comme produit de tête qui contient du (Z)-2-méthyle-2-butène-nitrile et le flux 5, qui contient du 3-pentène-nitrile et du 2-méthyle-3-butène-nitrile à un soutirage latéral de la colonne.

7. Procédé selon l'une quelconque des revendications 1 à 5, **caractérisé en ce que** les étapes de procédé (a), (b) et (c) sont exécutées ensemble dans un dispositif de distillation, dans lequel on obtient le flux 4 comme produit de tête qui contient du (Z)-2-méthyle-2-butène-nitrile, le flux 5 qui contient du 3-pentène-nitrile et du 2-méthyle-3-butène-nitrile à un soutirage latéral du dispositif de distillation, et le catalyseur d'isomérisation reste dans le bas de la colonne de distillation.

8. Procédé selon l'une quelconque des revendications 1 à 7, **caractérisé en ce que** le catalyseur d'isomérisation contient du nickel(0), un composé contenant du phosphore trivalent et complexant le nickel(0) comme ligand et éventuellement un acide de Lewis.

9. Procédé selon l'une quelconque des revendications 1 à 8, **caractérisé en ce que** l'on règle la pression et la température dans l'étape de procédé (b) de telle manière que le catalyseur d'isomérisation soit moins actif que dans l'étape de procédé (a) ou ne soit pas actif.

10. Procédé selon l'une quelconque des revendications 1 à 8, **caractérisé en ce que** le catalyseur d'hydrocyanation et le catalyseur d'isomérisation sont identiques.

11. Procédé selon l'une quelconque des revendications 1 à 10, **caractérisé en ce que** le flux d'éduit est obtenu par les étapes de procédé suivantes:
(a*) isomérisation d'un flux d'éduit, qui contient du 2-méthyle-3-butène-nitrile, sur au moins un catalyseur d'isomérisation dissous ou dispersé pour obtenir un flux 1 qui contient ledit au moins un catalyseur d'isomérisation, du 2-méthyle-3-butène-nitrile, du 3-pentène-nitrile et du (Z)-2-méthyle-2-butène-nitrile;
(b*) distillation du flux 1 avec obtention, comme produit de tête, d'un flux 2 qui contient du 2-méthyle-3-butène-nitrile, du 3-pentène-nitrile et du (Z)-2-méthyle-2-butène-nitrile et, comme produit de bas de colonne, d'un flux 3 qui contient ledit au moins un catalyseur d'isomérisation;
(c*) distillation du flux 2 avec obtention, comme produit de tête, d'un flux 4 qui, par rapport au flux 2, est enrichi en (Z)-2-méthyle-2-butène-nitrile par rapport à la somme de tous les pentène-nitriles du flux 2 et, comme produit de bas de colonne, d'un flux 5 qui, par rapport au flux 2, est enrichi en 3-pentène-nitrile et en 2-méthyle-3-butène-nitrile par rapport à la somme de tous les pentène-nitriles du flux 2;
(d*) distillation du flux 5 avec obtention, comme produit de bas de colonne, d'un flux 6 qui contient du 3-pentène-nitrile et, comme produit de tête, d'un flux 7 qui contient du 2-méthyle-3-butène-nitrile;
(h*) régénération du catalyseur pour l'augmentation de la teneur en nickel(0) des flux partiels 14 provenant du flux 3 ou 16 provenant du flux 10 avec production d'un flux 18;
(i*) éventuellement avec addition d'un agent de dilution F au flux 18 avec production d'un flux 19;
(j*) extraction du flux 18, éventuellement du flux 19, par rapport aux composants de catalyseur et/ou au(x) composant(s) parasite(s) par addition d'un flux de dinitrile 20 et d'un flux d'hydrocarbure 21 avec production de deux phases non miscibles 22 et 23, dans lequel le flux 22 contient la partie prépondérante des composants de catalyseur et le flux 23 contient la partie prépondérante des composants parasites;
(k*) séparation par distillation de l'hydrocarbure et des composants de catalyseur provenant du flux 22 avec production d'un flux 25, qui contient la partie prépondérante des composants de catalyseur et éventuellement renvoi partiel ou total du flux 25 dans les étapes de procédé (a*) et (e*);
(e*) hydrocyanation de 1,3-butadiène sur au moins un catalyseur d'hydrocyanation avec l'acide cyanhydrique avec obtention d'un flux 8, qui contient ledit au moins un catalyseur d'hydrocyanation, du 3-pentène-nitrile, du 2-méthyle-3-butène-nitrile, du 1,3-butadiène et des radicaux d'acide cyanhydrique,
(f*) distillation simple ou multiple du flux 8 avec obtention d'un flux 9, qui contient du 1,3-butadiène, d'un flux 10 qui contient ledit au moins un catalyseur d'hydrocyanation, et d'un flux 11, qui continent du 3-pentène-nitrile et du 2-méthyle-3-butène-nitrile,
(g*) distillation du flux 11 avec obtention, comme produit de bas de colonne, d'un flux 12 qui contient du 3-pentène-nitrile, et comme produit de tête, d'un flux 13 qui contient du 2-méthyle-3-butène-nitrile.

12. Procédé selon la revendication 11, **caractérisé en ce que** l'on effectue l'augmentation de la teneur du catalyseur en nickel(0) par régénération réductrice du catalyseur dans l'étape de procédé h*).

13. Procédé selon l'une quelconque des revendications 11 ou 12, **caractérisé en ce que** l'on opère la conduite du catalyseur sous forme de deux circuits de catalyseur séparés, dans lequel un premier circuit comprend les étapes (e*) et (f*) et l'autre circuit comprend les étapes (a*), (b*) et (c*).

14. Procédé selon l'une quelconque des revendications 11 à 13, **caractérisé en ce que** l'on utilise comme flux d'alimentation à (e*) du butadiène contenant un stabilisateur.

15. Procédé selon l'une quelconque des revendications 11 à 14, **caractérisé en ce que** l'on utilise comme catalyseurs des phosphites de la formule Ib
P(O-R¹)ₓ(O-R²)_{y}(O-R³)_{z}(O-R⁴)ₚ (Ib)
dans laquelle
R¹: radical aromatique avec un substituant alkyle en C₁-C₁₈ en position o par rapport à l'atome d'oxygène qui relie l'atome de phosphore au système aromatique, ou avec un substituant aromatique en position o par rapport à l'atome d'oxygène qui relie l'atome de phosphore au système aromatique, ou avec un système aromatique anellé en position o par rapport à l'atome d'oxygène qui relie l'atome de phosphore au système aromatique,
R² radical aromatique avec un substituant alkyle en C₁-C₁₈ en position m par rapport à l'atome d'oxygène qui relie l'atome de phosphore au système aromatique, ou avec un substituant aromatique en position m par rapport à l'atome d'oxygène qui relie l'atome de phosphore au système aromatique, ou avec un système aromatique anellé en position m par rapport à l'atome d'oxygène qui relie l'atome de phosphore au système aromatique, dans lequel le radical aromatique porte un atome d'hydrogène en position o par rapport à l'atome d'oxygène qui relie l'atome de phosphore au système aromatique,
R³: radical aromatique avec un substituant alkyle en C₁-C₁₈ en position p par rapport à l'atome d'oxygène qui relie l'atome de phosphore au système aromatique, ou avec un substituant aromatique en position p par rapport à l'atome d'oxygène qui relie l'atome de phosphore au système aromatique, dans lequel le radical aromatique porte un atome d'hydrogène en position o par rapport à l'atome d'oxygène qui relie l'atome de phosphore au système aromatique,
R⁴: radical aromatique, qui porte des substituants autres que ceux définis pour R¹, R² et R³ en position o, m et p par rapport à l'atome d'oxygène qui relie l'atome de phosphore au système aromatique, dans lequel, le radical aromatique porte un atome d'hydrogène en position o par rapport à l'atome d'oxygène qui relie l'atome de phosphore au système aromatique,
x: 1 ou 2,
y,z,p: 0, 1 ou 2 indépendamment l'un de l'autre, avec la condition que x+y+z+p = 3.

16. Procédé selon l'une quelconque des revendications 11 à 15, **caractérisé en ce que** l'on utilise comme catalyseurs des phosphites de la formule Ib
P(O-R¹)ₓ(O-R²)_{y}(O-R³)_{z}(O-R⁴)ₚ (Ib)
dans laquelle R¹, R² et R³ sont choisis indépendamment l'un de l'autre parmi le o-isopropylphényle, le m-tolyle et le p-tolyle, R⁴ est le phényle; x est égal à 1 ou 2, et y, z, p sont 0, 1 ou 2 indépendamment l'un de l'autre, avec la condition que x+y+z+p=3; et leurs mélanges, donc des mélanges de 2 ou plusieurs, de préférence 2 à 10, en particulier de préférence 2 à 6 des composés de la formule Ib.
